(19) Europäisches Patentamt
European Patent Office
Office européen des brevets

(11) EP 4 461 726 A1

(12) EUROPEAN PATENT APPLICATION
published in accordance with Art. 153(4) EPC

(43) Date of publication:
**13.11.2024 Bulletin 2024/46**

(21) Application number: **23737354.3**

(22) Date of filing: **03.01.2023**

(51) International Patent Classification (IPC):
***C07D 209/44*** (2006.01) ***A61K 31/4035*** (2006.01)
***A61K 31/4439*** (2006.01) ***A61K 31/4155*** (2006.01)
***A61K 31/506*** (2006.01) ***A61P 19/02*** (2006.01)
***C07D 403/04*** (2006.01) ***C07D 401/04*** (2006.01)
***C07D 487/04*** (2006.01) ***C07D 405/04*** (2006.01)

(52) Cooperative Patent Classification (CPC):
**A61K 31/4035; A61K 31/4155; A61K 31/4184; A61K 31/4439; A61K 31/496; A61K 31/506; A61P 19/02; A61P 29/00; C07D 209/44; C07D 401/04; C07D 403/04; C07D 405/04; C07D 487/04**

(86) International application number:
**PCT/KR2023/000101**

(87) International publication number:
**WO 2023/132606 (13.07.2023 Gazette 2023/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.01.2022 KR 20220000659**

(71) Applicant: **Innovo Therapeutics Inc.**
**Seoul 04174 (KR)**

(72) Inventors:
- **CHOI, Sei Hyun**
**Daejeon 34022 (KR)**
- **KIM, Hak Joong**
**Sejong-si 30150 (KR)**
- **LEE, Yu Rim**
**Daejeon 34016 (KR)**
- **JUNG, Sun Ho**
**Cheonan-si, Chungcheongnam-do 31091 (KR)**
- **PAGIRE, Haushabhau Shivaji**
**Gwangju 61005 (KR)**

(74) Representative: **Graf von Stosch Patentanwaltsgesellschaft mbH**
**Prinzregentenstraße 22**
**80538 München (DE)**

# (54) NOVEL ISOINDOLINONE DERIVATIVE COMPOUNDS AS CASPASE INHIBITORS

(57) The present invention provides a novel isoindolinone derivative compound, a hydrate thereof, a solvate thereof or a pharmaceutically acceptable salt thereof, as caspase inhibitors. The novel isoindolinone derivative compound of the present invention was confirmed to exhibit an excellent interleukin-1β(IL-1β) reducing effect and an excellent apoptosis-reducing effect, thus exhibiting excellent activity as a caspase inhibitor, and thus, it has been found that the novel isoindolinone derivative compound can be used for the prevention or treatment of caspase-related diseases.

Therefore, the novel isoindolinone derivative compound of the present invention can be effectively used in the prevention or treatment of caspase-related diseases in the medical and pharmaceutical fields.

EP 4 461 726 A1

## Description

### FIELD

The present invention relates to caspase inhibitors, and more specifically, to novel isoindolinone derivative compounds as caspase inhibitors.

### BACKGROUND

Caspase is a type of enzyme, a cysteine protease that exists as a tetramer in the form of $\alpha 2\beta 2$, and caspase inhibitor is a compound that can modulate inflammation or apoptosis caused by the action of caspases by interfering with their activity. Diseases that may be treated with these compounds to eliminate or reduce symptoms include osteoarthritis, rheumatoid arthritis, degenerative arthritis, destructive bone disorders, liver disease caused by hepatitis viruses, acute hepatitis, cirrhosis, brain injury caused by hepatitis viruses, human fulminant hepatic failure, sepsis, organ transplant rejection, ischemic heart disease, dementia, stroke, brain injury caused by AIDS, diabetes, and gastric ulcers.

Among the various structural compounds known as caspase inhibitors, isoxazoline derivatives have been disclosed in Korean Patent Applications No. 10-2004-0066726, No. 10-2006-0013107 and No. 10-2008-0025123. In addition, a prodrug of a caspase inhibitor based on an isoxazoline derivative is disclosed in WO 2007/015931 (Applicant: Vertex Pharmaceuticals Incorporated, USA).

### OBJECT OF THE INVENTION

The problem to be addressed by the present invention is provide novel structured caspase inhibitory compounds that exhibit superior inhibitory activity against caspases.

Further, the problem to be addressed by the present invention is to provide pharmaceutical composition for preventing or treating caspase-related disease comprising the novel structured caspase inhibitory compounds.

Further, the problem to be addressed by the present invention is to provide a method for preventing or treating diseases related to caspase inhibition using the caspase inhibitory compound having the novel structure.

Further, the problem to be addressed by the present invention is to provide a use of the caspase inhibitory compound having the novel structure for the prevention or treatment of caspase-related diseases.

Further, the problem to be addressed by the present invention is to provide a method for preparing a caspase inhibitory compound having the novel structure.

The problem to be addressed by the present invention is not limited to the problems mentioned above, and other technical problems not mentioned can be clearly understood by those skilled in the art from the description below.

### SUMMARY

To address the problem above, according to one aspect of the present invention, there is provided an isoindolinone derivative compound represented by the following Formula I, a hydrate thereof, a solvate thereof or a pharmaceutically acceptable salt thereof, wherein:

<Formula I>

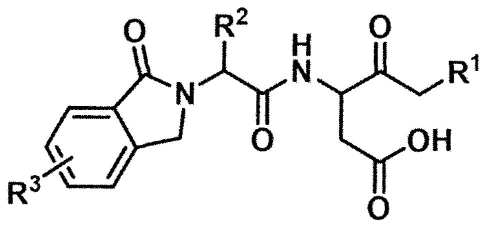

$R^1$ is halogen, or aryloxy substituted with 1 to 5 halogens,
$R^2$ is hydrogen, or $C_{1-6}$ straight or branched alkyl chain,
$R^3$ is halogen or Q, Q is substituted or unsubstituted with 1 to 3 independent $R^a$,
Q is aryl, aminoaryl, heteroaryl or non-aromatic heterocyclic, and Q is optionally fused with saturated or unsaturated 5- to 7-membered ring having 0 to 3 heteroatoms,
$R^a$ is $C_{1-6}$ straight or branched alkyl chain, $C_{1-6}$ alkoxy, halogen, $C_{1-6}$ haloalkyl, $CO_2R^b$, $COR^b$, $CONHR^b$, $CON(R^b)_2$,

$NHR^b$, $N(R^b)_2$, $NHCOR^b$, $S(O)_2R^b$, or heteroaryl fused to a saturated or unsaturated 5- to 7-membered ring having 0 to 3 heteroatoms,

$R^b$ is hydrogen or $C_{1-6}$ straight or branched alkyl chain.

According to another aspect of the present invention, there is provided a pharmaceutical composition for preventing or treating caspase-related disease comprising the isoindolinone derivative compound represented by the above Formula I, a hydrate thereof, a solvate thereof or a pharmaceutically acceptable salt thereof as an active ingredient.

According to another aspect of the present invention, there is provided a method for preventing or treating caspase-related disease, comprising: administering the isoindolinone derivative compound represented by the above Formula I , a hydrate thereof, a solvate thereof or a pharmaceutically acceptable salt thereof, to a subject in need thereof.

According to another aspect of the present invention, there is provided a use of the isoindolinone derivative compound represented by the above Formula I , a hydrate thereof, a solvate thereof or a pharmaceutically acceptable salt thereof, in manufacture of a medicament for preventing or treating caspase-related disease.

According to another aspect of the present invention, there is provided a pharmaceutical composition comprising the isoindolinone derivative compound represented by the above Formula I , a hydrate thereof, a solvate thereof or a pharmaceutically acceptable salt thereof and a pharmaceutically acceptable additive.

According to another aspect of the present invention, there is provided a method for preparing an isoindolinone derivative compound represented by the above Formula I .

The novel isoindolinone derivative compound of the present invention was confirmed to exhibit an excellent interleukin-1β(IL-1β) reducing effect and an excellent apoptosis-reducing effect, thus exhibiting excellent activity as a caspase inhibitor, and thus, it has been found that the novel isoindolinone derivative compound can be used for the prevention or treatment of caspase-related diseases.

Therefore, the novel isoindolinone derivative compound of the present invention can be effectively used in the prevention or treatment of caspase-related diseases in the medical and pharmaceutical fields.

The effects of the present invention are not limited to the above effects, but should be understood to include all effects that can be inferred from the description of the invention or the composition of the invention as recited in the patent claims.

DETAILED DESCRIPTION OF THE INVENTION

The present invention provides an isoindolinone derivative compound represented by the following Formula I, a hydrate thereof, a solvate thereof or a pharmaceutically acceptable salt thereof, wherein:

<Formula I>

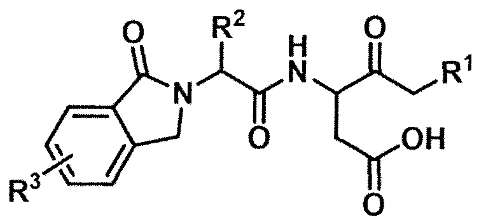

$R^1$ is halogen, or aryloxy substituted with 1 to 5 halogens,

$R^2$ is hydrogen, or $C_{1-6}$ straight or branched alkyl chain,

$R^3$ is halogen or Q, Q is substituted or unsubstituted with 1 to 3 independent $R^a$,

Q is aryl, aminoaryl, heteroaryl or non-aromatic heterocyclic, and Q is optionally fused with saturated or unsaturated 5- to 7-membered ring having 0 to 3 heteroatoms,

$R^a$ is $C_{1-6}$ straight or branched alkyl chain, $C_{1-6}$ alkoxy, halogen, $C_{1-6}$ haloalkyl, $CO_2R^b$, $COR^b$, $CONHR^b$, $CON(R^b)_2$, $NHR^b$, $N(R^b)_2$, $NHCOR^b$, $S(O)_2R^b$, or heteroaryl fused to a saturated or unsaturated 5- to 7-membered ring having 0 to 3 heteroatoms,

$R^b$ is hydrogen or $C_{1-6}$ straight or branched alkyl chain.

In one embodiment, $R^1$ may be F, or phenoxy substituted with 1 to 5 F.

In one embodiment, $R^2$ may be hydrogen, methyl, ethyl, or straight or branched chain propyl.

In one embodiment, $R^3$ may be Br or Q, wherein Q may be phenyl, pyridinyl, tetrahydropyridinyl, pyrazolyl, pyrimidinyl, dihydropyranyl, thiophenyl, pyrrolidinyl, piperazinyl, aminonaphthalenyl, naphthalenyl, quinolinyl, isoquinolinyl, benzofuranyl, benzothiazolyl, dihydrobenzodioxynyl, oxo-dihydrobenzoimidazolyl, benzothiophenyl, pyrazolopyridinyl or dihydroimidazopyrazinyl.

In one embodiment, $R^a$ may be methyl, methoxy, F, trifluoromethyl, carboxy, acetyl, amino, methylsulfonyl or benzoisothiazolyl.

Representative examples of isoindolinone derivative compounds according to the present invention are as follows:

[1] 5-fluoro-4-oxo-3-(2-(1-oxo-6-phenylisoindolin-2-yl)butanamido)pentanoic acid,
[2] 5-fluoro-3-(2-(6-(naphthalen-1-yl)-1-oxoisoindolin-2-yl)butanamido)-4-oxopentanoic acid,
[3] 5-fluoro-4-oxo-3-(2-(1-oxo-6-(pyrrolidin-1-yl)isoindolin-2-yl)butanamido)pentanoic acid,
[4] 5-fluoro-4-oxo-3-(2-(1-oxo-6-(pyridin-3-yl)isoindolin-2-yl)butanamido)pentanoic acid,
[5] 5-fluoro-4-oxo-3-(2-(1-oxo-6-(1H-pyrazol-4-yl)isoindolin-2-yl)butanamido)pentanoic acid,
[6] 5-fluoro-3-(2-(6-(4-(methylsulfonyl)piperazin-1 -yl)-1 -oxoisoindolin-2-yl)butanamido)-4-oxopentanoic acid,
[7] 5-fluoro-3-(2-(6-(1-(methylsulfonyl)-1,2,3,6-tetrahydropyridin-4-yl)-1-oxoisoindolin-2-yl)butanamido-4-oxopentanoic acid,
[8] 5-fluoro-4-oxo-3-(2-(1-oxo-6-(2-oxo-2,3-dihydro-1H-benzo[d]imidazol-5-yl)isoindolin-2-yl)butanamido)pentanoic acid,
[9] 3-(2-(6-bromo-1-oxoisoindolin-2-yl)butanamido)-5-fluoro-4-oxopentanoic acid,
[10] 5-fluoro-4-oxo-3-(2-(1-oxo-6-(trifluoromethyl)-5,6-dihydroimidazo[1,2-a]pyrazin-7(8H)-yl)isoindolin-2-yl)butanamido)pentanoic acid,
[11] 5-fluoro-3-(2-(6-(naphthalen-1-ylamino)-1-oxoisoindolin-2-yl)butanamido)-4-oxopentanoic acid,
[12] 5-fluoro-3-(2-(6-(2-fluoro-4-(methylsulfonyl)phenyl)-1 -oxoisoindolin-2-yl)butanamido)-4-oxopentanoic acid,
[13] 5-fluoro-3-(2-(6-(6-methoxypyridin-3-yl)-1-oxoisoindolin-2-yl)butanamido)-4-oxopentanoic acid,
[14] 5-fluoro-4-oxo-3-(2-(1-oxo-6-(4-(trifluoromethyl)phenyl)isoindolin-2-yl)butanamido)pentanoic acid,
[15] 3-(2-(6-(3,6-dihydro-2H-pyran-4-yl)-1-oxoisoindolin-2-yl)butanamido)-5-fluoro-4-pentanoic acid,
[16] 3-(2-(6-benzo[b]thiophen-3-yl)-1-oxoisoindolin-2-yl)butanamido)-5-fluoro-4-oxopentanoic acid,
[17] 4-(2-(1-((1-carboxy-4-fluoro-3-oxobutan-2-yl)amino)-1-oxobutan-2-yl)-3-oxoisoindolin-5-yl)pentanoic acid,
[18] 3-(2-(6-(4-acetylphenyl)-1-oxoisoindolin-2-yl)butanamido-5-fluoro-4-oxopentanoic acid,
[19] 3-(2-(6-(4-(benzo[d]isothiazol-3-yl)piperazin-1-yl)-1-oxoisoindolin-2-yl)butanamido)-5-fluoro-4-oxopentanoic acid,
[20] 5-fluoro-4-oxo-3-(2-(1-oxo-6-(6-(trifluoromethyl)pyridin-3-yl)isoindolin-2-yl)butanamido)pentanoic acid,
[21] (3-(2-(6-(2-aminopyrimidin-5-yl)-1-oxoisoindolin-2-yl)butanamido)-5-fluoro-4-oxopentanoic acid,
[22] 3-(2-(6-(benzofuran-3-yl)-1-oxoisoindolin-2-yl)butanamido)-5-fluoro-4-oxopentanoic acid,
[23] 3-(2-(6-(2-aminopyridin-4-yl)-1-oxoisoindolin-2-yl)butanamido)-5-fluoro-4-oxopentanoic acid,
[24] 5-fluoro-3-(2-(6-(1-methyl-1H-pyrazol-4-yl)-1-oxoisoindolin-2-yl)butanamido)-4-oxopentanoic acid,
[25] 5-fluoro-3-(2-(6-(1-methyl-1H-pyrazol-3-yl)-1-oxoisoindolin-2-yl)butanamido)-4-oxopentanoic acid,
[26] 3-(2-(6-benzofuran-5-yl)-1-oxoisoindolin-2-yl)butanamido)-5-fluoro-4-oxopentanoic acid,
[27] 3-(2-(6-(2,3-dihydrobenzo[b][1,4]dioxin-6-yl)-1-oxoisoindolin-2-yl)butanamido)-5-fluoro-4-oxopentanoic acid,
[28] 5-fluoro-4-oxo-3-(2-(1-oxo-6-(thiophen-3-yl)isoindolin-2-yl)butanamido)pentanoic acid,
[29] 5-fluoro-3-(2-(6-(isoquinolin-4-yl)-1-oxoisoindolin-2-yl)butanamido)-4-oxopentanoic acid,
[30] 5-fluoro-4-oxo-3-(2-(1 -oxo-6-(quinolin-6-yl)isoindolin-2-yl)butanamido)pentanoic acid,
[31] 5-fluoro-4-oxo-3-(2-(1 -oxo-6-(quinolin-4-yl)isoindolin-2-yl)butanamido)pentanoic acid,
[32] 3-(2-(6-(benzo[d]thiazol-5-yl)-1-oxoisoindolin-2-yl)butanamido)-5-fluoro-4-oxopentanoic acid,
[33] 5-fluoro-4-oxo-3-(2-(1-oxo-6-(pyrazolo[1,5-a]pyridin-3-yl)isoindolin-2-yl)butanamido)pentanoic acid,
[34] 5-fluoro-4-oxo-3-(2-(1-oxo-6-(quinolin-5-yl)isoindolin-2-yl)butanamido)pentanoic acid,
[35] 5-fluoro-3-(2-(6-(isoquinolin-8-yl)-1-oxoisoindolin-2-yl)butanamido)-4-oxopentanoic acid,
[36] 5-fluoro-3-(2-(6-(isoquinolin-5-yl)-1-oxoisoindolin-2-yl)butanamido)-4-oxopentanoic acid,
[37] 5-fluoro-4-oxo-3-(2-(1-oxo-6-(quinolin-3-yl)isoindolin-2-yl)butanamido)pentanoic acid,
[38] 5-fluoro-4-oxo-3-(2-(1-oxo-6-(2-oxo-2,3-dihydro-1H-benzo[d]imidazol-5-yl)isoindolin-2-yl)acetamido)pentanoic acid,
[39] 3-(2-(6-(benzofuran-3-yl)-1-oxoisoindolin-2-yl)acetamido)-5-fluoro-4-oxopentanoic acid,
[40] 3-(2-(6-(benzo[b]thiophen-3-yl)-1-oxoisoindolin-2-yl)acetamido)-5-fluoro-4-oxopentanoic acid,
[41] 3-(2-(6-(benzofuran-5-yl)-1-oxoisoindolin-2-yl)acetamido)-5-fluoro-4-oxopentanoic acid,
[42] 3-((*S*)-2-(6-(2,3-dihydrobenzo[b][1,4]dioxin-6-yl)-1-oxoisoindolin-2-yl)-3-methylbutanamido)-5-fluoro-4-oxopentanoic acid,
[43] 5-fluoro-3-((*S*)-3-methyl-2-(1-oxo-6-(pyrrolidin-1-yl)isoindolin-2-yl)butanamido)4-oxopentanoic acid,
[44] 3-((*S*)-2-(6-(benzofuran-3-yl)-1-oxoisoindolin-2-yl)-3-methylbutanamido)-5-fluoro-4-oxopentanoic acid,
[45] 5-fluoro-3-((*S*)-3-methyl-2-(1-oxo-6-(2-oxo-2,3-dihydro-1H-benzo[d]imidazol-5-yl)isoindolin-2-yl)butanamido)-4-oxopentanoic acid,

[46] 5-fluoro-4-oxo-3-(2-(1-oxo-6-(pyrrolidin-1-yl)isoindolin-2-yl)propanamido)pentanoic acid,

[47] 5-fluoro-4-oxo-3-(2-(1-oxo-6-(2-oxo-2,3-dihydro-1H-benzo[d]imidazol-5 - yl)isoindolin-2-yl)propanamido)pentanoic acid,

[48] 3-(2-(6-(2,3-dihydrobenzo[b][1,4]dioxin-6-yl)-1-oxoisoindolin-2-yl)propanamido-5-fluoro-4-oxopentanoic acid,

[49] 3-(2-(6-(benzofuran-3-yl)-1-oxoisoindolin-2-yl)propanamido)-5-fluoro-4-oxopentanoic acid,

[50] (*S*)-3-((*S*)-2-(6-(benzofuran-3-yl)-1-oxoisoindolin-2-yl)butanamido-4-oxo-5-(2,3,5,6-tetrafluorophenoxy)pentanoic acid,

[51] (*S*)-3-((*S*)-2-(6-(2,3-dihydrobenzo[b][1,4]dioxin-6-yl)-1-oxoisoindolin-2-yl)butanamido)-4-oxo-5-(2,3,5,6-tetrafluorophenoxy)pentanoic acid,

[52] (*S*)-4-oxo-3-((*S*)-2-(1-oxo-6-(2-oxo-2,3-dihydro-1H-benzo[d]imidazol-5-yl)isoindolin-2-yl)butanamido)-5-(2,3,5,6-tetrafluorophenoxy)pentanoic acid,

[53] (*S*)-4-oxo-3-((*S*)-2-(1-oxo-6-(pyrrolidin-1-yl)isoindolin-2-yl)butanamido)-5-(2,3,5,6-tetrafluorophenoxy)pentanoic acid,

[54] (*R*)-3-((*S*)-2-(6-(2,3-dihydrobenzo[b][1,4]dioxin-6-yl)-1-oxoisoindolin-2-yl)butanamido)-4-oxo-5-(2,3,5,6-tetrafluorophenoxy)pentanoic acid,

[55] 3-((*S*)-2-(6-(2,3-dihydrobenzo[b][1,4]dioxin-6-yl)-1-oxoisoindolin-2-yl)butanamido)-5-fluoro-4-oxopentanoic acid,

[56] 5-fluoro-4-oxo-3-((*S*)-2-(1-oxo-6-(2-oxo-2,3-dihydro-1H-benzo[d]imidazol-5-yl)isoindolin-2-yl)butanamido) pentanoic acid,

[57] 3-((*S*)-2-(5-(benzofuran-3-yl)-1-oxoisoindolin-2-yl)butanamido)-5-fluoro-4-oxopentanoic acid,

[58] 3-((*S*)-2-(5-(2,3-dihydrobenzo[b][1,4]dioxin-6-yl)-1-oxoisoindolin-2-yl)butanamido)-5-fluoro-4-oxopentanoic acid,

[59] 3-((*S*)-2-(5-(benzo[b]thiophen-3-yl)-1-oxoisoindolin-2-yl)butanamido)-5-fluoro-4-oxopentanoic acid,

[60] 3-((*S*)-2-(5-(benzofuran-5-yl)-1-oxoisoindolin-2-yl)butanamido)-5-fluoro-4-oxopentanoic acid,

[61] 5-fluoro-3-((*S*)-2-(5-(naphthalen-1-yl)-1-oxoisoindolin-2-yl)butanamido)-4-oxopentanoic acid,

[62] 5-fluoro-4-oxo-3-((*S*)-2-(1-oxo-5-(2-(trifluoromethyl)-5,6-dihydroimidazo[1,2-a]pyrazin-7(8H)-yl)isoindolin-2-yl) butanamido)pentanoic acid,

[63] 3 -((*S*)-2-(5 -(4-(benzo [d] isothiazol-3-yl)piperazin-1-yl)-1-oxoisoindolin-2-yl)butanamido)-5-fluoro-4-oxopentanoic acid, and

[64] 5-fluoro-4-oxo-3-((*S*)-2-(1-oxo-5-(pyrrolidin-1-yl)isoindolin-2-yl)butanamido)pentanoic acid.

This specification uses the following definitions when defining the compound of Formula I unless specifically defined.

The term "alkyl" refers to a straight or branched chain hydrocarbonyl group, preferably $C_1$-$C_{12}$ alkyl. Examples of alkyl include, but are not limited to, methyl, ethyl, *n*-propyl, *iso*propyl, *n*-butyl, *iso*-butyl, *tert*-butyl, *n*-pentyl, *iso*-pentyl, *n*-hexyl, 3-methylhexyl, 2,2-dimethylpentyl, 2,3-dimethylpentyl, *n*-heptyl, *n*-octyl, *n*-nonyl, *n*-decyl, *n*-undecyl and *n*-dodecyl, etc..

The term "halogen" or "halo" refers to fluorine (F), chlorine (Cl), bromine (Br) or iodine (I).

The term "alkoxy" means an alkyloxy with 1 to 10 carbon atoms, unless otherwise defined.

The term "haloalkyl" and "haloalkoxy" means alkyl or alkoxy substituted with one or more halogen atoms.

The term "heteroatom" means N, O or S.

The term "aryl" means aromatic hydrocarbon, preferably $C_5$-$C_{12}$ aryl, more preferably $C_6$-$C_{10}$ aryl. For example, aryl includes, but are not limited to, phenyl, naphthyl, tetrahydronaphthyl, etc..

The term "heteroaryl" or "aromatic heterocycle" means a 3- to 12-membered, more preferably 5- to 10-membered aromatic hydrocarbon forming a single or fused cyclic ring that has one or more heteroatoms selected from N, O and S as ring atoms, and that can be fused with a benzo or $C_3$-$C_8$ cycloalkyl. For example,, heteroaryl includes, but are not limited to, pyridinyl, pyrimidinyl, pyridazinyl, pyrazinyl, oxadiazolyl, isoxadiazolyl, tetrazolyl, triazolyl, indolyl, indazolyl, isoxazolyl, oxazolyl, thiazolyl, isothiazolyl, furanyl, benzofuranyl, imidazolyl, thiophenyl, benzthiazole, benzimidazole, quinolinyl, indolinyl, tetrahydropyridinyl, pyrazolyl, dihydropyranyl, etc..

A non-aromatic heterocyclic ring is a non-aromatic carbocyclic ring having one or more heteroatom, such as N, O or S, within the ring. The ring may be 5, 6, 7 or 8-membered and/or fused to another ring such as a cycloalkyl or aromatic ring. Examples of such compounds include 3-1H-benzimidazol-2-one, 3-1-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoic acid, benzotriazol-1-yl, benzopyrrolidine, benzopiperidine, benzoxolane, benzothiolane and benzothiane, etc..

Arylalkyl, alkylaryl and heteroarylalkyl refer to a group formed by combining aryl and alkyl or heteroaryl and alkyl as defined above, and include, for example, benzyl, thiophene methyl, pyrimidine methyl etc, but are not limited thereto.

The aryl groups include polycyclic aromatic ring systems, wherein a carbocyclic aromatic ring or heteroaryl ring is fused with one or more other rings. Examples of such compounds include naphthyl (naphthalenyl), tetrahydronaphthyl, benzimidazolyl, benzothienyl, benzofuranyl, indolyl, quinolinyl, isoquinolinyl, benzothiazolyl, benzooxazolyl, benzimidazolyl, isoquinolinyl, isoindolyl, acridinyl, benzoisoxazolyl, dihydrobenzodioxynyl, oxo-dihydrobenzoimidazolyl, benzothiophenyl, pyrazolopyridinyl or dihydroimidazopyrazinyl. Also included within the scope of the term "aryl" as used herein are compounds having one or more carbocyclic aromatic and/or heteroaryl rings fused to a cycloalkyl or non-aromatic heterocyclic ring, e.g., indanyl or tetrahydrobenzopyranyl.

The compound represented by Formula I according to the present invention can be prepared and used in the form of prodrugs, hydrates, solvates and pharmaceutically acceptable salts to enhance *in vivo* absorption or increase solubility, so the prodrugs, hydrates, solvates and pharmaceutically acceptable salts are also within the scope of the present invention. Furthermore, the compounds represented by Formula I have chiral carbons, so that there are stereoisomers thereof, and these stereoisomers are also included within the scope of the present invention.

The term "prodrug" refers to a substance that is transformed *in vivo* into a parent drug. Prodrugs are often used because, in some cases, they are easier to administer than the parent drug. For example, they may be bioavailable by oral administration, whereas the parent drug may not be. Prodrugs may also have improved solubility in pharmaceutical compositions than the parent drug. For example, a prodrug may be an *in vivo* hydrolysable ester of the compound according to the present invention and a pharmaceutically acceptable salt thereof. Another example of a prodrug may be a short peptide (poly-amino acid) in which the peptide is coupled to an acid group that is metabolically converted to reveal the active site.

The term "hydrate" refers to a compound of the present invention or a salt thereof having a stoichiometric or non-stoichiometric amount of water bound by non-covalent intermolecular forces.

The term "solvate" refers to a compound of the present invention or a salt thereof having a stoichiometric or non-stoichiometric amount of solvent bound by non-covalent intermolecular forces. Preferred solvents therefor include solvents that are volatile, non-toxic, and/or suitable for administration to humans.

The term "isomer" refers to a compound of the present invention or a salt thereof that has the same chemical formula or molecular formula but is structurally or sterically different. Such isomers include both structural isomer such as tautomer, and stereoisomers such as R or S isomers with asymmetric carbon center and geometric isomers (trans, cis). All of these isomers and their mixtures thereof are also included within the scope of the present invention.

The term "pharmaceutically acceptable salt" refers to a salt form of a compound that does not cause serious irritation to the organism to which the compound is administered and does not impair the biological activity and physical properties of the compound. The pharmaceutical salts include an acid addition salt formed by an acid having a pharmaceutically acceptable anion and forming a non-toxic acid addition salt, for example, inorganic acids such as hydrochloric acid, sulfuric acid, nitric acid, phosphoric acid, hydrobromic acid, hydrogen iodide, etc., organic carbon acids such as tartaric acid, formic acid, citric acid, acetic acid, trichloroacetic acid, trifluoroacetic acid, gluconic acid, benzoic acid, lactic acid, fumaric acid, malic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline and triethylamine etc. The compound of Formula I according to the present invention can also be converted into its salt by conventional methods.

The present invention also provides a method of preparing a compound of Formula I, comprising the following steps.

(1) Preparing a compound of Formula I-2 by reacting a compound of Formula 1-1 below with one of the compounds of $R^3$-$B(OH)_2$, $R^3$-H, and Formula I-c below

This step may be a Suzuki coupling reaction in which 1) an aryl bromide compound of Formula I-1 below is reacted with $R^3$-$B(OH)_2$ or a pinacolboron compound of Formula I-c below to obtain an aryl-aryl compound. The catalyst used at this time may be a catalyst commonly used in Suzuki coupling reactions, for example, a palladium catalyst may be used, but is not limited thereto. In addition, the reaction can be carried out at a reaction temperature conventionally used in Suzuki coupling reactions using a solvent conventionally used in Suzuki coupling reactions, for example, but not limited to, a toluene or 1,4-dioxane solvent at elevated temperature (80 - 110 °C) for a appropriate reaction time (e.g., 10 hours or so). Additionally, this step may be 2) a Birkwald reaction in which the compound of Formula I-1 is reacted with an amine compound to obtain an aryl-amine type compound. The catalyst used at this time may be a catalyst commonly used in the Birkwald reaction, for example, a palladium catalyst may be used, but is not limited thereto. In addition, the reaction may be carried out at a reaction temperature conventionally used in the Burkhardt reaction using a solvent conventionally used in

the Burkhardt reaction, for example, but not limited to, a toluene solvent at elevated temperature (e.g., 80 °C) for a appropriate reaction time (e.g., 10 hours or so).

(2) Hydrolyzing the compound of Formula I-2 below to prepare the compound of Formula I-3 below

This step is a hydrolysis reaction in which the compound of Formula I-2 below is reacted with lithium hydroxide. The solvent used at this time can be a solvent conventionally used in hydrolysis reactions, for example, a tetrahydrofuran-aqueous solution mixed solvent, etc., and the compound of Formula I-3 can be obtained by reacting at a appropriate temperature (e.g., room temperature) for a appropriate reaction time (e.g., about 24 hours) to obtain the compound of Formula I-3.

(3) Preparing a compound of Formula 1-5 below by reacting the compound of Formula I -3 below with a compound of Formula 1-4 below

This step is a reaction in which a compound of Formula I-3 having a carboxylic acid group is reacted with a compound of Formula I-4 having an amine group to produce an amide bond. The solvent used in this step may be a solvent conventionally used in amide bond reactions, for example, a DMF solvent, and the like, and the reaction may be carried out at a appropriate temperature (for example, room temperature).

(4) Hydrolyzing the compound of Formula I-5 below to prepare the compound of Formula I-6 below

This step is a hydrolysis reaction in which the compound of Formula I-5 below is reacted with lithium hydroxide. The solvent used in this step may be a solvent conventionally used in hydrolysis reactions, such as a tetrahydrofuran-aqueous solution mixed solvent, and the like, and the compound of Formula I-6 can be obtained by reacting at a appropriate temperature (e.g., room temperature) for a appropriate reaction time (e.g., about 24 hours) to obtain the compound of Formula I-6.

(5) Acidifying the compound of Formula I-6 below

This step is a deprotection grouping reaction in which a compound of Formula I-6 below in the form of dimethyl chital is acid treated to obtain a ketone compound. The solvent used in this step may be any solvent conventionally used in deprotection grouping reactions, such as, but not limited to, an aqueous solution of hydrochloric acid.

<Formula I-1>

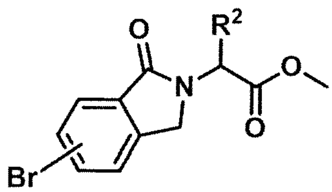

<Formula I-c>

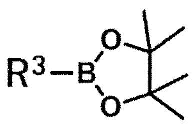

<Formula I-2>

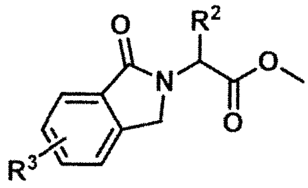

<Formula I-3>

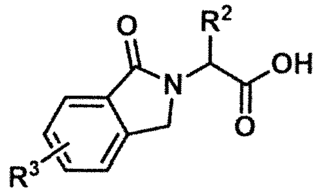

<Formula I-4>

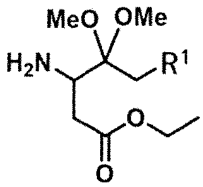

<Formula I-5>

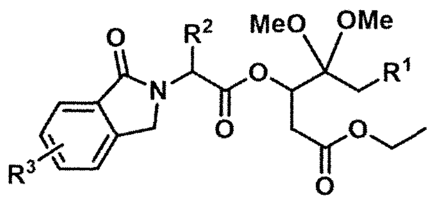

<Formula I-6>

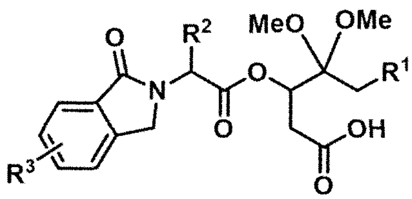

In the above formula, $R^1$, $R^2$, and $R^3$ are the same as defined in Formula I above.

In one embodiment, the compound of Formula 1-1 may be obtained by reacting the compound of Formula I-a below with the compound of Formula I-b below.

<Formula I-a>

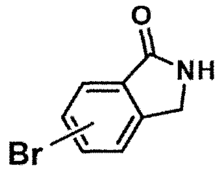

<Formula I-b>

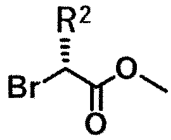

In the above formula, the definition of $R^2$ is the same as the definition of Formula I above.

Schemes 1 to 64 of the Examples are illustrated as a method for preparing the compound of Formula I of the present invention, and the above preparing process does not limit the method of preparing the compound of Formula I according to the present invention. It is obvious that the preparation methods of Schemes 1 to 64 are illustrative only and can be easily modified by those skilled in the art depending on the specific substituent.

The present invention also provides a pharmaceutical composition for preventing or treating caspase-related disease, comprising the isoindolinone derivative compound represented by Formula I, a hydrate thereof, a solvate thereof or a pharmaceutically acceptable salt thereof, as an active ingredient.

The present invention also provides a method for preventing or treating caspase-related disease, comprising: administering the isoindolinone derivative compound represented by the above Formula I , a hydrate thereof, a solvate thereof or a pharmaceutically acceptable salt thereof, to a subject in need thereof.

The present invention also provides a use of the isoindolinone derivative compound represented by the above Formula I , a hydrate thereof, a solvate thereof or a pharmaceutically acceptable salt thereof, in manufacture of a medicament for preventing or treating caspase-related disease.

In one embodiment, the caspase-related disease may be osteoarthritis or pain.

Caspase inhibitory activity was measured for the isoindolinone derivative compounds of the present invention, and it was found that they exhibited caspase inhibitory activity, either by a reduction in interleukin-1β (IL-1β) or a reduction in apoptosis.

The present invention also provides a pharmaceutical composition comprising the isoindolinone derivative compound represented by the above Formula I , a hydrate thereof, a solvate thereof or a pharmaceutically acceptable salt thereof and a pharmaceutically acceptable additive.

The additive may include a pharmaceutically acceptable carrier or diluent, each of which may be formulated according to conventional methods in the form of oral formulations such as powders, granules, tablets, capsules, suspensions, emulsions, syrups, aerosols; topicals; suppositories; and sterile injectable solutions.

The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate and mineral oil, and the like. They also include diluents or excipients such as fillers, bulking agents, binders, wetting agents, disintegrating agents, and surfactants. Oral solid dosage forms include tablets, pills, powders, granules, capsules, and the like, which may include at least one excipient, such as starch, calcium carbonate, sucrose, lactose, gelatin, and the like, and may include lubricants such as magnesium stearate and talc. Oral liquid preparations may include suspensions, oral solutions, emulsions, syrups, and the like, and may include diluents such as water and liquid paraffin, wetting agents, sweeteners, flavourings, preservatives, and the like. Parenteral preparations include sterilized aqueous solutions, non-aqueous solvents, suspensions, emulsions, creams, lyophilised preparations, and suppositories; non-aqueous solvents and suspensions include propylene glycol, polyethylene glycol, vegetable oils such as olive oil, and injectable esters such as ethylolate. Substrates for suppositories may be witepsol, macrogol, tween 61, cacao gum, laurin gum, glycerogelatin, etc..

The dosage of the active ingredient in the pharmaceutical composition of the present invention depends on the condition and weight of the patient, the extent of the disease, the formulation of the active ingredient, the route and duration of administration, and may be appropriately adjusted depending on the patient. For example, the active ingredient can be administered at a dose of 0.0001 to 1000 mg/kg per day, preferably 0.01 to 100 mg/kg, and the dose may be administered

once or in several divided doses per day. Furthermore, the pharmaceutical composition of the present invention may comprise the active ingredient from 0.001 to 90% by weight, based on the total weight of the composition.

The pharmaceutical composition of the present invention may be administered to mammals such as rats, mice, livestock, and humans by various routes, for example, orally, by dermal, intraperitoneally, rectally or intravenously, intramuscular, subcutaneous, intrauterine dura, or intracerebroventricular injection.

Hereinafter, the present disclosure is described in more detail with Examples and Experimental examples. However, the following Examples and Experimental examples are intended to illustrate the present invention, and the scope of the present invention is not limited thereto.

Example 1. 5-fluoro-4-oxo-3-(2-(1-oxo-6-phenylisoindolin-2-yl)butanamido)pentanoic acid

<Scheme 1>

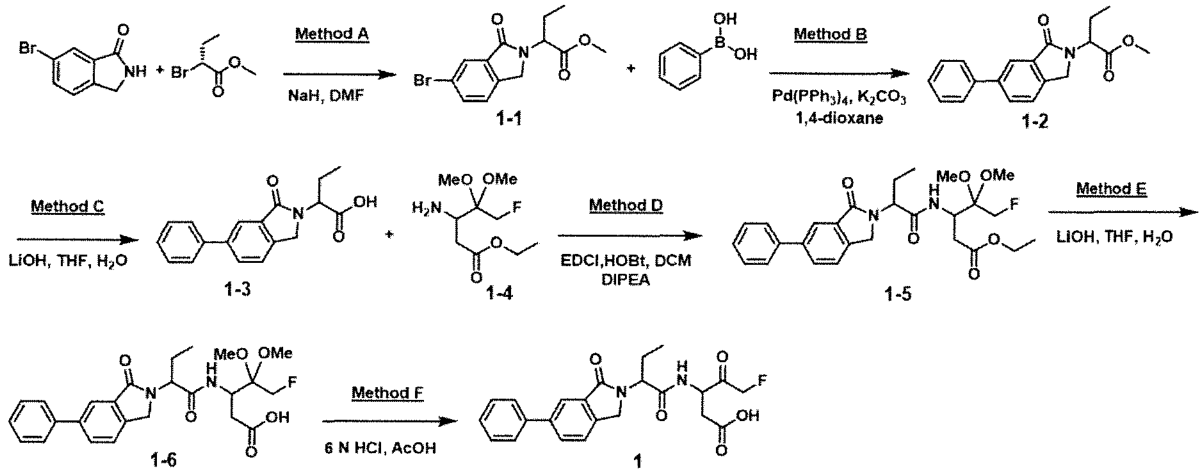


Step 1-1 (Method A): 6-Bromoindoline-1-one (500 mg, 2.36 mmol) was dissolved in N,N-dimethylformamide (10 mL), then sodium hydrogen hydride (99.4 mg, 2.59 mmol) and methyl (R)-2-bromobutanoate (426.86 mg, 2.36 mmol) were added and the stirred at room temperature for 12 hours. After completion of the reaction, water (50 mL) was added and extracted with ethyl acetate (50 mL x 2). The organic layer was dried over anhydrous sodium sulfate, filtered and concentrated, then separated using column chromatography to give the target compound 1-1 (324 mg, 44%).

Step 1-2 (Method B): Compound 1-1 (150 mg, 0.48 mmol) was dissolved in 1,4-dioxane (10 mL), then phenylboronic acid (70.3 mg, 0.58 mmol), 2 M aqueous potassium carbonate solution (0.6 mL, 1.2 mmol), and tetrakis(triphenylphosphine)palladium (27.76 mg, 0.024 mmol) were added and stirred at 110°C for 10 hours. After completion of the reaction, brine (50 mL) was added, and extracted with ethyl acetate (50 mL x 2). The organic layer was dried over anhydrous sodium sulfate, filtered, concentrated, and separated using column chromatography to give target compound **1-2** (108 mg, 72%).

Step 1-3 (Method C): Compound **1-2** (108 mg, 0.349 mmol) was dissolved in tetrahydrofuran/water (3/1, 150 mL), then lithium hydroxide (33.44 mg, 1.39 mmol) was added and stirred at room temperature for 24 hours. After completion of the reaction, the tetrahydrofuran solvent was concentrated and removed, acidified by adding 1 N aqueous hydrochloric acid (pH 1), and extracted with ethyl acetate (30 mL x 2). The organic layer was dried over anhydrous sodium sulfate, filtered, concentrated, and separated using column chromatography to give the target compound **1-3** (99 mg, 96%).

Step 1-4 (Method D): Compound **1-3** (75 mg, 0.254 mmol) was dissolved in dichloromethane (10 mL), then 1-ethyl-3-(3-dimethylaminopropyl)carbodiimide (48.68 mg, 0.254 mmol), compounds **1-4** synthesized by known methods (WO2006/090997) (59. 53 mg, 0.267 mmol), 1-hydroxybenzo triazole (34.32 mg, 0.254 mmol) and N,N-diisopropylethylamine (DIPEA, 39.48 mg, 0.305 mmol) were added and stirred at room temperature for 24 hours. After completion of the reaction, sodium hydrogen carbonate saturated aqueous solution (30 mL) was added and extracted with dichloromethane (20 mL x 2). The organic layer was dried over anhydrous sodium sulfate, filtered and concentrated, and then separated using column chromatography to give the target compounds **1-5** (93 mg, 73%).

Step 1-5 (Method E): Compound **1-5** (93 mg, 0.186 mmol) was dissolved in tetrahydrofuran/water (3/1, 50 mL), then lithium hydroxide (17.8 mg, 0.743 mmol) was added and stirred at room temperature for 24 hours. After completion of the reaction, the tetrahydrofuran solvent was removed by concentrating, then acidified by adding aqueous 1 N hydrochloric acid (pH 1), and extracted with ethyl acetate (30 mL x 2). The organic layer was dried over anhydrous sodium sulfate, filtered, concentrated, and separated using column chromatography to give the target compounds **1-6** (69 mg, 78%).

Step 1-6 (Method F): Compound **1-6** (69 mg, 0.146 mmol) was dissolved in acetic acid (2 mL), then 6 N aqueous hydrochloric acid solution (2 mL) was added and stirred at room temperature for 6 hours. After completion of the reaction, the reaction solution was diluted with water (20 mL) and extracted with ethyl acetate (30 mL x 2). The organic layer was dried over anhydrous sodium sulfate, filtered and concentrated, and then separated using column chromatography to give the target compound **1** (17 mg, 27%).

$^1H$ NMR (400 MHz, DMSO-$d_6$) d 12.96 (s, 1H), 8.01-7.82 (m, 2H), 7.80-7.61 (m, 3H), 7.61-7.20 (m, 4H), 4.86-4.31 (m, 6H), 2.79-2.22 (m, 2H), 2.23-1.64 (m, 2H), 0.94-0.67(m, 3H).

MS (ESI, LR) Calculated for $C_{23}H_{24}FN_2O_5$ ($MH^+$): 427.2, found: 427.2.

**Example 2. 5-fluoro-3-(2-(6-(naphthalen-1-yl)-1-oxoisoindolin-2-yl)butanamido)-4-oxopentanoic acid**

<Scheme 2>

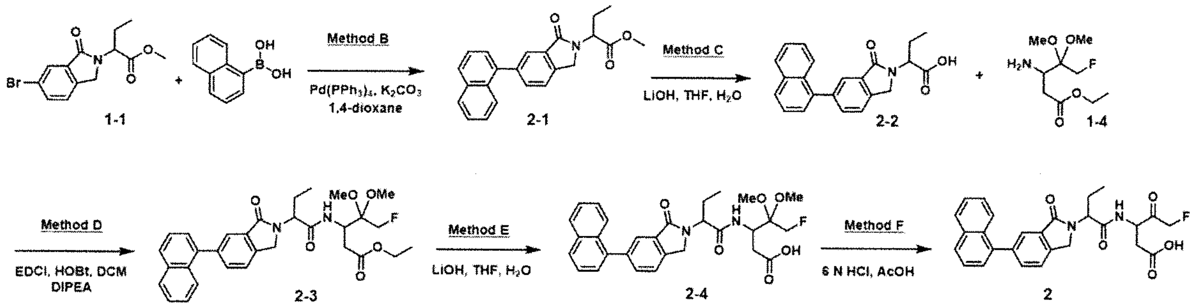


Using compound **1-1** and naphthalen-1-boronic acid as starting materials, the target compound **2** (21 mg) was obtained using Method B/C/D/E/F sequentially.

$^1H$ NMR (400 MHz, DMSO-$d_6$) d 8.88-8.76 (m, 1H), 8.01 (dd, $J$ = 13.73, 8.24 Hz, 2H), 7.81-7.65 (m, 4H), 7.64-7.43 (m, 4H), 5.28-4.39 (m, 6H), 2.74-2.42 (m, 2H), 2.21-1.70 (m, 2H), 0.95-0.70 (m, 3H).

MS (ESI, LR) Calculated for $C_{27}H_{26}FN_2O_5$ ($MH^+$): 477.2, found: 477.2.

**Example 3. 5-fluoro-4-oxo-3-(2-(1-oxo-6-(pyrrolidin-1-yl)isoindolin-2-yl)butanamido)pentanoic acid**

<Scheme 3>

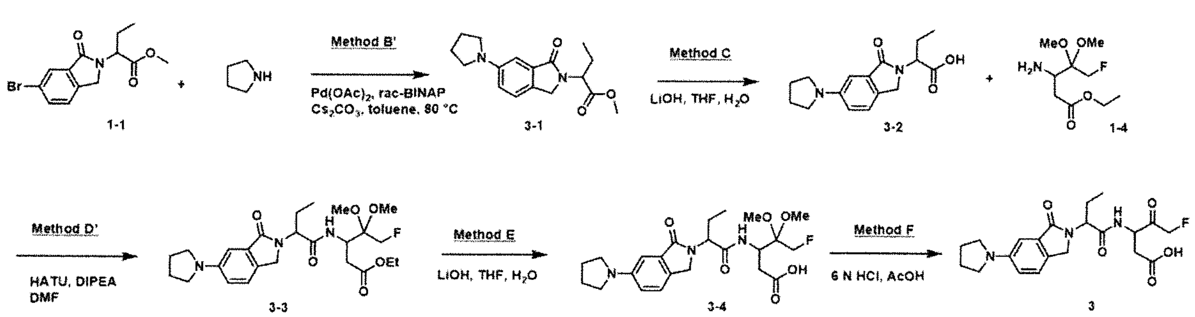


Step 3-1 (Method B'): Compound 1-1 (150 mg, 0.481 mmol) was dissolved in toluene (10 mL) in a sealed tube, then cesium carbonate (172.22 mg, 0.529 mmol), palladium acetate (1.08 mg, 0. 005 mmol), racemic-2,2'-bis(diphenylphosphino)-1,1'-binaphthyl (59.84 mg, 0.096 mmol), pyrrolidine (47.84 mg, 0.673 mmol) were added, sealed, and stirred at 80 °C for 10 hours. After completion of the reaction, it was diluted with brine (50 mL) and extracted with ethyl acetate (30 mL x 2). The organic layer was dried over anhydrous sodium sulfate, filtered, and concentrated, and then

separated using column chromatography to give the target compound **3-1** (109 mg, 75%).

Step 3-2: Using compound **3-1** (109 mg, 0.36 mmol), the target compound **3-2** (91 mg, 86%) was obtained using Method C.

Step 3-3 (Method D'): After dissolving compound **3-2** (91 mg, 0.316 mmol) in N,N-dimethylformamide (3 mL), HATU (144 mg, 0.379 mmol) and DIPEA (61.33 mg, 0.473 mmol) were added and stirred at room temperature for 10 min. To the reaction solution was added compound **1-4** (73.97 mg, 0.331 mmol) and stirred at room temperature for 24 hours. After completion of the reaction, sodium hydrogen carbonate saturated aqueous solution (30 mL) was added and extracted with ethyl acetate (40 mL x 2). The organic layer was dried over anhydrous sodium sulfate, filtered and concentrated, and then separated using column chromatography to give the target compound **3-3** (111 mg, 71%). [HATU(1-[Bis(dimethylamino)methylene]-1H-1,2,3-triazolo[4,5-b]pyridinium 3-oxide hexafluorophosphate, Hexafluorophosphate Azabenzotriazole Tetramethyl Uronium), DIPEA (N,N-Diisopropylethylamine)]

Step 3-4/3-5: Using compound **3-3** as a starting material, the target compound **3** (31 mg) was obtained using Method E/F sequentially.

$^1$H NMR (400 MHz, MeOD) d 12.46 (s, 1H), 8.81-8.44 (m, 1H), 7.40-7.20 (m, 1H), 6.84-6.60 (m, 2H), 5.35-3.87 (m, 6H), 3.40-3.09 (m, 4H), 2.78-2.34 (m, 2H), 2.05-1.57 (m, 6H), 0.90-0.65 (m, 3H).

MS (ESI, LR) Calculated for $C_{21}H_{27}FN_3O_5$ ($MH^+$): 420.2, found: 420.2.

**Example 4. 5-fluoro-4-oxo-3-(2-(1-oxo-6-(pyridin-3-yl)isoindolin-2-yl)butanamido)pentanoic acid**

<Scheme 4>

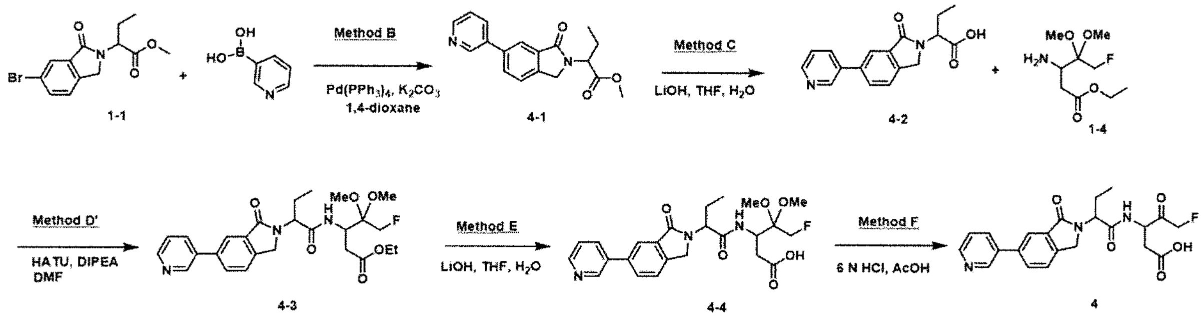


Using compound **1-1** and pyridin-3-boronic acid as starting materials, the target compound **4** (31 mg) was obtained using Method B/C/D'/E/F sequentially.

$^1$H NMR (400 MHz, DMSO-$d_6$) d 8.84 (s, 1H), 8.59-8.47 (m, 1H), 8.19-7.81 (m, 4H), 7.72 (*t, J* = 7.02 Hz, 1H), 7.55 (t, J= 7.63 Hz, 1H), 5.26-4.03 (m, 6H), 2.96-2.54 (m, 2H), 2.20-1.81 (m, 2H), 1.07-0.79 (m, 3H).

MS (ESI, LR) Calculated for $C_{22}H_{23}FN_3O_5$ ($MH^+$): 428.2, found: 428.1.

**Example 5. 5-fluoro-4-oxo-3-(2-(1-oxo-6-(1H-pyrazol-4-yl)isoindolin-2-yl)butanamido)pentanoic acid**

<Scheme 5>

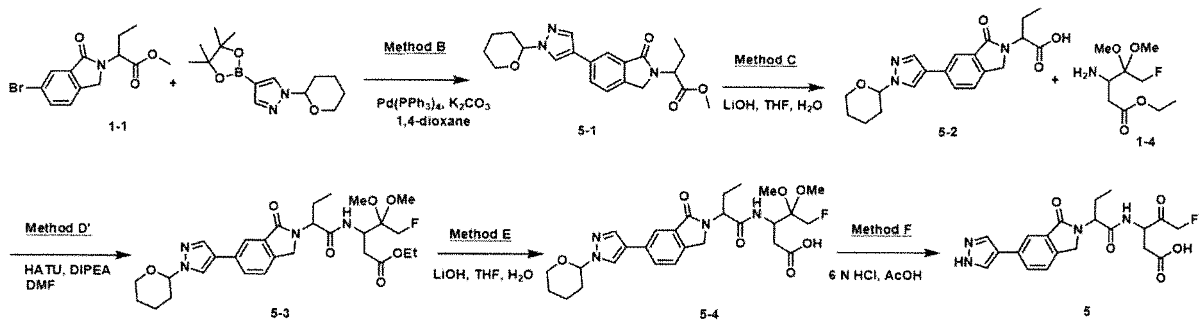

Using compound **1-1** and 1-(tetrahydro-2H-pyran-2-yl)-4-(4,4,5,5-tetramethyl-1,3,2-dioxaborolan-2-yl)-1H-pyrazole as starting materials, the target compound **5** (21 mg) was obtained using Method B/C/D7E/F sequentially.

$^1H$ NMR (400 MHz, DMSO-$d_6$) d 12.86 (s, 2H), 8.89-8.61 (m, 1H), 8.18 (s, 2H), 7.98-7.73 (m, 2H), 7.58 (d, *J*= 7.63 Hz, 1H), 5.32-4.28 (m, 6H), 2.84-2.37 (m, 2H), 2.10-1.68 (m, 2H), 0.93-0.70 (m, 3H).

MS (ESI, LR) Calculated for $C_{20}H_{22}FN_4O_5$ ($MH^+$) 417.1, found: 417.1.

**Example 6. 5-fluoro-3-(2-(6-(4-(methylsulfonyl)piperazin-1-yl)-1-oxoisoindolin-2-yl)butanamido)-4-oxopentanoic acid**

<Scheme 6>

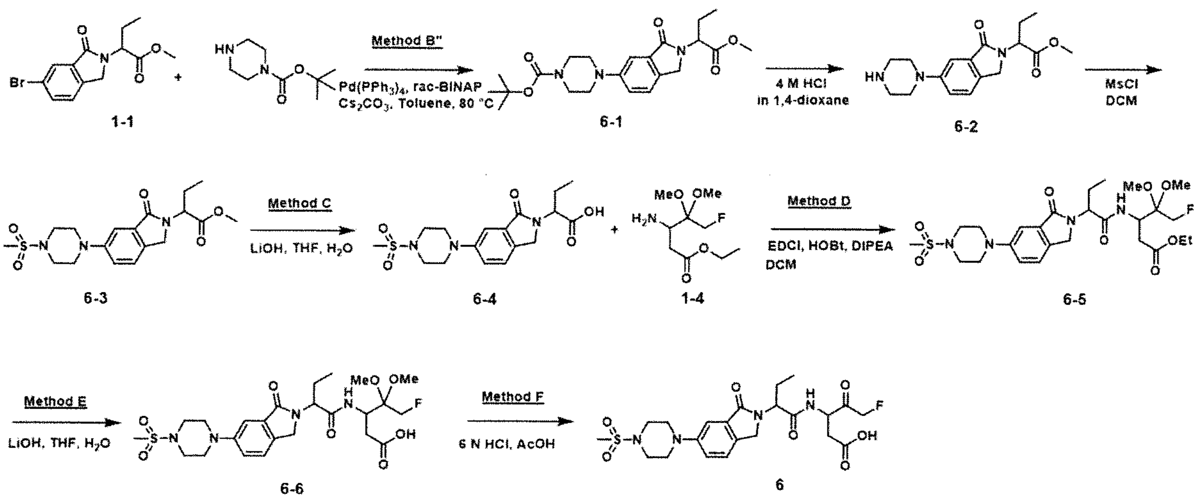


Step 6-1 (Method B"): After dissolving compound **1-1** (150 mg, 0.481 mmol) in toluene (15 mL), cesium carbonate (172.22 mg, 0.529 mmol), tetrakis(triphenylphosphine)palladium (27.76 mg, 0.024 mmol), and racemic-2,2'-bis(diphenylphosphino)-1,1'-binaphthyl (59.84 mg, 0.096 mmol), tert-butyl 1-piperazine carboxylate (47.84 mg, 0.673 mmol) were added, sealed and stirred at 80 °C for 10 hours. After completion of the reaction, it was diluted with brine (50 mL) and extracted with ethyl acetate (30 mL x 2). The organic layer was dried over anhydrous sodium sulfate, filtered, concentrated, and separated using column chromatography to give the target compound **6-1** (120 mg, 60%).

Step 6-2: After dissolving compound **6-1** (120 mg, 0.287 mmol) in ethyl acetate (5 mL), hydrogen chloride solution (4.0 M 1,4-dioxane solution, 2 mL) was added and stirred at room temperature for 12 hours. After completion of the reaction, the reaction was concentrated to give the target compound **6-2** (95 mg, 93%) as a white solid.

Step 6-3: Compound **6-2** (95 mg, 0.268 mmol) was dissolved in dichloromethane (30 mL), then methanesulfonyl chloride (33.83 mg, 0.295 mmol) and trimethylamine (54.33 mg, 0.537 mmol) were added and stirred at room temperature for 12 hours. After completion of the reaction, it was diluted with water (40 mL) and extracted with dichloromethane (3 x 50 mL). The organic layer was dried over anhydrous sodium sulfate, filtered and concentrated, and separated using column chromatography to give the target compound **6-3** (88 mg, 82%).

Step 6-4/6-5/6-6/6-7: Using compound **6-3** (88 mg, 0.223 mmol), the target compound **6** (21 mg) was obtained using Method C/D/E/F sequentially.

$^1H$ NMR (400 MHz, DMSO-$d_6$) d 8.72-8.58 (m, 1H), 7.46 (dd, *J* = 8.39, 4.20 Hz, 1H), 7.29 (d, *J* = 8.39 Hz, 1H), 7.20 (dd, *J* = 5.72, 2.29 Hz, 1H), 5.36-4.29 (m, 6H), 3.55-3.11 (m, 8H), 2.93 (s, 3H), 2.59-2.34 (m, 2H), 2.03-1.62 (m, 2H), 0.90-0.64 (m, 3H).

MS (ESI, LR) Calculated for $C_{22}H_{30}FN_4O_7S$ ($MH^+$): 513.2, found: 513.2.

**Example** 7. **5-fluoro-3-(2-(6-(1-(methylsulfonyl)-1,2,3,6-tetrahydropyridin-4-yl)-1-oxoisoindolin-2-yl)butanamido)-4-oxopentanoic acid**

<Scheme 7>

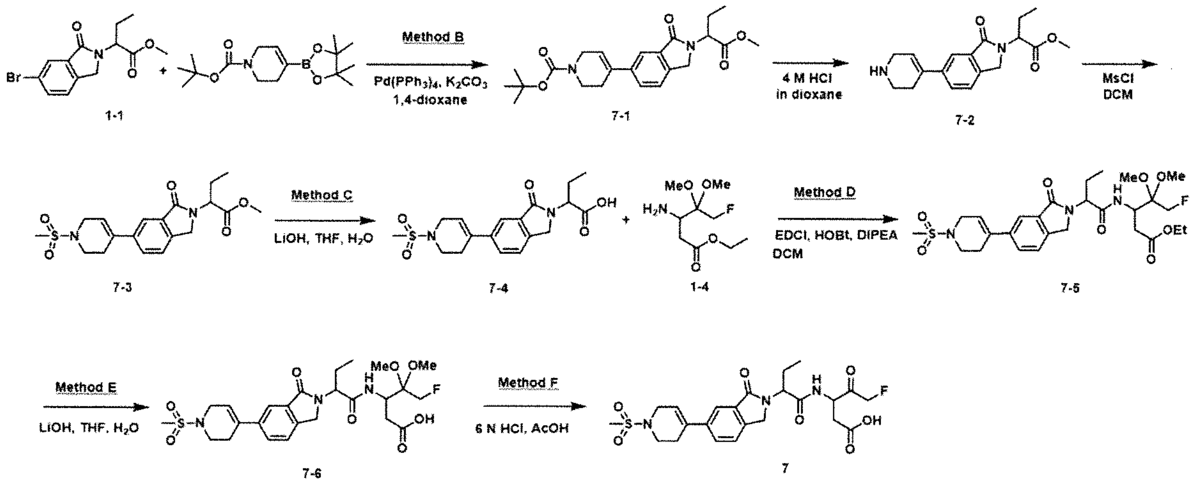


Step 7-1: Using compound **1-1** and (150 mg, 0.48 mmol), tert-butyl 4-(4,4,5,5-tetramethyl-1,3,2-dioxaborolan-2-yl)-3,6-dihydropyridin-1(2H)-carboxylate (178.3 mg, 0.58 mmol), the target compound **7-1** (124 mg, 62 %) was obtained using Method B.

Step 7-2: Compound **7-1** (124 mg, 0.297 mmol) was dissolved in ethyl acetate (5 mL), then hydrogen chloride solution (4.0 M 1,4-dioxane solution, 2 mL) was added, and the mixture was stirred at room temperature for 12 hours. After completion of the reaction, the target compound **7-2** (99 mg, 95%) was obtained by concentration.

Step 7-3: Compound **7-2** (99 mg, 0.282 mmol) was dissolved in dichloromethane (30 mL), then methanesulfonyl chloride (35.56 mg, 0.31 mmol) and triethylamine (57.11 mg, 0.564 mmol) were added and stirred at room temperature for 12 hours. After completion of the reaction, it was diluted with water (40 mL) and extracted with dichloromethane (3 x 50 mL). The organic layer was dried over anhydrous sodium sulfate, filtered, concentrated, and separated using column chromatography to give the target compound **7-3** (89 mg, 80%).

Step 7-4/7-5/7-6/7-7: Using compound **7-3** (89 mg, 0.227 mmol), the target compound **7** (19 mg) was obtained using Method C/D/E/F sequentially.

$^1$H NMR (400 MHz, DMSO-$d_6$) d 12.49 (s, 1H), 8.87-8.69 (m, 1H), 7.79-7.50 (m, 3H), 6.31 (s, 1H), 5.34-4.23 (m, 6H), 3.88 (s, 2H), 3.40 (t, *J*= 5.72 Hz, 2H), 2.95 (s, 3H), 2.87-2.35 (m, 4H), 2.10-1.64 (m, 2H), 0.93-0.66 (m, 3H).

MS (ESI, LR) Calculated for $C_{23}H_{29}FN_3O_7S$ ($MH^+$): 510.2, found: 510.1.

**Example 8. 5-fluoro-4-oxo-3-(2-(1-oxo-6-(2-oxo-2,3-dihydro-1H-benzo[d]imidazol-5-yl)isoindolin-2-yl)butanamido)pentanoic acid**

<Scheme 8>

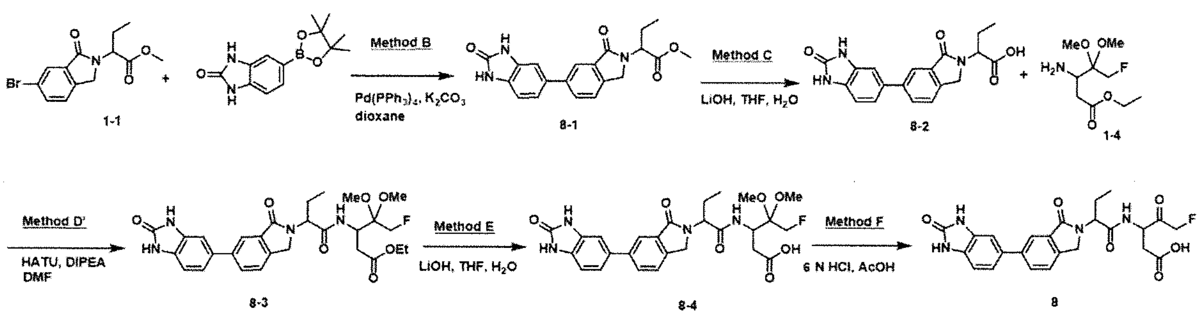


Using compound **1-1** and 5-(4,4,5,5-tetramethyl-1,3,2-dioxaborolan-2-yl)-1,3-dihydro-2H-benzo[d]imidazol-2-one as starting materials, the target compound **8** (19 mg) was obtained using Method B/C/D'/E/F sequentially.

$^1$H NMR (400 MHz, DMSO-$d_6$) d 10.80-10.64 (m, 2H), 8.80-8.64 (m, 1H), 7.90-7.74 (m, 2H), 7.70-7.58 (m, 1H), 7.28 (d, *J*= 7.93 Hz, 1H), 7.20 (s, 1H), 7.01 (d, J= 7.93 Hz, 1H), 5.25-4.32 (m, 6H), 2.70-2.39 (m, 2H), 2.14-1.64 (m, 2H),

0.95-0.68 (m, 3H).

MS (ESI, LR) Calculated for $C_{24}H_{23}FN_4O_6$ ($MH^+$): 483.2, found: 483.0.

**Example 9. 3-(2-(6-bromo-1-oxoisoindolin-2-yl)butanamido)-5-fluoro-4-oxopentanoic acid**

<Scheme 9>

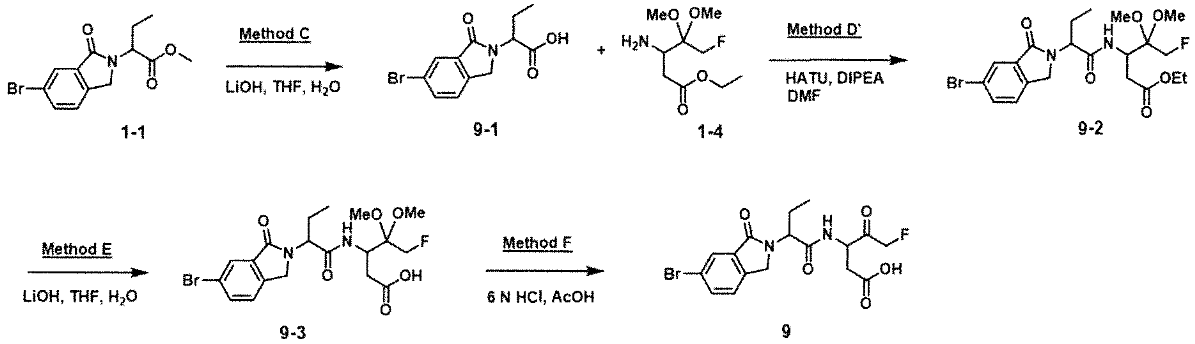


Using compound 1-1 as a starting material, the target compound 9 (23 mg) was obtained using Method C/D'/E/F sequentially.

$^1H$ NMR (400 MHz, DMSO-$d_6$) d 12.51 (s, 1H), 8.89-8.67 (m, 1H), 7.94-7.73 (m, 2H), 7.65-7.50 (m, 1H), 5.36-4.23 (m, 6H), 2.90-2.34 (m, 2H), 2.10-1.67 (m, 2H), 0.94-0.71 (m, 3H).

MS (ESI, LR) Calculated for $C_{17}H_{19}BrFN_2O_5$ ($MH^+$): 429.0, found: 429.0.

**Example 10. 5-fluoro-4-oxo-3-(2-(1-oxo-6-(2-(trifluoromethyl)-5,6-dihydroimidazo[1,2-a]pyrazin-7(8H)-yl)isoindolin-2-yl)butanamido)pentanoic acid**

<Scheme 10>

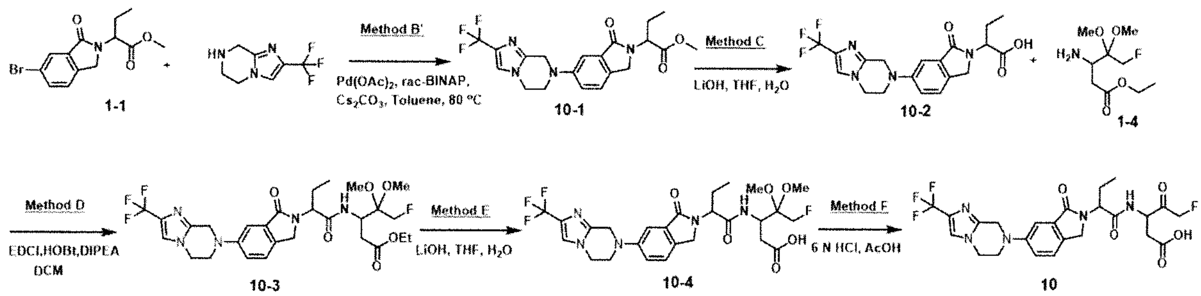


Using compound **1-1** and 2-(trifluoromethyl)-5,6,7,8-tetrahydroimidazo[1,2-a]pyrazine as starting materials, the target compound **10** (31 mg) was obtained using Method B'/C/D/E/F sequentially.

$^1H$ NMR (400 MHz, DMSO-$d_6$) d 12.58 (s, 1H), 8.92-8.66 (m, 1H), 7.79 (s, 1H), 7.64-7.14 (m, 3H), 5.39-3.67 (m, 12H), 2.97-2.24 (m, 2H), 2.14-1.62 (m, 2H), 1.05-0.60 (m, 3H).

MS (ESI, LR) Calculated for $C_{24}H_{26}F_4N_5O_5$ ($MH^+$): 540.2, found: 540.1.

**Example 11. 5-fluoro-3-(2-(6-(naphthalen-1-ylamino)-1-oxoisoindolin-2-yl)butanamido)-4-oxopentanoic acid**

<Scheme 11>

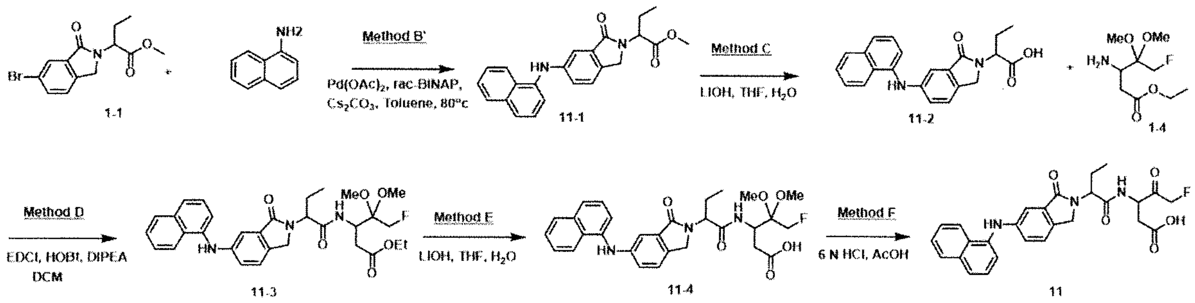


Using compound **1-1** and 1-naphthylamine as starting materials, the target compound 11 (18 mg) was obtained using Method B'/C/D/E/F sequentially.

$^1H$ NMR (400 MHz, DMSO-$d_6$) d 12.18 (s, 1H), 8.55-8.41 (m, 1H), 8.19 (d, *J* = 8.77 Hz, 1H), 8.12 (d, *J* = 8.39 Hz, 1H), 7.92 (d, *J* = 8.01 Hz, 1H), 7.61 (d, *J* = 8.01 Hz, 1H), 7.56-7.39 (m, 4H), 7.35 (d, *J* = 7.25 Hz, 1H), 7.28 (d, *J* = 7.63 Hz, 1H), 7.14 (s, 1H), 4.77-4.17 (m, 6H), 2.70-2.25 (m, 2H), 1.95-1.58 (m, 2H), 0.79 (t, *J* = 7.25 Hz, 3H).

MS (ESI, LR) Calculated for $C_{27}H_{27}FN_3O_5$ ($MH^+$): 492.2, found: 492.1.

**Example 12. 5-fluoro-3-(2-(6-(2-fluoro-4-(methylsulfonyl)phenyl)-1-oxoisoindolin-2-yl)butanamido)-4-oxopentanoic acid]**

<Scheme 12>

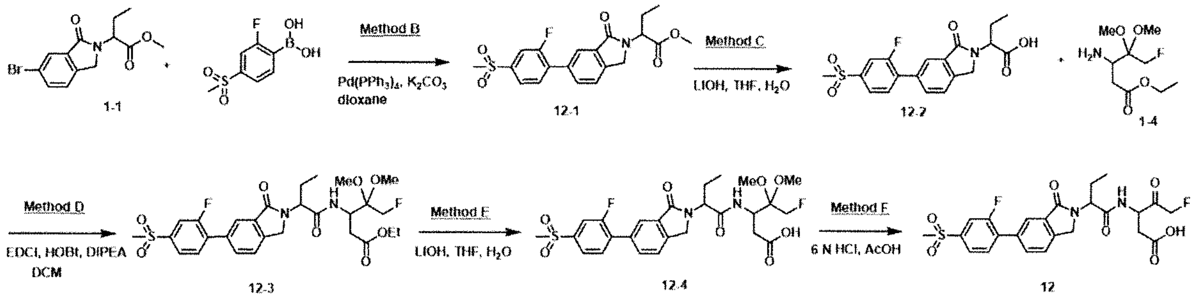


**Using compound 1-1 and (2-fluoro-4-(methylsulfonyl)phenyl)boronic acid as starting** materials, the target compound **12** (32 mg) was obtained using Method B/C/D/E/F sequentially.

$^1H$ NMR (400 MHz, DMSO-$d_6$) d 7.99-7.72 (m, 4H), 7.67-7.48 (m, 3H), 5.34-4.23 (m, 6H), 3.34 (s, 3H), 2.91-2.40 (m, 2H), 2.15-1.68 (m, 2H), 0.96-0.75 (m, 3H).

MS (ESI, LR) Calculated for $C_{24}H_{25}F_2N_2O_7S$ ($MH^+$): 523.1, found: 523.1.

**Example 13. 5-fluoro-3-(2-(6-(6-methoxypyridin-3-yl)-1-oxoisoindolin-2-yl)butanamido)-4-oxopentanoic acid**

<Scheme 13>

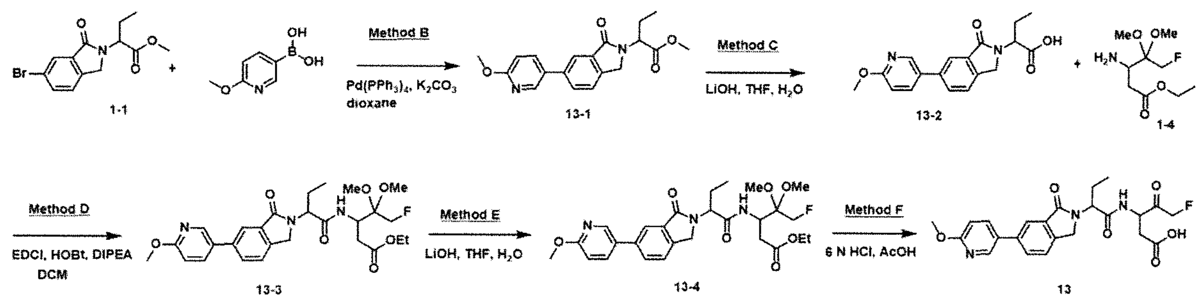


Using compound **1-1** and (6-methoxypyridin-3-yl)boronic acid as starting materials, the target compound **13** (31 mg) was obtained using Method B/C/D/E/F sequentially.

$^1$H NMR (400 MHz, DMSO-$d_6$) d 12.53 (s, 1H), 8.54 (s, 1H), 8.10 (d, J=8.85 Hz, 1H), 7.95-7.80 (m, 2H), 7.74-7.50 (m, 2H), 6.93 (d, *J*= 8.54 Hz, 1H), 5.35-4.22 (m, 6H), 3.90 (s, 3H), 2.91-2.37 (m, 2H), 2.14-1.67 (m, 2H), 0.94--0.73 (m, 3H).

MS (ESI, LR) Calculated for $C_{23}H_{25}FN_3O_6$ ($MH^+$): 458.2, found: 458.1.

**Example 14. 5-fluoro-4-oxo-3-(2-(1-oxo-6-(4-(trifluoromethyl)phenyl)isoindolin-2-yl)butanamido)pentanoic acid**

<Scheme 14>

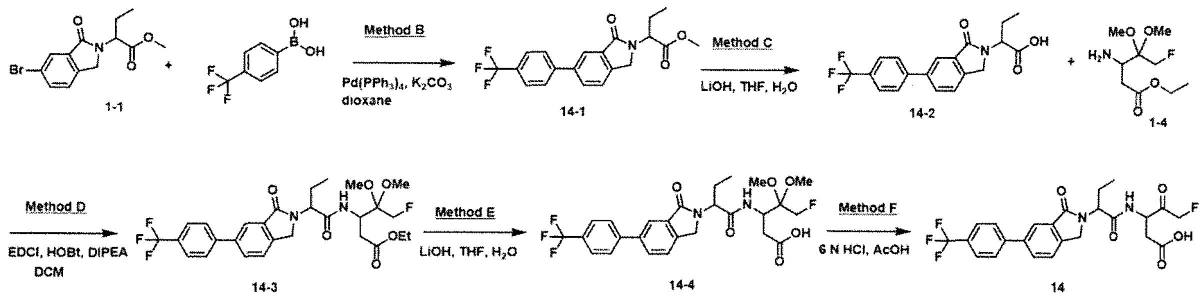


Using compound **1-1** and (4-(trifluoromethyl)phenyl)boronic acid as starting materials, the target compound **14** (28 mg) was obtained using Method B/C/D/E/F sequentially.

$^1$H NMR (400 MHz, DMSO-$d_6$) d 12.93 (s, 1H), 8.18-7.37 (m, 8H), 5.33-4.30 (m, 6H), 2.78-2.23 (m, 2H), 2.25-1.62 (m, 2H), 1.00-0.66 (m, 3H).

MS (ESI, LR) Calculated for $C_{24}H_{23}F_4N_2O_5$ ($MH^+$): 495.1, found: 495.1.

**Example 15. 3-(2-(6-(3,6-dihydro-2H-pyran-4-yl)-1-oxoisoindolin-2-yl)butanamido)-5-fluoro-4-oxopentanoic acid**

<Scheme 15>

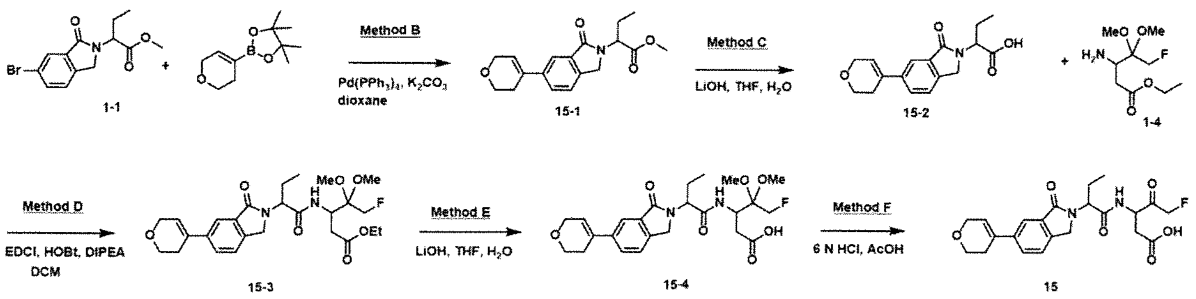


Using compound **1-1** and 2-(3,6-dihydro-2H-pyran-4-yl)-4,4,5,5-tetramethyl-1,3,2-dioxaborolan as starting materials, the target compound 15 (18 mg) was obtained using Method B/C/D/E/F sequentially.

$^1$H NMR (400 MHz, DMSO-$d_6$) d 7.79-7.69 (m, 1H), 7.67-7.50 (m, 3H), 6.86 (s, 1H), 4.87-3.78 (m, 6H), 3.75-3.54 (m, 2H), 3.49-3.27 (m, 2H), 1.85-1.61 (m, 2H), 1.62-1.44 (m, 2H), 1.41-1.28 (m, 2H), 0.97-0.73 (m, 3H).

MS (ESI, LR) Calculated for $C_{22}H_{26}FN_2O_6$ (MH$^+$): 433.2, found: 433.1.

**Example 16.3-(2-(6-(benzo[b]thiophen-3-yl)-1-oxoisoindolin-2-yl)butanamido)-5-fluoro-4-oxopentanoic acid**

<Scheme 16>

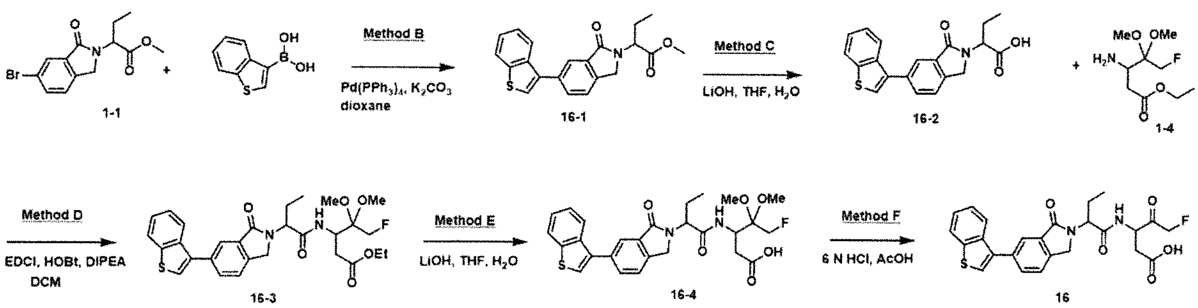


Using compound **1-1** and benzo[b]thiophen-3-ylboronic acid as starting materials, the target compound **16** (23 mg) was obtained using Method B/C/D/E/F sequentially.

$^1$H NMR (400 MHz, DMSO-$d_6$) d 12.55 (s, 1H), 8.17-8.07 (m, 1H), 8.01-7.73 (m, 6H), 7.53-7.41 (m, 2H), 5.37-4.41 (m, 6H), 2.94-2.36 (m, 2H), 2.16-1.61 (m, 2H), 1.00-0.75 (m, 3H).

MS (ESI, LR) Calculated for $C_{25}H_{24}FN_2O_5S$ (MH$^+$): 483.1, found: 483.1.

**Example 17. 4-(2-(1-((1-carboxy-4-fluoro-3-oxobutan-2-yl)amino)-1-oxobutan-2-yl)-3-oxoisoindolin-5-yl)benzoic acid**

<Scheme 17>

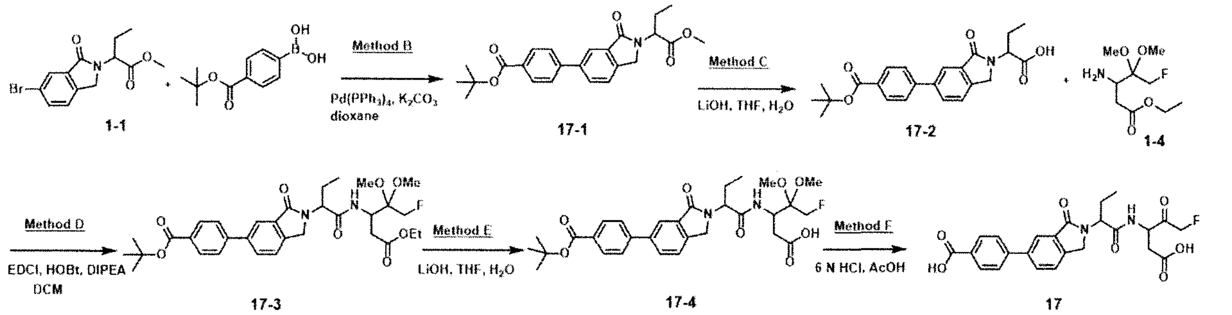


Using compound **1-1** and (4-(tert-butoxycarbonyl)phenyl)boronic acid as starting materials, the target compound **17** (18 mg) was obtained using Method B/C/D/E/F sequentially.

$^1H$ NMR (400 MHz, DMSO-$d_6$) d 12.97 (s, 2H), 8.24-7.58 (m, 8H), 5.03-4.26 (m, 6H), 2.91-2.34 (m, 2H), 2.19-1.63 (m, 2H), 1.08-0.69 (m, 3H).

MS (ESI, LR) Calculated for $C_{24}H_{24}FN_2O_7$ ($MH^+$): 471.1, found: 471.1.

**Example 18. 3-(2-(6-(4-acetylphenyl)-1-oxoisoindolin-2-yl)butanamido)-5-fluoro-4-oxopentanoic acid**

<Scheme 18>

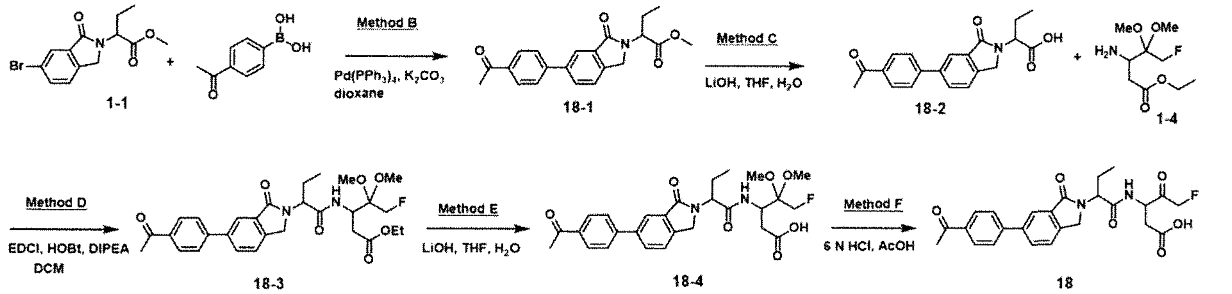


Using compound **1-1** and (4-acetylphenyl)boronic acid as starting materials, the target compound **18** (29 mg) was obtained using Method B/C/D/E/F sequentially.

$^1H$ NMR (400 MHz, DMSO-$d_6$) d 8.19-7.31 (m, 8H), 5.33-4.13 (m, 6H), 2.62 (s, 3H), 2.84-2.18 (m, 2H), 2.12-1.33 (m, 2H), 0.95-0.68 (m, 3H).

MS (ESI, LR) Calculated for $C_{25}H_{26}FN_2O_6$ ($MH^+$): 469.2, found: 469.1.

**Example 19.3-(2-(6-(4-(benzo[d]isothiazol-3-yl)piperazin-1-yl)-1-oxoisoindolin-2-yl)butanamido)-5-fluoro-4-oxopentanoic acid**

<Scheme 19>

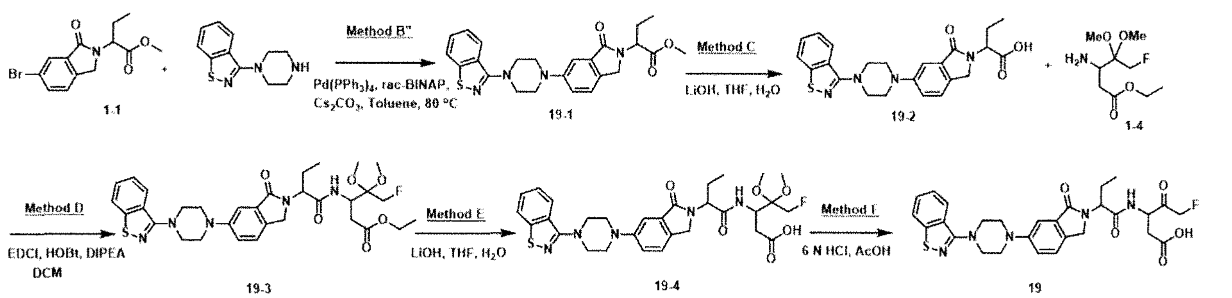

Using compound **1-1** and 3-(piperazin-1-yl)benzo[d]isothiazole as starting materials, the target compound **19** (22 mg) was obtained using Method B7C/D/E/F sequentially.

$^1$H NMR (400 MHz, DMSO-$d_6$) d 8.20-8.03 (m, 2H), 7.66-7.42 (m, 4H), 7.41-7.30 (m, 1H), 7.28-7.20 (m, 1H), 5.31-4.10 (m, 6H), 3.62 (s, 4H), 3.44 (s, 4H), 2.87-2.34 (m, 2H), 2.14-1.67 (m, 2H), 0.96-0.73(m, 3H).

MS (ESI, LR) Calculated for $C_{28}H_{31}FN_5O_5S$ ($MH^+$): 568.2, found: 568.2.

**Example 20. 5-fluoro-4-oxo-3-(2-(1-oxo-6-(6-(trifluoromethyl)pyridin-3-yl)isoindolin-2-yl)butanamido)pentanoic acid**

<Scheme 20>

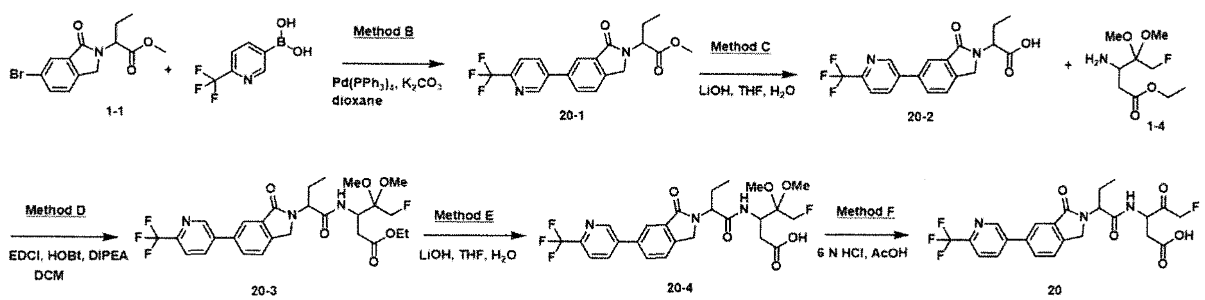


Using compound **1-1** and (6-(trifluoromethyl)pyridin-3-yl)boronic acid as starting materials, the target compound **20** (31 mg) was obtained using Method B/C/D/E/F sequentially.

$^1$H NMR (400 MHz, DMSO-$d_6$) d 12.89 (s, 1H), 9.18 (s, 1H), 8.83 (s, 1H), 8.47 (d, *J* = 6.10 Hz, 1H), 8.18-8.06 (m, 2H), 8.01 (d, *J* = 8.01 Hz, 1H), 7.80 (d, *J* = 7.63 Hz, 1H), 5.40-4.40 (m, 6H), 2.85-2.39 (m, 2H), 2.16-1.72 (m, 2H), 0.98-0.77(m, 3H).

MS (ESI, LR) Calculated for $C_{23}H_{22}F_4N_3O_5$ ($MH^+$): 496.1, found: 496.1.

**Example 21. (3-(2-(6-(2-aminopyrimidin-5-yl)-1-oxoisoindolin-2-yl)butanamido)-5-fluoro-4-oxopentanoic acid**

<Scheme 21>

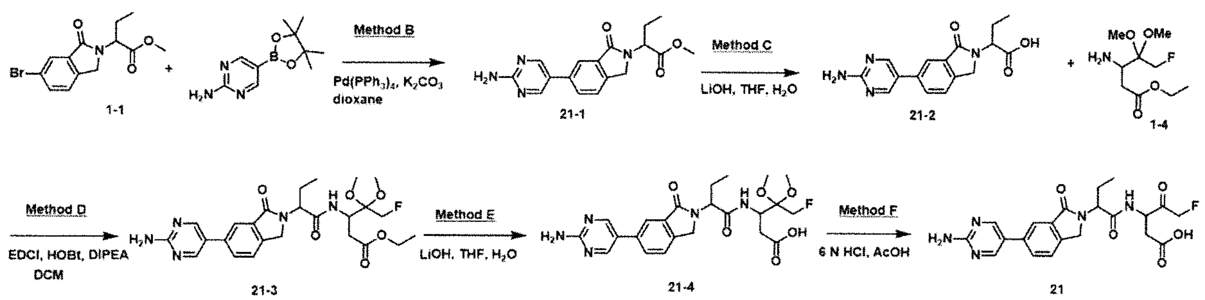


Using compound **1-1** and 5-(4,4,5,5-tetramethyl-1,3,2-dioxaborolan-2-yl)pyrimidin-2-amine as starting materials, the target compound **21** (17 mg) was obtained using Method B/C/D/E/F sequentially.

$^1$H NMR (400 MHz, DMSO-$d_6$) d 12.57 (s, 1H), 8.87-8.69 (m, 1H), 8.64 (s, 2H), 7.98-7.76 (m, 2H), 7.67 (d, *J* = 7.93 Hz, 1H), 6.82 (s, 2H), 5.35-4.20 (m, 6H), 2.95-2.37 (m, 2H), 2.14-1.68 (m, 2H), 0.98-0.71 (m, 3H).

MS (ESI, LR) Calculated for $C_{21}H_{23}FN_5O_5$ ($MH^+$): 444.2, found: 444.1.

**Example 22. 3-(2-(6-(benzofuran-3-yl)-1-oxoisoindolin-2-yl)butanamido)-5-fluoro-4-oxopentanoic acid**

<Scheme 22>

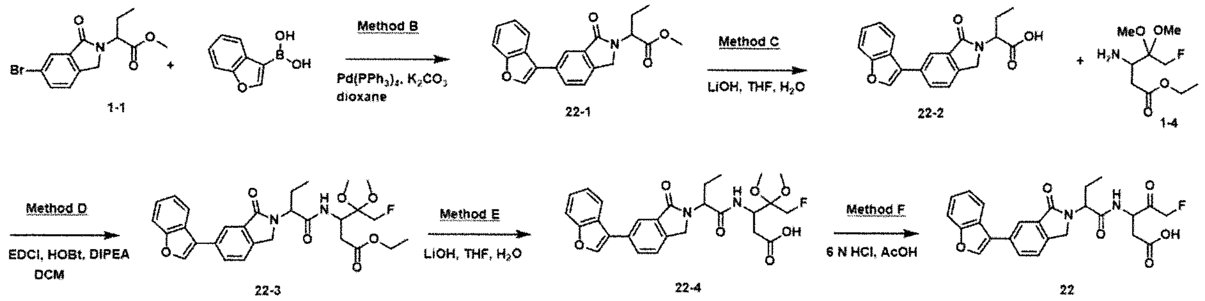


1-1, 22-1, 22-2, 1-4, 22-3, 22-4, 22

Using compound **1-1** and benzofuran-3-ylboronic acid as starting materials, the target compound **22** (24 mg) was obtained using Method B/C/D/E/F sequentially.

$^1$H NMR (400 MHz, DMSO-$d_6$) d 12.50 (s, 1H), 8.87-8.73 (m, 1H), 8.50 (s, 1H), 8.06-7.79 (m, 2H), 7.93 (d, $J$ = 6.71 Hz, 1H), 7.80-7.73 (m, 1H), 7.69 (d, $J$ = 7.32 Hz, 1H), 7.46-7.35 (m, 2H), 5.33-4.25 (m, 6H), 2.92-2.36 (m, 2H), 2.11-1.70 (m, 2H), 0.96-0.74 (m, 3H).

MS (ESI, LR) Calculated for $C_{25}H_{24}FN_2O_6$ (MH$^+$): 467.2, found: 467.1.

**Example 23. 3-(2-(6-(2-aminopyridin-4-yl)-1-oxoisoindolin-2-yl)butanamido)-5-fluoro-4-oxopentanoic acid**

<Scheme 23>

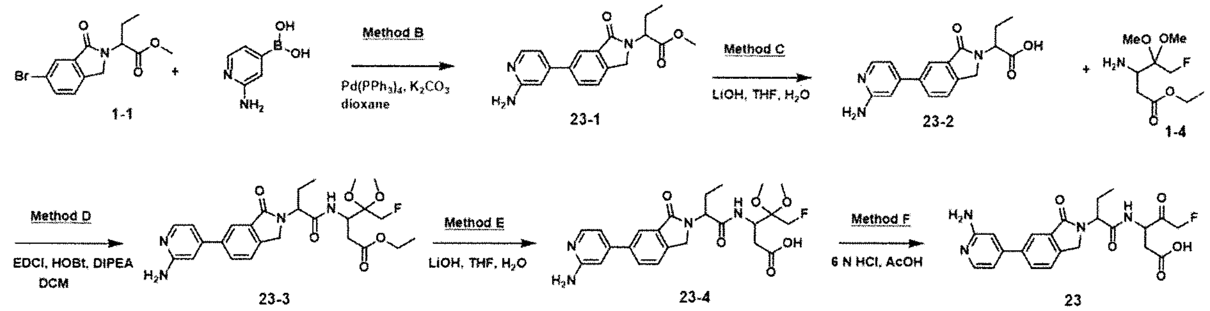


1-1, 23-1, 23-2, 1-4, 23-3, 23-4, 23

Using compound **1-1** and (2-aminopyridin-4-yl)boronic acid as starting materials, the target compound **23** (4 mg) was obtained using Method B/C/D/E/F sequentially.

MS (ESI, LR) Calculated for $C_{22}H_{24}FN_4O_5$ (MH$^+$): 443.2, found: 443.0.

**Example 24. 5-fluoro-3-(2-(6-(1-methyl-1H-pyrazol-4-yl)-1-oxoisoindolin-2-yl)butanamido)-4-oxopentanoic acid**

<Scheme 24>

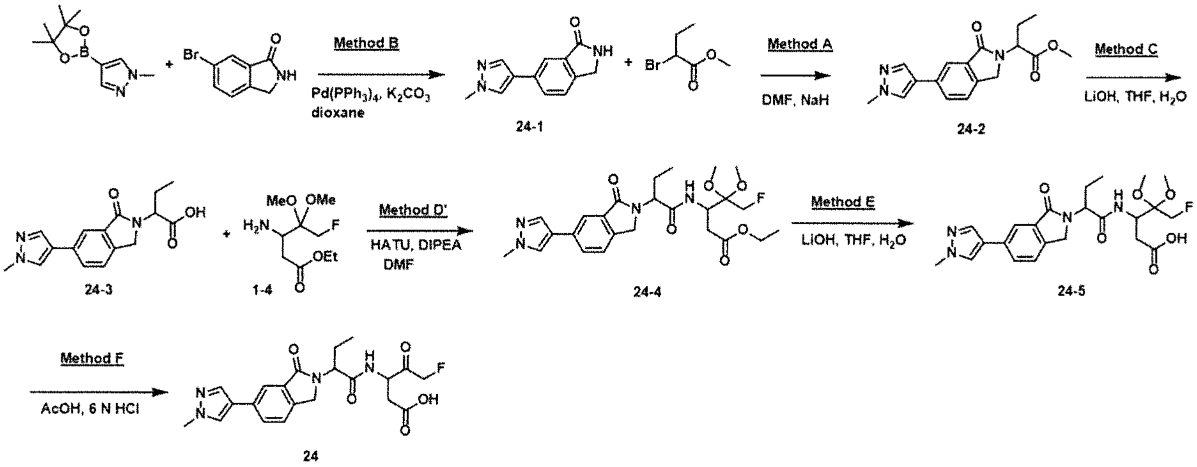


Using 1-methyl-4-(4,4,5,5-tetramethyl-1,3,2-dioxaborolan-2-yl)-1H-pyrazole and 6-bromoisoindolin-1-one as starting materials, the target compound **24** (28 mg) was obtained using Method B/A/C/D7E/F sequentially.

$^1$H NMR (400 MHz, DMSO-$d_6$) d 12.76 (s, 1H), 8.27 (d, $J$ = 4.58 Hz, 1H), 7.96 (d, $J$ = 4.58 Hz, 1H), 7.91-7.72 (m, 2H), 7.68-7.47 (m, 2H), 5.39-4.22 (m, 6H), 3.87 (s, 3H), 2.91-2.39 (m, 2H), 2.15-1.69 (m, 2H), 1.02-0.74 (m, 3H).

MS (ESI, LR) Calculated for $C_{21}H_{24}FN_4O_5$ (MH$^+$): 431.2, found: 431.2.

**Example 25. 5-fluoro-3-(2-(6-(1-methyl-1H-pyrazol-3-yl)-1-oxoisoindolin-2-yl)butanamido)-4-oxopentanoic acid**

<Scheme 25>

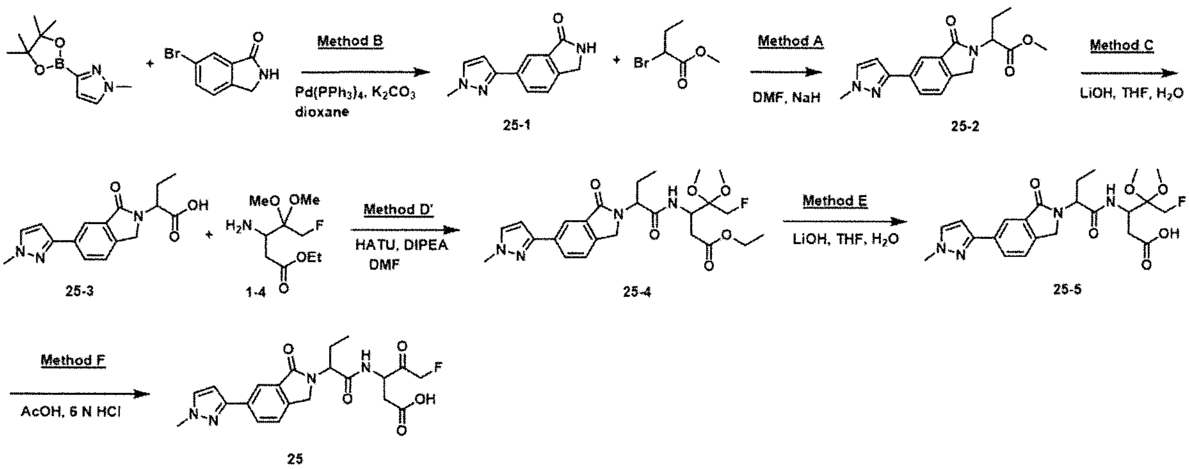


Using 1-methyl-3-(4,4,5,5-tetramethyl-1,3,2-dioxaborolan-2-yl)-1H-pyrazole and 6-bromoisoindolin-1-one as starting materials, the target compound 25 (21mg) was obtained using Method B/A/C/D'/E/F sequentially.

$^1$H NMR (400 MHz, DMSO-$d_6$) d 8.87-8.68 (m, 1H), 8.03 (t, $J$ = 8.01 Hz, 2H), 7.75 (d, $J$ = 2.29 Hz, 1H), 7.65-7.47 (m, 1H), 6.87-6.68 (m, 1H), 5.28-4.27 (m, 6H), 3.89 (s, 3H), 2.77-2.25 (m, 2H), 2.17-1.62 (m, 2H), 1.00-0.58 (m, 3H).

MS (ESI, LR) Calculated for $C_{21}H_{24}FN_4O_5$ (MH$^+$): 431.2, found: 431.2.

**Example 26. 3-(2-(6-(benzofuran-5-yl)-1-oxoisoindotin-2-yl)butanamido)-5-fluoro-4-oxopentanoic acid**

<Scheme 26>

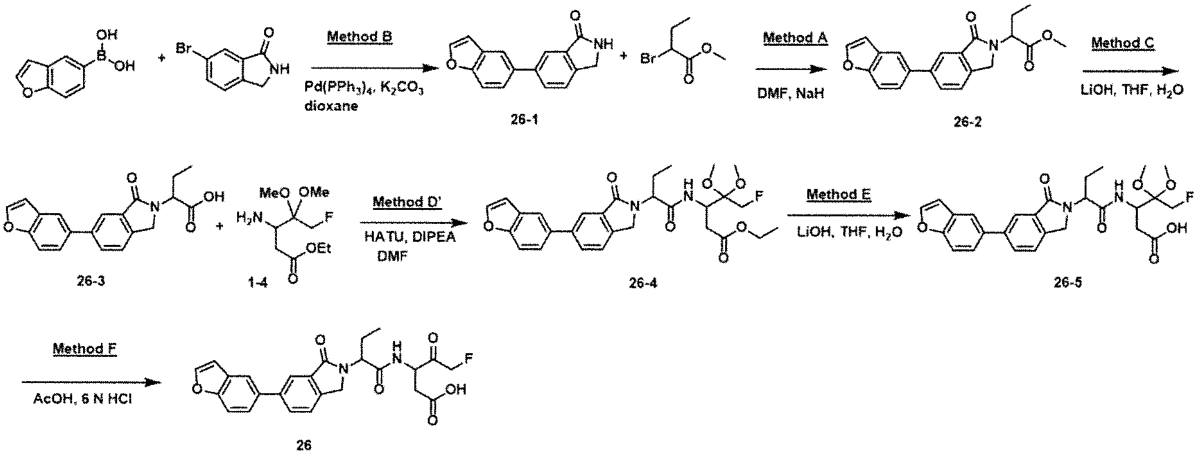


Using benzofuran-5-ylboronic acid and 6-bromoisoindolin-1-one as starting materials, the target compound 26 (19 mg) was obtained using Method B/A/C/D'/E/F sequentially.

$^1$H NMR (400 MHz, DMSO-$d_6$) d 8.82-8.63 (m, 1H), 8.11-7.85 (m, 4H), 7.77-7.54 (m, 3H), 7.01 (s, 1H), 5.28-4.23 (m, 6H), 2.76-2.25 (m, 2H), 2.13-1.63 (m, 2H), 0.95-0.62 (m, 3H).

MS (ESI, LR) Calculated for $C_{25}H_{22}FN_2O_6$ ($MH^+$): 467.2, found: 467.1.

**Example 27. 3-(2-(6-(2,3-dihydrobenzo[b] [1,4]dioxin-6-yl)-1-oxoisoindolin-2-yl)butanamido)-5-fluoro-4-oxo-pentanoic acid**

<Scheme 27>

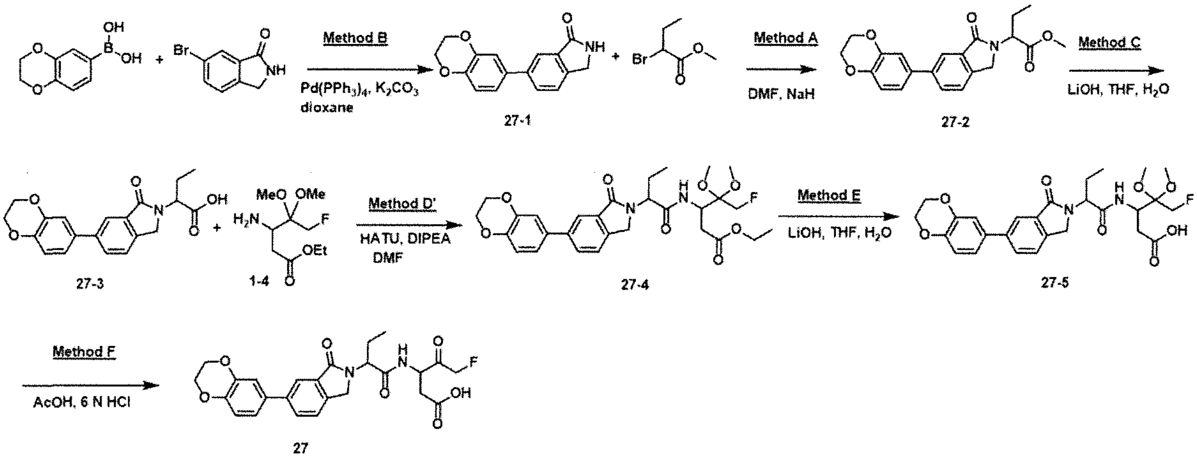


Using (2,3-dihydrobenzo[b][1,4]dioxin-6-yl)boronic acid and 6-bromoisoindolin-1-one as starting materials, the target compound **27** (21 mg) was obtained using Method B/A/C/D'/E/F sequentially.

$^1$H NMR (400 MHz, DMSO-$d_6$) d 8.79-8.63 (m, 1H), 7.82 (t, $J$ = 9.16 Hz, 2H), 7.68-7.55 (m, 1H), 7.22-7.12 (m, 2H), 6.95 (d, $J$ = 8.01 Hz, 1H), 5.27-4.32 (m, 6H), 4.28 (s, 4H), 2.77-2.29 (m, 2H), 2.16-1.62 (m, 2H), 0.93-0.62 (m, 3H).

MS (ESI, LR) Calculated for $C_{25}H_{26}FN_2O_7$ ($MH^+$): 485.2, found: 485.2.

**Example 28. 5-fluoro-4-oxo-3-(2-(1-oxo-6-(thiophen-3-yl)isoindolin-2-yl)butanamido)pentanoic acid**

<Scheme 28>

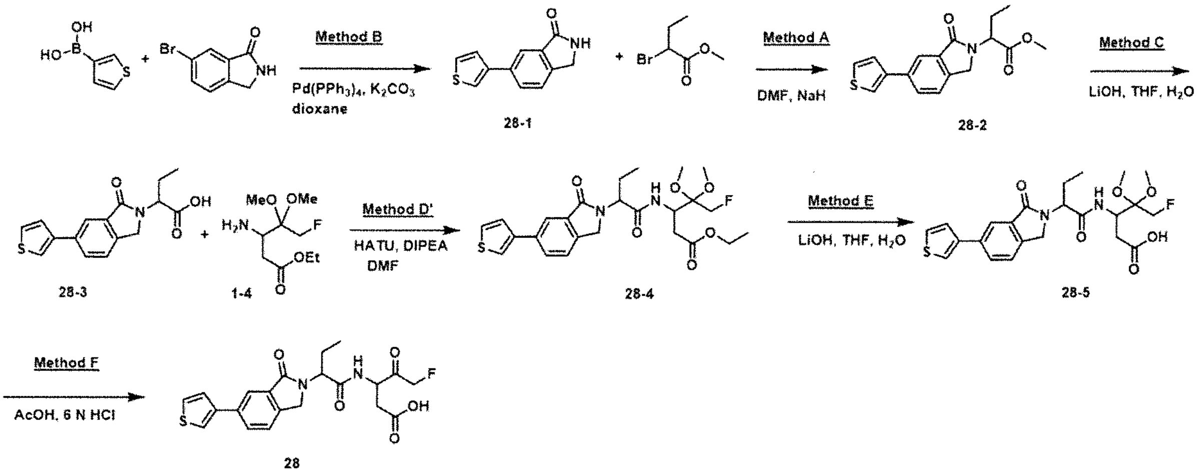


Using thiophen-3-ylboronic acid and 6-bromoisoindolin-1-one as starting materials, the target compound **28** (27 mg) was obtained using Method B/A/C/D7E/F sequentially.

$^1$H NMR (400 MHz, MeOD) d 8.20-7.34 (m, 7H), 5.26-4.22 (m, 6H), 2.95-2.35 (m, 2H), 2.31-1.75 (m, 2H), 1.11-0.73 (m, 3H).

MS (ESI, LR) Calculated for $C_{21}H_{22}FN_2O_5S$ ($MH^+$): 433.1, found: 433.1.

**Example 29. 5-fluoro-3-(2-(6-(isoquinolin-4-yl)-1-oxoisoindolin-2-yl)butanamido)-4-oxopentanoic acid**

<Scheme 29>

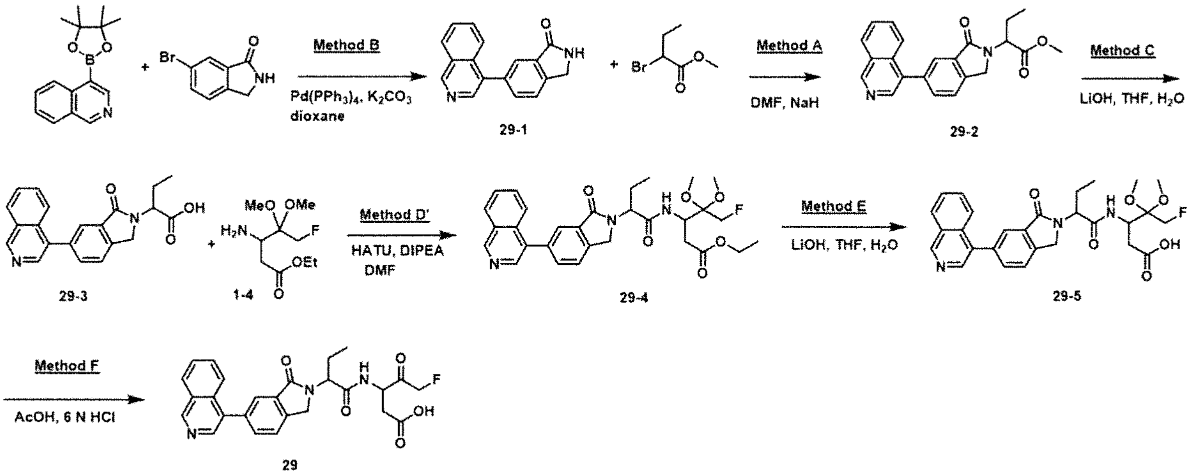


Using 4-(4,4,5,5-tetramethyl-1,3,2-dioxaborolan-2-yl)isoquinoline and 6-bromoisoindolin-1-one as starting materials, the target compound 29 (31 mg) was obtained using Method B/A/C/D7E/F sequentially.

$^1$H NMR (400 MHz, DMSO-$d_6$) d 12.90 (s, 1H), 9.35 (s, 1H), 8.45 (s, 1H), 8.21 (d, $J$ = 7.63 Hz, 1H), 7.86-7.68 (m, 5H), 7.63-7.47 (m, 2H), 5.32-4.21 (m, 6H), 2.90-2.36 (m, 2H), 2.14-1.71 (m, 2H), 0.99-0.73 (m, 3H).

MS (ESI, LR) Calculated for $C_{26}H_{25}FN_3O_5$ ($MH^+$): 478.2, found: 478.2.

**Example 30. 5-fluoro-4-oxo-3-(2-(1-oxo-6-(quinolin-6-yl)isoindolin-2-yl)butanamido)pentanoic acid**

<Scheme 30>

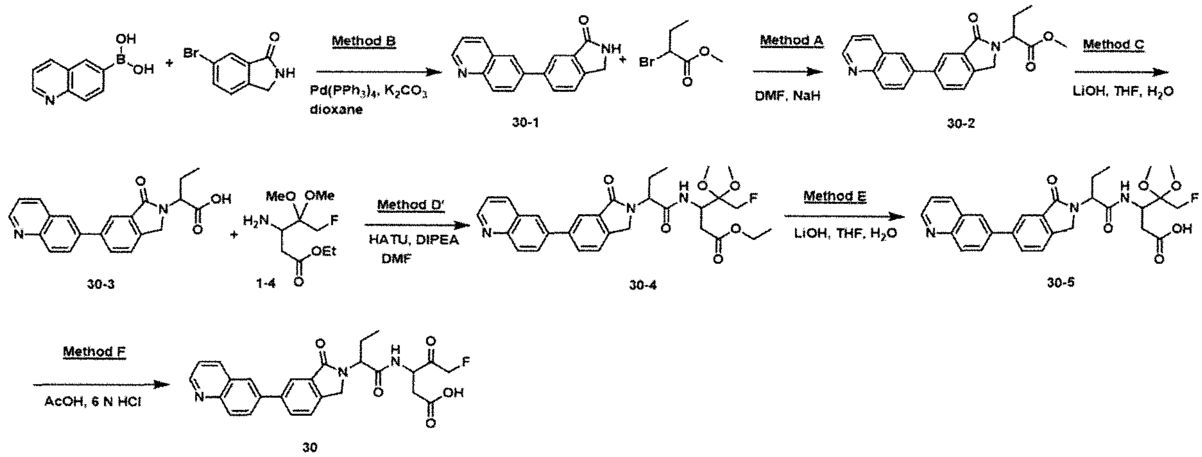


Using quinolin-6-ylboronic acid and 6-bromoisoindolin-1-one as starting materials, the target compound **30** (29 mg) was obtained using Method B/A/C/D7E/F sequentially.

$^1$H NMR (400 MHz, DMSO-$d_6$) d 12.92 (s, 1H), 8.99-8.89 (m, 1H), 8.55-8.39 (m, 2H), 8.26-8.08 (m, 4H), 7.89-7.74 (m, 1H), 7.72-7.51 (m, 2H), 5.39-4.36 (m, 6H), 2.95-2.39 (m, 2H), 2.20-1.68 (m, 2H), 1.00-0.76 (m, 3H).

MS (ESI, LR) Calculated for $C_{26}H_{25}FN_3O_5$ ($MH^+$): 478.2, found: 478.1.

**Example 31. 5-fluoro-4-oxo-3-(2-(1-oxo-6-(quinolin-4-yl)isoindolin-2-yl)butanamido)pentanoic acid**

<Scheme 31>

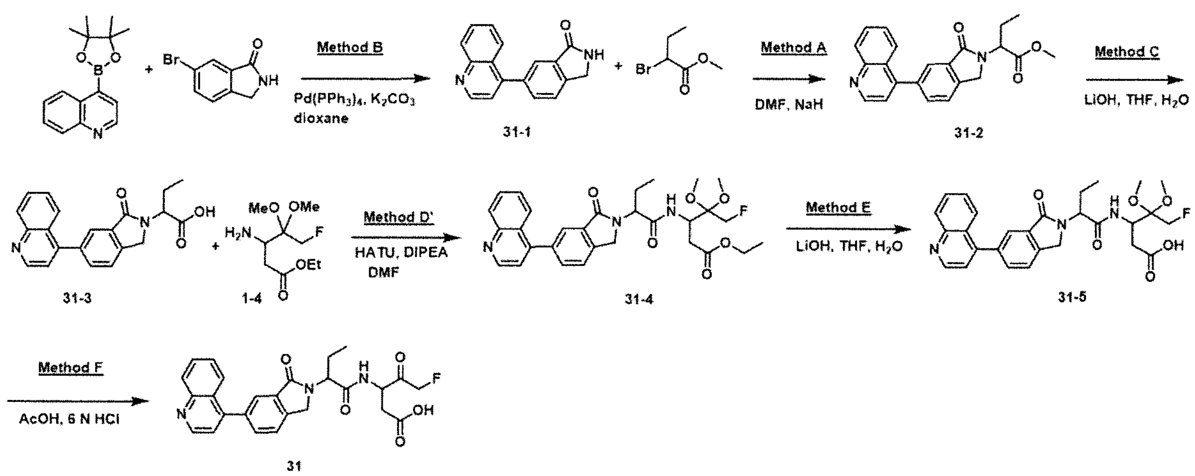


Using 4-(4,4,5,5-tetramethyl-1,3,2-dioxaborolan-2-yl)quinoline and 6-bromoisoindolin-1-one as starting materials, the target compound 31 (27 mg) was obtained using Method B/A/C/D7E/F sequentially.

$^1$H NMR (400 MHz, DMSO-$d_6$) d 12.99 (s, 1H), 8.98 (s, 1H), 8.13 (d, $J$ = 7.32 Hz, 1H), 7.93-7.75 (m, 4H), 7.68-7.48 (m, 4H), 5.36-4.51 (m, 6H), 2.89-2.38 (m, 2H), 2.18-1.78 (m, 2H), 1.03-0.76 (m, 3H).

MS (ESI, LR) Calculated for $C_{26}H_{25}FN_3O_5$ ($MH^+$): 478.2, found: 477.5.

**Example 32. 3-(2-(6-(benzo[d]thiazol-5-yl)-1-oxoisoindolin-2-yl)butanamido)-5-fluoro-4-oxopentanoic acid**

<Scheme 32>

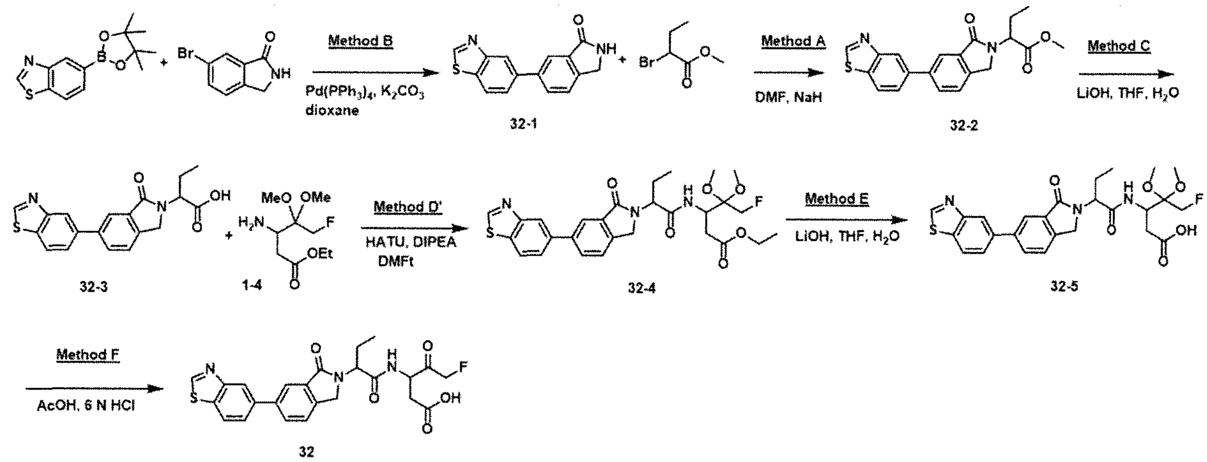


Using 5-(4,4,5,5-tetramethyl-1,3,2-dioxaborolan2-yl)benzo[d]thiazole and 6-bromoisoindolin-1-one as starting materials, the target compound 32 (24 mg) was obtained using Method B/A/C/D'/E/F sequentially.

$^1H$ NMR (400 MHz, DMSO-$d_6$) d 9.46 (s, 1H), 8.61-8.38 (m, 2H), 8.28 (d, *J* = 8.24 Hz, 1H), 8.09-8.00 (m, 2H), 7.87 (d, *J* = 8.24 Hz, 1H), 7.75 (d, *J* = 7.93 Hz, 1H), 5.54-4.24 (m, 6H), 2.18-1.66 (m, 2H), 0.97-0.76 (m, 3H).

MS (ESI, LR) Calculated for $C_{24}H_{23}FN_3O_5S$ ($MH^+$): 484.1, found: 483.4

**Example 33. 5-fluoro-4-oxo-3-(2-(1-oxo-6-(pyrazolo[1,5-a]pyridin-3-yl)isoindolin-2-yl)butanamido)pentanoic acid**

<Scheme 33>

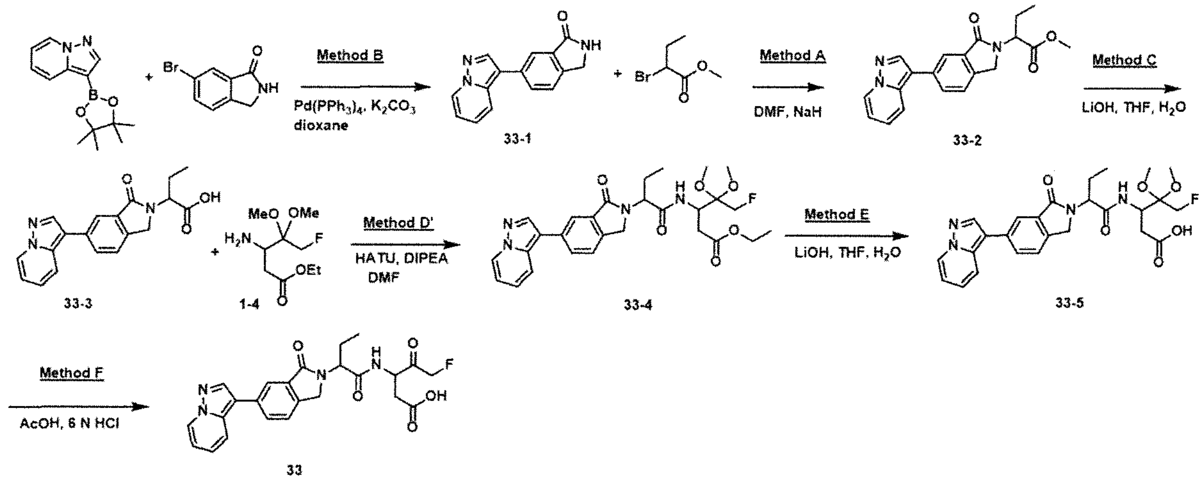


Using 3-(4,4,5,5-tetramethyl-1,3,2-dioxaborolan-2-yl)pyrazolo[1,5-a]pyridine and 6-bromoisoindolin-1-one as starting materials, the target compound **33** (20 mg) was obtained using Method B/A/C/D7E/F sequentially.

$^1H$ NMR (400 MHz, DMSO-$d_6$) d 12.94 (s, 1H), 8.76 (d, *J* = 7.02 Hz, 1H), 8.47 (s, 1H), 8.05-7.89 (m, 3H), 7.70 (d, *J* = 7.63 Hz, 1H), 7.37 (t, *J* = 7.02 Hz, 1H), 6.98 (t, *J* = 6.41 Hz, 1H), 5.35-4.28 (m, 6H), 2.93-2.41 (m, 2H), 2.16-1.66 (m, 2H), 1.00-0.75 (m, 3H).

MS (ESI, LR) Calculated for $C_{24}H_{24}FN_4O_5$ ($MH^+$): 467.2, found: 467.1.

**Example 34. 5-fluoro-4-oxo-3-(2-(1-oxo-6-(quinolin-5-yl)isoindolin-2-yl)butanamido)pentanoic acid**

<Scheme 34>

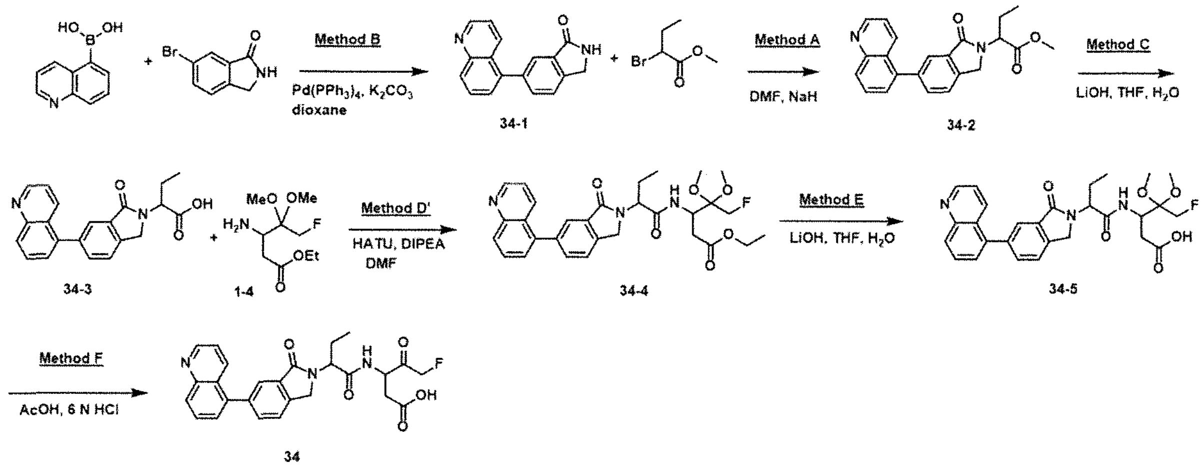


Using quinolin-5-ylboronic acid and 6-bromoisoindolin-1-one as starting materials, the target compound **34** (31 mg) was obtained using Method B/A/C/D'/E/F sequentially.

$^1$H NMR (400 MHz, DMSO-$d_6$) d 12.98 (s, 1H), 8.95 (d, *J* = 3.66 Hz, 1H), 8.18 (d, *J* = 8.24 Hz, 1H), 8.10 (d, *J* = 8.54 Hz, 1H), 7.91-7.71 (m, 4H), 7.62 (d, *J* = 7.02 Hz, 1H), 7.53 (dd, *J*= 8.39, 3.66 Hz, 1H), 5.32-4.52 (m, 6H), 2.91-2.41 (m, 2H), 2.18-1.72 (m, 2H), 1.00-0.78 (m, 3H).

MS (ESI, LR) Calculated for $C_{26}H_{25}FN_3O_5$ ($MH^+$): 478.2, found: 478.2.

**Example 35. 5-fluoro-3-(2-(6-(isoquinolin-8-yl)-1-oxoisoindolin-2-yl)butanamido)-4-oxopentanoic acid**

<Scheme 35>

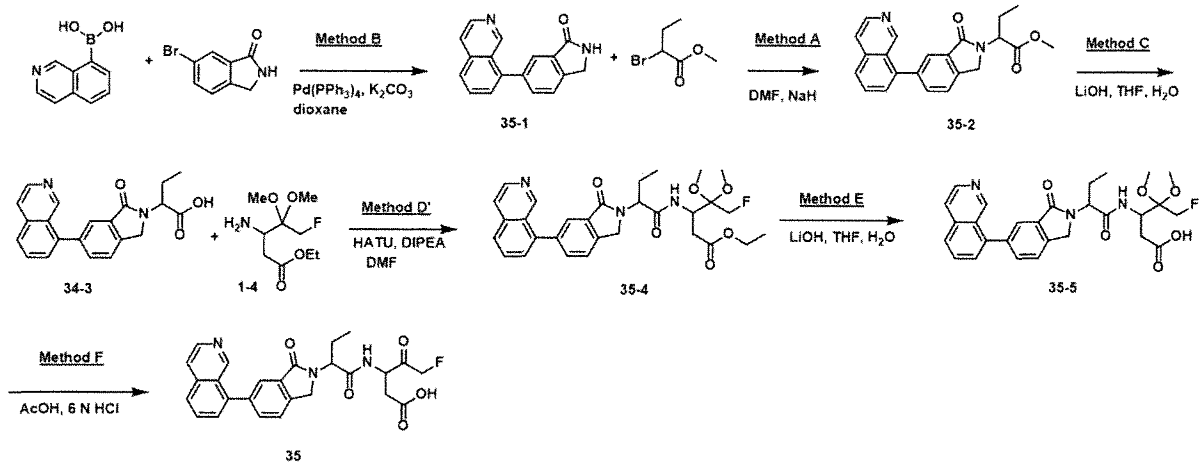


Using isoquinolin-8-ylboronic acid and 6-bromoisoindolin-1-one as starting materials, the target compound **35** (19 mg) was obtained using Method B/A/C/D7E/F sequentially.

$^1$H NMR (400 MHz, DMSO-$d_6$) d 9.14 (s, 1H), 8.59-8.46 (m, 1H), 8.05 (d, *J*= 7.63 Hz, 1H), 7.96-7.72 (m, 6H), 7.66 (d, *J*= 7.63 Hz, 1H), 5.35-4.47 (m, 6H), 2.85-2.38 (m, 2H), 2.16-1.75 (m, 2H), 0.98-0.75 (m, 3H).

MS (ESI, LR) Calculated for $C_{26}H_{25}FN_3O_5$ ($MH^+$): 478.2, found: 478.2.

**Example 36. 5-fluoro-3-(2-(6-(isoquinolin-5-yl)-1-oxoisoindolin-2-yl)butanamido)-4-oxopentanoic acid**

<Scheme 36>

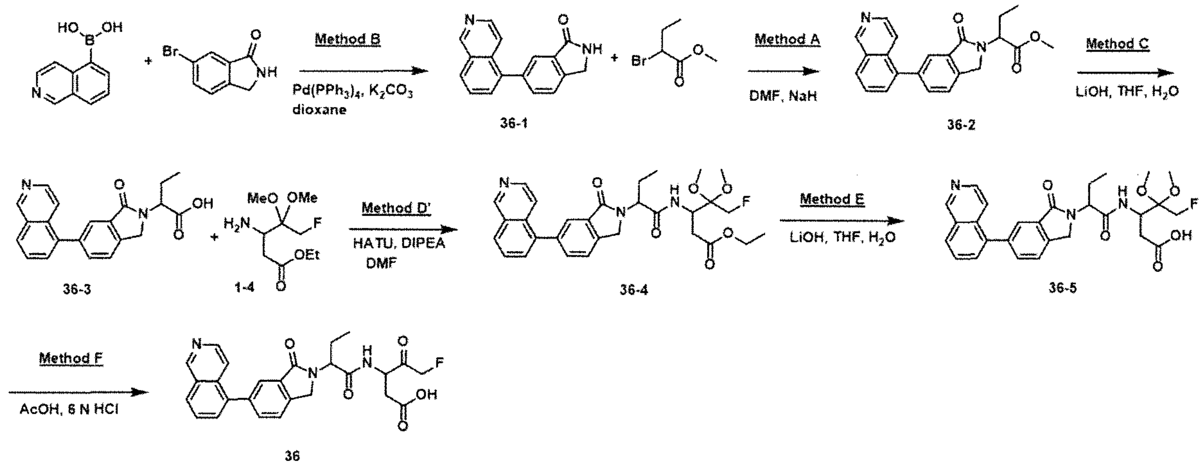


Using isoquinolin-5-ylboronic acid and 6-bromoisoindolin-1-one as starting materials, the target compound **36** (23 mg) was obtained using Method B/A/C/D'/E/F sequentially.

$^1$H NMR (400 MHz, DMSO-$d_6$) d 9.42 (s, 1H), 8.50 (d, $J$ = 5.80 Hz, 1H), 8.24-8.18 (m, 1H), 7.84-7.73 (m, 6H), 7.65 (d, $J$ = 4.88 Hz, 1H), 5.34-4.50 (m, 6H), 2.84-2.30 (m, 2H), 2.17-1.78 (m, 2H), 0.96-0.80 (m, 3H).

MS (ESI, LR) Calculated for $C_{26}H_{25}FN_3O_5$ ($MH^+$): 478.2, found: 478.1.

**Example 37. 5-fluoro-4-oxo-3-(2-(1-oxo-6-(quinolin-3-yl)isoindolin-2-yl)butanamido)pentanoic acid**

<Scheme 37>

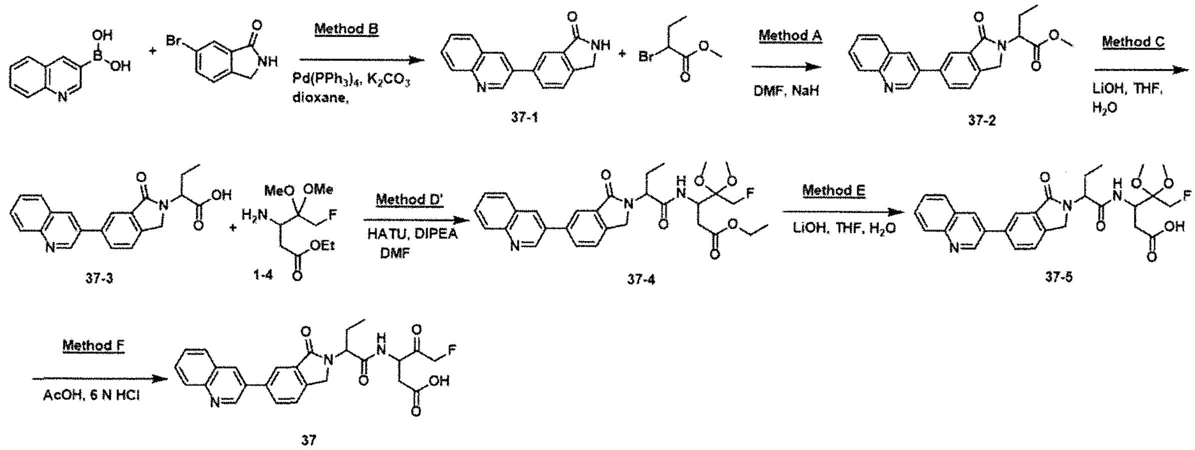


Using quinolin-3-ylboronic acid and 6-bromoisoindolin-1-one as starting materials, the target compound 37 (25 mg) was obtained using Method B/A/C/D'/E/F sequentially.

$^1$H NMR (400 MHz, DMSO-$d_6$) d 12.60 (s, 1H), 9.32 (s, 1H), 8.78 (s, 1H), 8.35-7.97 (m, 4H), 7.95-7.49 (m, 4H), 5.40-4.28 (m, 6H), 2.94-2.31 (m, 2H), 2.19-1.69 (m, 2H), 1.00-0.61 (m, 3H).

MS (ESI, LR) Calculated for $C_{26}H_{25}FN_3O_5$ ($MH^+$): 478.2, found: 478.2.

**Example 38. 5-fluoro-4-oxo-3-(2-(1-oxo-6-(2-oxo-2,3-dihydro-1H-benzo[d]imidazol-5-yl)isoindolin-2-yl)acetamido)pentanoic acid**

<Scheme 38>

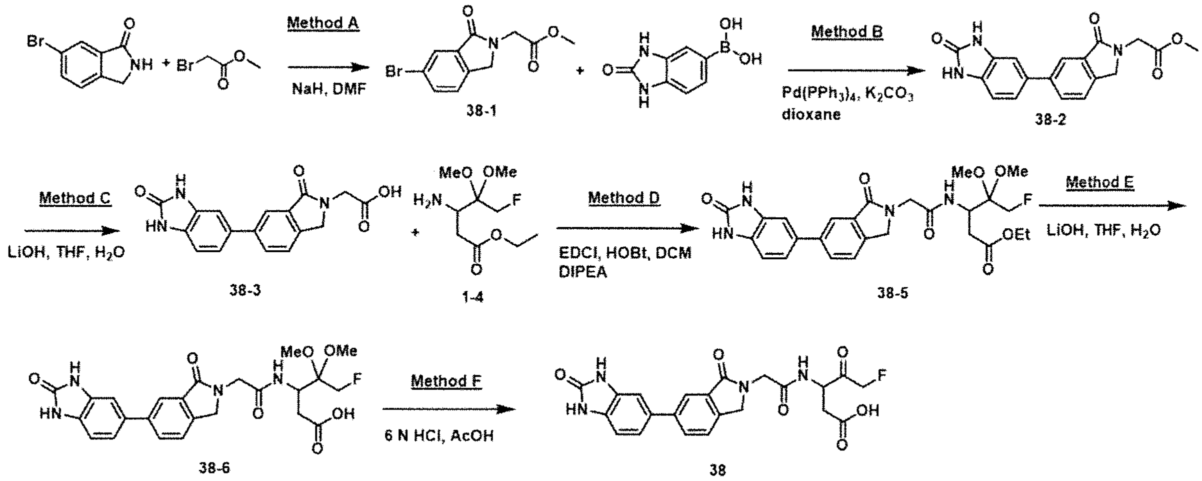


Using methyl 2-bromoacetate and 6-bromoisoindolin-1-one as starting materials, the target compound **38** (23 mg) was obtained using Method A/B/C/D/E/F sequentially.

$^1$H NMR (400 MHz, DMSO-$d_6$) d 12.23 (s, 1H), 10.72 (s, 2H), 8.41 (s, 1H), 7.98-7.77 (m, 2H), 7.66 (s, 1H), 7.37-7.14 (m, 2H), 7.11-6.94 (m, 1H), 5.48-3.94 (m, 7H), 3.59-2.69 (m, 2H).

MS (ESI, LR) Calculated for $C_{22}H_{20}FN_4O_6$ (MH$^+$): 455.1, found: 455.1.

**Example 39. 3-(2-(6-(benzofuran-3-yl)-1-oxoisoindolin-2-yl)acetamido)-5-fluoro-4-oxopentanoic acid**

<Scheme 39>

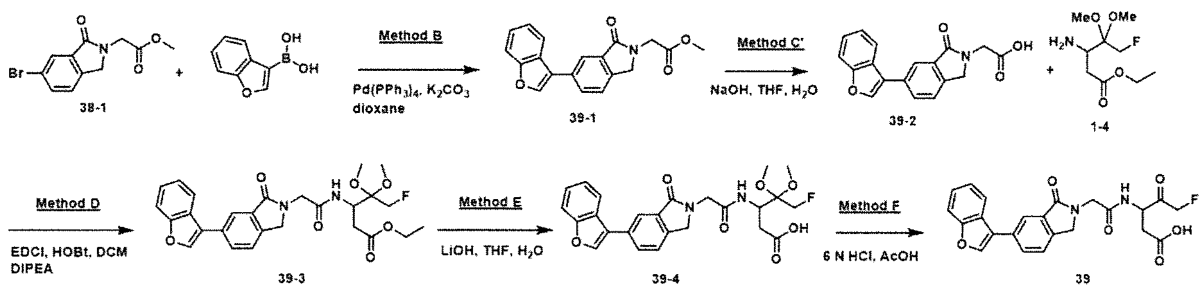


Using compound **38-1** and benzofuran-3-ylboronic acid as starting materials, the target compound **39** (33 mg) was obtained using Method B/C'/D/E/F sequentially.

$^1$H NMR (400 MHz, DMSO-$d_6$) d 12.93 (s, 1H), 8.52 (s, 1H), 8.12-7.85 (m, 3H), 7.83-7.50 (m, 4H), 7.49-7.31 (m, 2H), 5.45-3.93 (m, 3H), 4.59 (s, 2H), 4.32 (s, 2H), 2.95-2.25 (m, 2H).

MS (ESI, LR) Calculated for $C_{23}H_{20}FN_2O_6$ (MH$^+$): 439.1, found: 439.1.

**Example 40. 3-(2-(6-(benzo[b]thiophen-3-yl)-1-oxoisoindolin-2-yl)acetamido)-5-fluoro-4-oxopentanoic acid**

<Scheme 40>

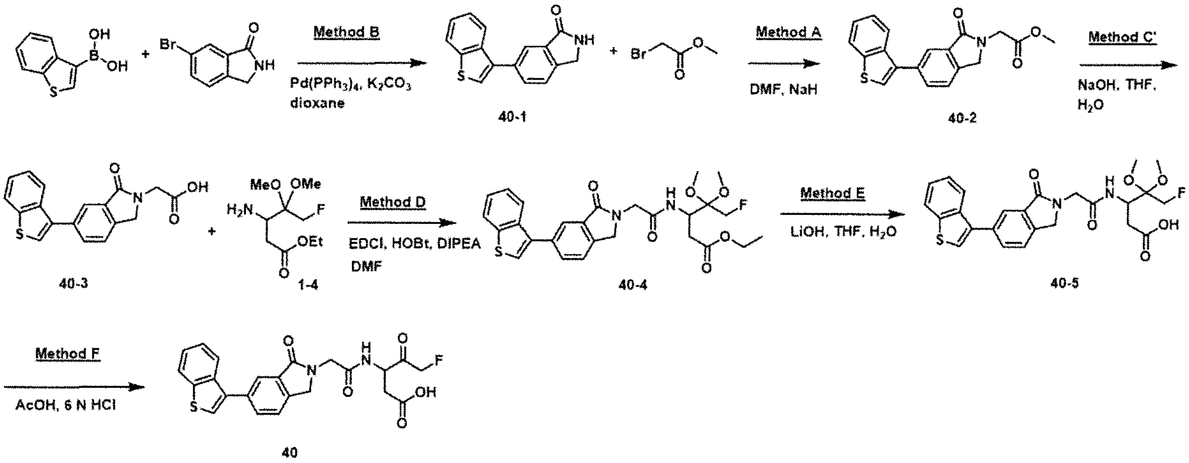


40-1 40-2 40-3 1-4 40-4 40-5 40

Using 6-bromoisoindolin-1-one and benzothiophen-3-ylboronic acid as starting materials, the target compound **40** (30 mg) was obtained using Method B/A/C'/D/E/F sequentially.

$^1$H NMR (400 MHz, DMSO-$d_6$) d 12.52 (s, 1H), 8.15-8.02 (m, 1H), 8.00-7.70 (m, 3H), 7.68-7.38 (m, 5H), 5.41-5.06 (m, 1H), 4.80-4.16 (m, 6H), 2.97-2.38 (m, 2H).

MS (ESI, LR) Calculated for $C_{23}H_{20}FN_2O_5S$ (MH$^+$): 455.1, found: 455.1.

**Example 41. 3-(2-(6-(benzofuran-5-yl)-1-oxoisoindolin-2-yl)acetamido)-5-fluoro-4-oxopentanoic acid**

<Scheme 41>

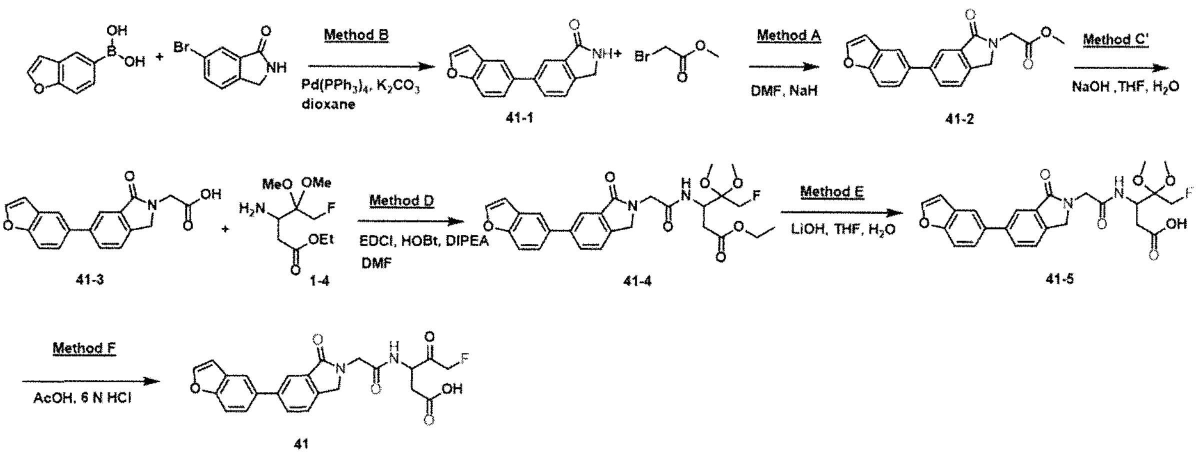


41-1 41-2 41-3 1-4 41-4 41-5 41

Using 6-bromoisoindolin-1-one and benzofuran-5-ylboronic acid as starting materials, the target compound **41** (29 mg) was obtained using Method B/A/C'/D/E/F sequentially.

$^1$H NMR (400 MHz, DMSO-$d_6$) d 12.91 (s, 1H), 8.05 (d, $J$ = 2.14 Hz, 1H), 8.01 (s, 1H), 7.98-7.93 (m, 2H), 7.77-7.51 (m, 4H), 7.06-7.00 (m, 1H), 5.41-3.96 (m, 7H), 2.98-2.41 (m, 2H).

MS (ESI, LR) Calculated for $C_{23}H_{20}FN_2O_6$ (MH$^+$): 439.1, found: 439.1.

**Example 42. 3-((S)-2-(6-(2,3-dihydrobenzo[b][1,4]dioxin-6-yl)-1-oxoisoindolin-2-yl)-3-methylbutanamido)-5-fluoro-4-oxopentanoic acid**

<Scheme 42>

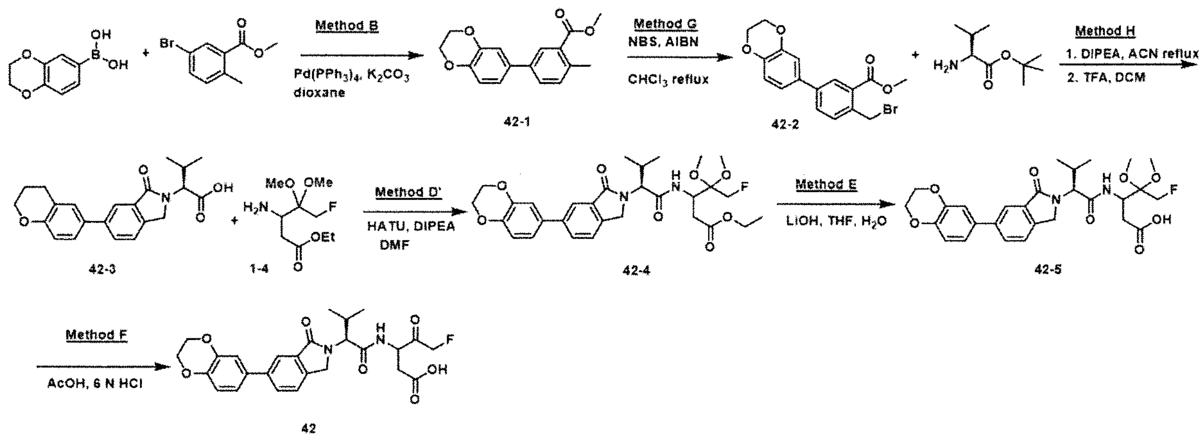


Step 42-1: Using methyl 5-bromo-2-methylbenzoate and (400 mg, 1.75 mmol) (2,3-dihydrobenzo[b][1,4]dioxin-6-yl)boronic acid (330 mg, 1.83 mmol) as starting materials, the target compound **42-1** (444 mg, 89%) was obtained using Method B.

Step 42-2 (Method G): After dissolving compound **42-1** (450 mg, 1.58 mmol) in chloroform (60 mL), N-bromosuccinimide and (NBS, 333.55 mg, 1.87 mmol) AIBN (25 mg, 0.16 mmol) were added and stirred at reflux for 24 hours. After completion of the reaction, the reaction solution was washed with brine (50 mL), the organic layer was dried with anhydrous sodium sulfate, filtered, concentrated, and separated by column chromatography to give the target compound **42-2** (345 mg, 60 %).

Step 42-3 (Method H): Compound 42-2 (345 mg, 0.95 mmol) was dissolved in acetonitrile (40 mL), then tert-butyl L-valinate (172.8 mg, 0.997 mmol) and DIPEA (147.3 mg, 1.14mmol) were added and stirred at reflux for 12 hours. After completion of the reaction, the solvent was concentrated by distillation under reduced pressure and extracted with brine (50 mL) and ethyl acetate (40 mL x 2). The organic layer was dried over anhydrous sodium sulfate, filtered, and concentrated. The concentrate was dissolved in dichloromethane (10 mL), trifluoroacetic acid (3 mL) was added, and the mixture was stirred at room temperature for 2 hours. After completion of the reaction, the mixture was concentrated, diluted with brine (30 mL), and extracted using ethyl acetate (40 mL x 2). The organic layer was dried over anhydrous sodium sulfate, filtered, concentrated, and separated using column chromatography to give the target compound **42-3** (199 mg, 57%).

Step 42-4/42-5: Using compound **42-3** (150 mg, 0.408 mmol), the target compound **42** (44 mg) was obtained using Method D'/E/F sequentially.

$^1$H NMR (400 MHz, DMSO-$d_6$) d 12.45 (s, 1H), 9.05-8.91 (m, 1H), 7.90-7.76 (m, 2H), 7.70-7.59 (m, 1H), 7.25-7.12 (m, 2H), 6.95 (dd, *J* = 8.09, 2.14 Hz, 1H), 5.34-4.34 (m, 6H), 4.28 (s, 4H), 2.90-2.46 (m, 2H), 2.35-2.17 (m, 1H), 1.00-0.69 (m, 6H).

MS (ESI, LR) Calculated for $C_{26}H_{28}FN_2O_7$ ($MH^+$): 499.2, found: 499.1.

**Example 43. 5-fluoro-3-((S)-3-methyl-Z-(1-oxo-6-(pyrrolidin-1-yl)isoindolin-2-yl)butanamido)-4-oxopentanoic acid**

<Scheme 43>

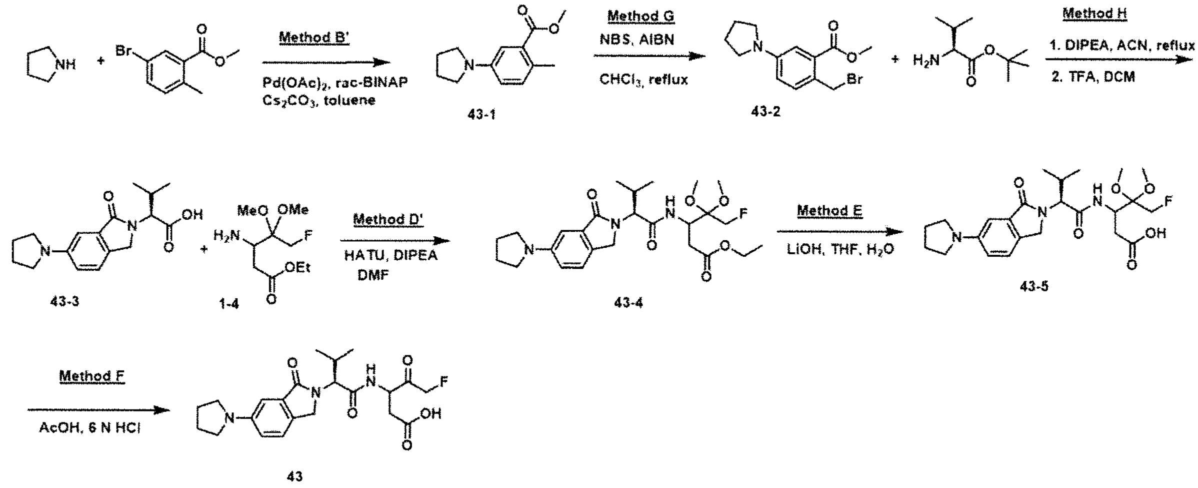


Using methyl 5-bromo-2-methylbenzoate and pyrrolidine as starting materials, the target compound **43** (66 mg) was obtained using Method B'/G/H/D'/E/F sequentially.

$^1$H NMR (400 MHz, DMSO-$d_6$) d 8.96 (s, 1H), 7.92-7.29 (m, 1H), 7.23-6.69 (m, 2H), 5.57-4.24 (m, 6H), 4.10-3.11 (m, 4H), 3.05-2.45 (m, 3H), 2.42-1.85 (m, 4H), 1.54-0.64 (m, 6H).

MS (ESI, LR) Calculated for $C_{22}H_{29}FN_3O_5$ (MH$^+$): 434.2, found: 434.1.

**Example 44. 3-((S)-2-(6-(benzofuran-3-yl)-1-oxoisoindolin-2-yl)-3-methylbutanamido)-5-fluoro-4-oxopentanoic acid**

<Scheme 44>

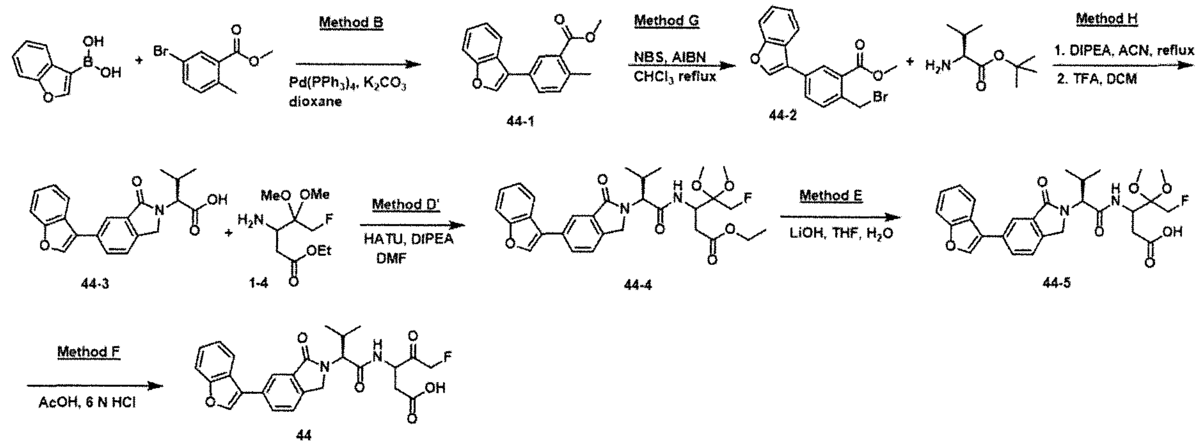


Using methyl 5-bromo-2-methylbenzoate and benzofuran-3-ylboronic acid as starting materials, the target compound **44** (71 mg) was obtained using Method B/G/H/D'/E/F sequentially.

$^1$H NMR (400 MHz, DMSO-$d_6$) d 9.06-8.84 (m, 1H), 8.50 (s, 1H), 8.12-7.89 (m, 3H), 7.84-7.65 (m, 2H), 7.52-7.34 (m, 2H), 5.37-4.44 (m, 6H), 2.86-2.46 (m, 2H), 2.39-2.16 (m, 1H), 1.13-0.67 (m, 6H).

MS (ESI, LR) Calculated for $C_{26}H_{26}FN_2O_6$ (MH$^+$): 481.2, found: 481.1.

**Example 45. 5-fluoro-3-((S)-3-methyl-2-(1-oxo-6-(2-oxo-2,3-dihydro-1H-benzo[d]imidazol-5-yl)isoindolin-2-yl)butanamido)-4-oxopentanoic acid**

<Scheme 45>

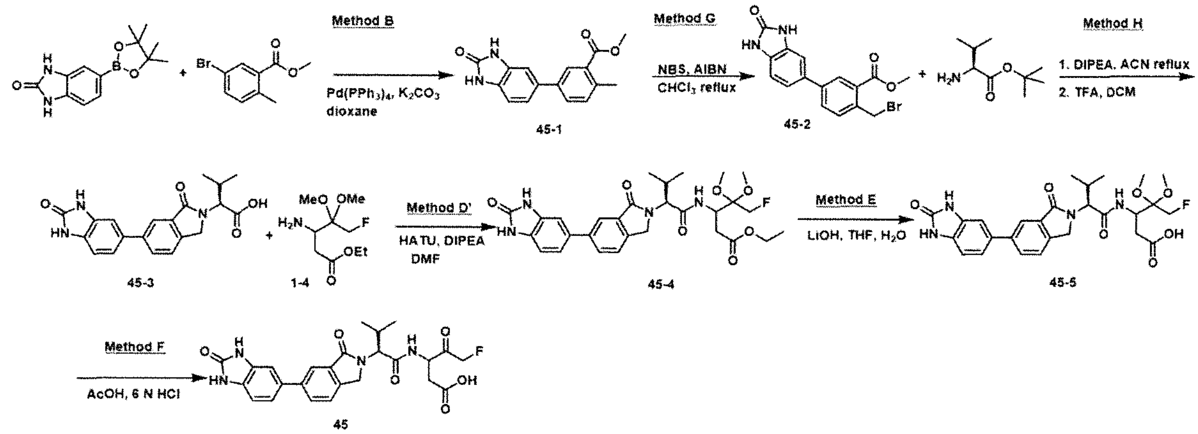


Using methyl 5-bromo-2-methylbenzoate and 5-(4,4,5,5-tetramethyl-1,3,2-dioxaborolan-2-yl)-1,3-dihydro-2H-benzo[d]imidazol-2-one as starting materials, the target compound 45 (54 mg) was obtained using Method B/G/H/D'/E/F sequentially.

$^1$H NMR (400 MHz, DMSO-$d_6$) d 12.43 (s, 1H), 10.72 (d, $J$ = 9.46 Hz, 2H), 9.05-8.91 (m, 1H), 7.79-7.76 (m, 2H), 7.70-7.61 (m, 1H), 7.28 (d, $J$ = 7.93 Hz, 1H), 7.20 (s, 1H), 7.01 (d, $J$= 7.93 Hz, 1H), 5.32-4.21 (m, 6H), 2.92-2.44 (m, 2H), 2.35-2.16 (m, 1H), 1.06-0.70 (m, 6H).

MS (ESI, LR) Calculated for $C_{25}H_{26}FN_4O_6$ ($MH^+$): 497.2, found: 497.1.

**Example 46. 5-fluoro-4-oxo-3-(2-(1-oxo-6-(pyrrolidin-1-yl)isoindolin-2-yl)propanamido)pentanoic acid**

<Scheme 46>

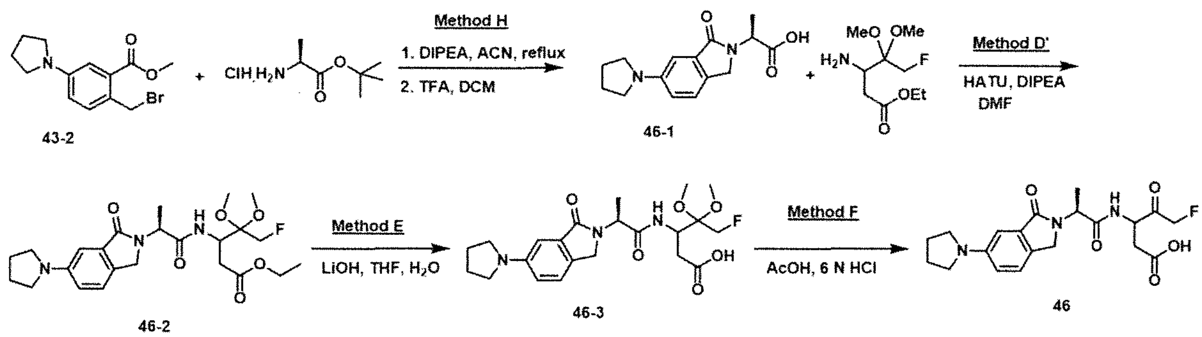


Using compound **43-2** and tert-butyl L-alanine 1 hydrochloride as starting materials, the target compound **46** (24 mg) was obtained using Method H/D'/E/F sequentially.

$^1$H NMR (400 MHz, DMSO-$d_6$) d 12.81 (s, 1H), 7.36 (d, $J$ = 8.01 Hz, 1H), 6.80 (dd, $J$ = 8.39, 2.29 Hz, 1H), 6.75-6.67 (m, 1H), 5.41-4.12 (m, 6H), 3.57-2.94 (m, 4H), 2.81-2.27 (m, 2H), 1.98 (s, 4H), 1.47 (d, $J$ = 7.63 Hz, 3H).

MS (ESI, LR) Calculated for $C_{20}H_{25}FN_3O_5$ ($MH^+$): 406.2, found: 406.1.

**Example 47. 5-fluoro-4-oxo-3-(2-(1-oxo-6-(2-oxo-2,3-dihydro-1H-benzoldlimidazol-5-yl)isoindolin-2-yl)propanamido)pentanoic acid**

<Scheme 47>

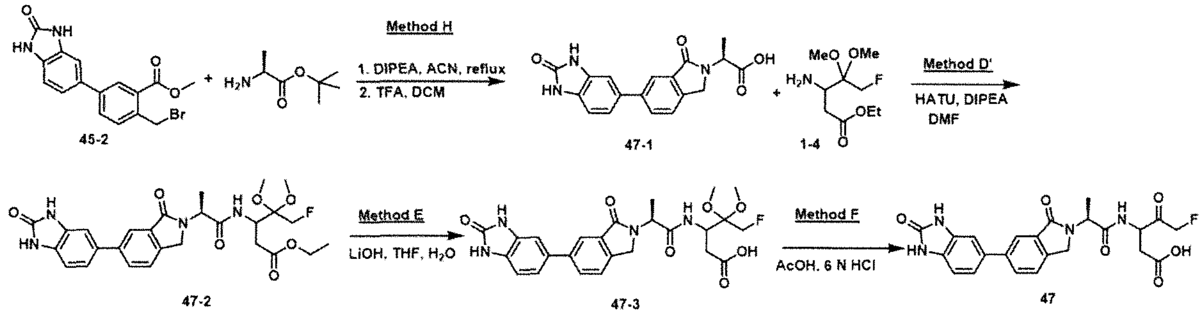


Using compound **45-2** and tert-butyl L-alanine 1 hydrochloride as starting materials, the target compound **47** (31 mg) was obtained using Method H/D'/E/F sequentially.

$^1$H NMR (400 MHz, DMSO-$d_6$) d 12.91 (s, 1H), 10.72 (s, 2H), 7.90-7.76 (m, 3H), 7.66 (dd, *J* = 7.63, 3.81 Hz, 1H), 7.29 (d, *J* = 8.39 Hz, 1H), 7.20 (s, 1H), 7.01 (dd, *J* = 8.01, 4.20 Hz, 1H), 5.39-4.18 (m, 6H), 2.85-2.27 (m, 2H), 1.57-1.47 (m, 3H).

MS (ESI, LR) Calculated for $C_{23}H_{22F}N_4O_6$ ($MH^+$): 469.1, found: 469.1.

**Example 48. 3-(2-(6-(2,3-dihydrobenzo[b][1,4]dioxin-6-yl)-1-oxoisoindolin-2-yl)propanamido)-5-fluoro-4-oxopentanoic acid**

<Scheme 48>

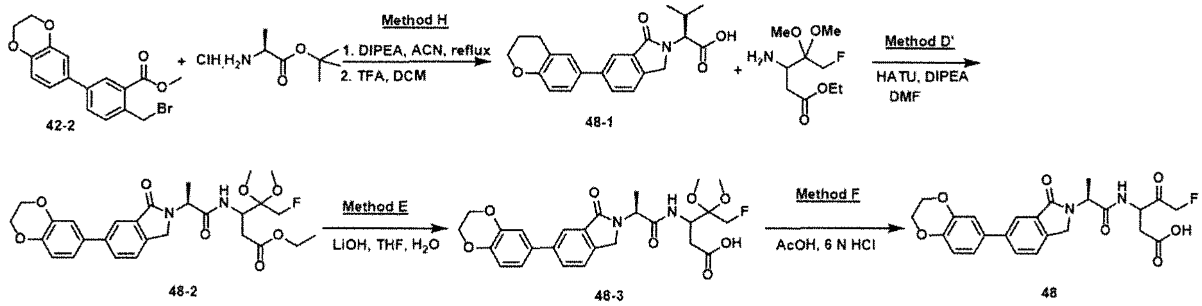


Using compound **42-2** and tert-butyl L-alanine 1 hydrochloride as starting materials, the target compound **48** (19 mg) was obtained using Method H/D'/E/F sequentially.

$^1$H NMR (400 MHz, DMSO-$d_6$) d 7.91-7.74 (m, 2H), 7.64 (d, *J* = 7.63 Hz, 1H), 7.25-7.12 (m, 2H), 6.95 (d, *J* = 8.01 Hz, 1H), 4.99-3.94 (m, 6H), 4.28 (s, 4H), 2.63-2.22 (m, 2H), 1.51 (d, *J* = 7.63 Hz, 3H).

MS (ESI, LR) Calculated for $C_{24}H_{24}FN_2O_7$ ($MH^+$): 471.1, found: 471.1.

**Example 49. 3-(2-(6-(benzofuran-3-yl)-1-oxoisoindolin-2-yl)propanamido)-5-fluoro-4-oxopentanoic acid**

<Scheme 49>

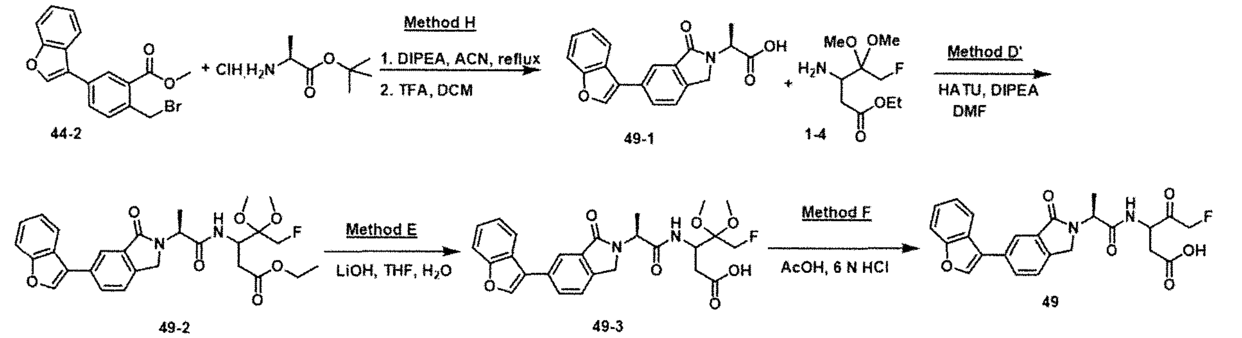


Using compound **44-2** and tert-butyl L-alanine 1 hydrochloride as starting materials, the target compound **49** (29 mg) was obtained using Method H/D'/E/F sequentially.

$^1$H NMR (400 MHz, DMSO-$d_6$) d 8.52 (s, 1H), 8.07-7.50 (m, 6H), 7.47-7.29 (m, 2H), 5.41-4.23 (m, 6H), 2.96-2.17 (m, 2H), 1.54 (d, *J* = 7.25 Hz, 3H).

MS (ESI, LR) Calculated for $C_{24}H_{22}FN_2O_6$ ($MH^+$): 453.1, found: 453.1.

**Example 50. (*S*)-3-((*S*)-2-(6-(benzofuran-3-yl)-1-oxoisoindolin-2-yl)butanamido)-4-oxo-5-(2,3,5,6-tetrafluoro-phenoxy)pentanoic acid**

<Scheme 50>

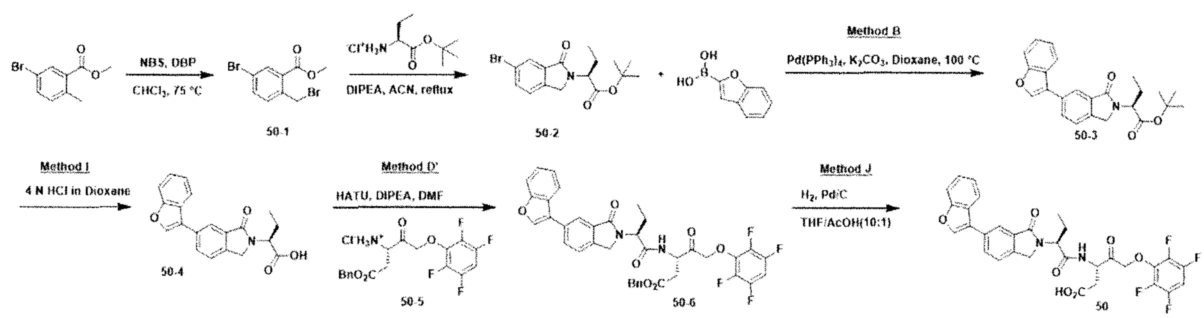


Step 50-1: 5-Bromo-2-methylbenzoate (25 g, 109.14 mmol) was dissolved in chloroform (100 mL), then NBS and (19.424 g, 109.14 mmol) dibenzoyl peroxide (1.322 g, 5.46 mmol) were added and stirred at 75 °C for 1 hr. The reaction solution was washed with water (100 mL), the organic layer was dried over anhydrous magnesium sulfate, filtered and concentrated to give the target compound **50-1.** This concentrate was used in the next reaction without further purification.

Step 50-2: Compound **50-1** was dissolved in acetonitrile (300 mL), then [(1S)-1-tert-butoxycarbonylpropyl] ammonium chloride (21.287 g, 108.78 mmol) and DIPEA (56.84 mL, 326.33 mmol) were added and the reaction was stirred at reflux for 5 hours. After completion of the reaction, the reaction solution was diluted with ethyl acetate (500 mL), then washed sequentially with water (300 mL), ammonium chloride saturated aqueous solution (300 mL) and brine (300 mL), the organic layer was dried over magnesium anhydride, filtered, concentrated and separated by column chromatography to give the target compound **50-2** (25.1 g, 65% over 2 steps).

MS (ESI, LR) Calculated for $C_{16}H_{20}BrNO_3Na$ ($MNa^+$): 376.1, found: 376.1.

Step 50-3: Using compound **50-2** and benzofuran-2-ylboronic acid as starting materials, the target compound **50-3** was obtained using Method B.

MS (ESI, LR) Calculated for $C_{24}H_{25}NO_4Na$ ($MNa^+$): 414.2, found: 414.2.

Step 50-4 (Method I): Compound **50-3** (0.456 g, 1.17 mmol) was dissolved in 1,4-dioxane (20 mL), then hydrogen chloride solution (4 N HCl in dioxane, 20 mL) was added and stirred at room temperature for 8 hours. After completion of the reaction, the solvent was concentrated, diethyl ether (20 mL) and ethyl acetate (20 mL) were added, and the resulting solid was filtered and dried to give the target compound **50-4** (0.365 g, 84%).

MS (ESI, LR) Calculated for $C_{20}H_{17}NO_4Na$ ($MNa^+$): 358.1, found: 358.1.

Step 50-5: Compound **50-5** was synthesized using a known method (WO2008/068615 A1).

MS (ESI, LR) Calculated for $C_{18}H_{16}F_4NO_4$ ($MH^+$): 386.1, found: 386.1.

Step 50-6 (Method D'): Using compound **50-4** and compound **50-5,** the target compound **50-6** was obtained using Method D'.

MS (ESI, LR) Calculated for $C_{38}H_{31}F_4N_2O_7$ ($MH^+$): 703.2, found: 703.2.

Step 50-7 (Method J): Compound **50-6** (0.499 g, 0.71 mmol) was dissolved in tetrahydrofuran (7 mL), acetic acid (0.7 mL) and 10% palladium/carbon (0.053 g, 0.05 mmol) were added, and the mixture was stirred in the presence of hydrogen for 12 hours. After completion of the reaction, palladium was filtered through a Celite filter, the filtrate was concentrated and separated by HPLC to give the target compound **50** (0.328 mg, 75.5%).

$^1H$ NMR (400 MHz, DMSO-$d_6$) δ12.50 (s, 1H), 8.88 (dd, *J* = 11.7, 7.5 Hz, 1H), 8.54 (d, *J* = 1.9 Hz, 1H), 8.08-8.01 (m, 2H), 8.00-7.93 (m, 1H), 7.79 (dd, *J* = 8.5, 1.8 Hz, 1H), 7.77-7.71 (m, 1H), 7.65-7.51 (m, 1H), 7.45 (dtd, *J* = 15.9, 7.3, 1.4 Hz, 2H), 5.36-5.16 (m, 2H), 4.88-4.81 (m, 1H), 4.81-4.65 (m, 2H), 4.59 (dd, *J* = 17.9, 3.0 Hz, 1H), 4.07 (q, *J* = 7.1 Hz, 1H), 2.82 (ddd, *J* = 16.9, 5.9, 1.7 Hz, 1H), 2.69-2.59 (m, 1H), 2.03 (s, 2H), 1.93-1.80 (m, 1H), 1.22 (t, *J* = 7.1 Hz, 1H), 0.91 (*t, J*= 7.3 Hz, 3H).

MS (ESI, LR) Calculated for $C_{31}H_{25}F_4N_2O_7$ ($MH^+$): 613.2, found: 613.2.

**Example 51. (*S*)-3-((*S*)-2-(6-(2,3-dihydrobenzo[b][1,4]dioxin-6-yl)-1-oxoisoindolin-2-yl)butanamido)-4-oxo-5-(2,3,5,6-tetrafluorophenoxy)pentanoic acid**

<Scheme 51>

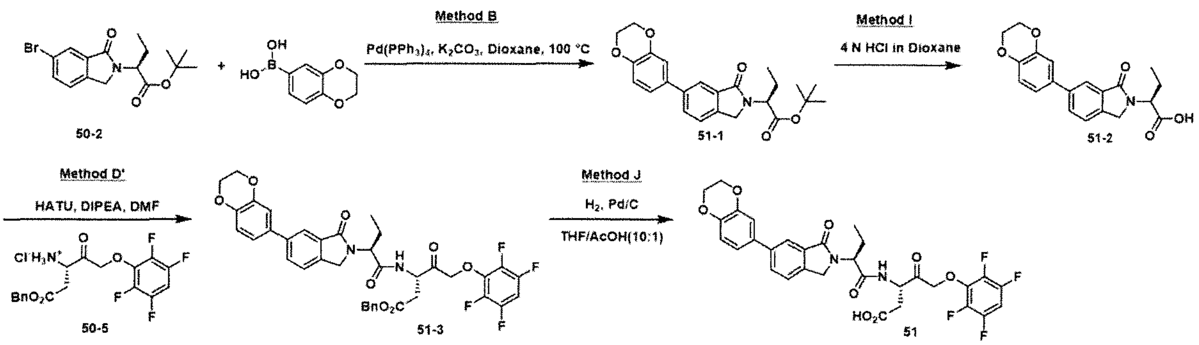


Using compound **50-2** as starting materials, the target compound **51** was obtained using Method B/I/D7J sequentially.

$^1H$ NMR (400 MHz, DMSO-$d_6$) δ12.51 (s, 1H), 8.85 (dd, *J* = 11.2, 7.5 Hz, 1H), 7.91-7.82 (m, 2H), 7.68 (d, *J* = 7.8 Hz, 1H), 7.66-7.52 (m, 1H), 7.23 (d, *J* = 8.0 Hz, 2H), 7.00 (d, *J* = 8.2 Hz, 1H), 5.27 (d, *J* = 4.1 Hz, 2H), 4.81 (dd, *J* = 9.7, 5.7 Hz, 1H), 4.74-4.62 (m, 2H), 4.53 (d, *J* = 17.7 Hz, 1H), 4.33 (s, 4H), 4.07 (q, *J* = 7.1 Hz, 1H), 2.80 (dd, *J* = 16.9, 6.0 Hz, 1H), 2.62 (dd, *J* = 16.9, 7.0 Hz, 1H), 2.03 (s, 2H), 1.84 (dq, *J* = 9.5, 7.2 Hz, 1H), 1.21 (t, *J* = 7.1 Hz, 1H), 0.88 (t, *J* = 7.3 Hz, 3H).

MS (ESI, LR) Calculated for $C_{31}H_{27}H_4N_2O_8$ ($MH^+$): 631.17, found: 631.2.

**Example 52. (*S*)-4-oxo-3-((*S*)-2-(1-oxo-6-(2-oxo-2,3-dihydro-1H-benzo[d]imidazol-5-yl)isoindolin-2-yl)butanamido)-5-(2,3,5,6-tetrafluorophenoxy)pentanoic acid**

<Scheme 52>

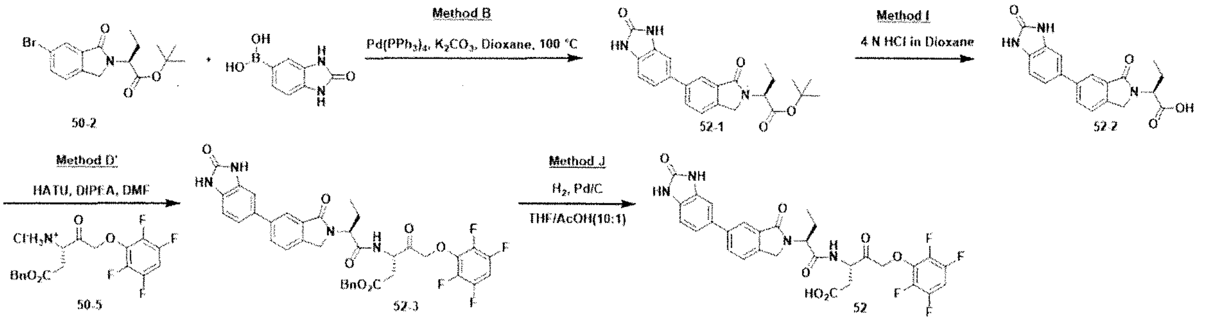


Using compound 50-2 and (2-oxo-2,3-dihydro-1H-benzo[d]imidazol-5-yl)boronic acid as starting materials, the target compound 52 was obtained using Method B/I/D7J sequentially.

$^1H$ NMR (400 MHz, DMSO-$d_6$) δ12.48 (s, 1H), 10.73 (d, *J* = 7.7 Hz, 2H), 8.84 (d, *J* = 7.4 Hz, 1H), 7.89-7.80 (m, 2H), 7.66 (d, *J* = 7.9 Hz, 1H), 7.55 (ddd, *J* = 11.0, 7.3, 3.6 Hz, 1H), 7.29 (dd, *J* = 8.1, 1.8 Hz, 1H), 7.21 (d, *J* = 1.8 Hz, 1H), 7.03 (d, *J* = 8.0 Hz, 1H), 5.25 (d, *J* = 4.2 Hz, 2H), 4.79 (dd, *J* = 9.6, 5.7 Hz, 1H), 4.73-4.60 (m, 2H), 4.51 (d, *J* = 17.6 Hz, 1H), 4.03 (q, *J* = 7.1 Hz, 1H), 2.78 (dd, *J* = 16.9, 5.9 Hz, 1H), 2.60 (dd, *J* = 16.9, 7.0 Hz, 1H), 2.00-1.92 (m, 2H), 1.87-1.77 (m, 1H), 1.18 (t, *J* = 7.1 Hz, 1H), 0.86 (t, *J* = 7.3 Hz, 3H).

MS (ESI, LR) Calculated for $C_{30}H_{25}F_4N_4O_7$ ($MH^+$): 629.2, found: 629.2.

**Example 53. (*S*)-4-oxo-3-((*S*)-2-(1-oxo-6-(pyrrolidin-1-yl)isoindolin-2-yl)butanamido)-5-(2,3,5,6-tetrafluorophenoxy)pentanoic acid**

<Scheme 53>

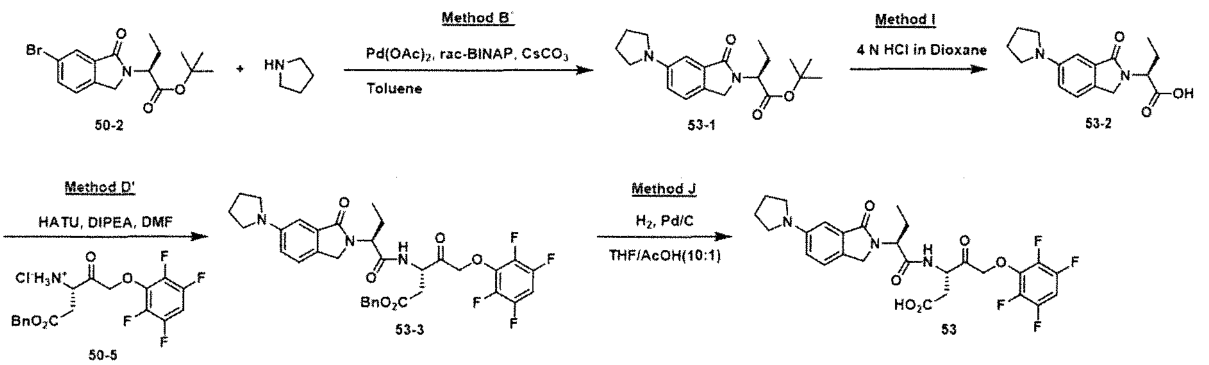


Using compound **50-2** and pyrrolidine as starting materials, the target compound **53** was obtained using Method B'/I/D'/J sequentially.

$^1H$ NMR (400 MHz, $CDCl_3$) δ8.11 (s, 1H), 7.47 (s, 1H), 7.33 (dd, *J* = 15.5, 8.3 Hz, 1H), 7.15 - 7.04 (m, 1H), 7.04-6.91 (m, 1H), 6.72 (s, 1H), 4.95 (s, 1H), 4.68 (d, *J* = 17.7 Hz, 2H), 4.41 (dd, *J* = 17.3, 11.7 Hz, 1H), 4.12 (q, *J* = 7.2 Hz, 1H), 3.39 (q, *J* = 5.5 Hz, 4H), 2.84-2.69 (m, 1H), 2.09 (h, *J* = 4.5 Hz, 6H), 1.96-1.83 (m, 1H), 1.26 (t, *J* = 7.1 Hz, 1H), 0.96 (td, *J* = 7.4, 3.7 Hz, 3H).

MS (ESI, LR) Calculated for $C_{27}H_{28}F_4N_3O_6$ ($MH^+$): 566.2, found: 566.2.

**Example 54. (*R*)-3-((*S*)-2-(6-(2,3-dihydrobenzo[b][1,4]dioxin-6-yl)-1-oxoisoindolin-2-yl)butanamido)-4-oxo-5-(2,3,5,6-tetrafluorophenoxy)pentanoic acid**

<Scheme 54>

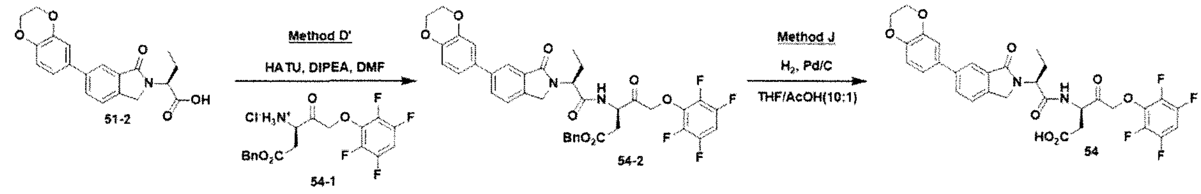


Step 54-1: Compound **54-1** was synthesized using a known method (WO2008/068615 A1).

MS (ESI, LR) Calculated for $C_{18}H_{16}F_4NO_4$ ($MH^+$): 386.1, found: 386.1.

Step 54-2: Using compound 51-2 and compound 54-1 as starting materials, the target compound **54** was obtained using Method D'/J sequentially.

$^1$H NMR (400 MHz, DMSO-$d_6$) δ12.51 (s, 1H), 8.85 (dd, *J* = 11.2, 7.5 Hz, 1H), 7.92-7.82 (m, 2H), 7.68 (dd, *J* = 7.8, 1.8 Hz, 1H), 7.58 (dddd, *J*= 18.1, 10.8, 8.9, 5.3 Hz, 1H), 7.23 (dd, *J* = 8.1, 1.4 Hz, 2H), 7.00 (d, *J* = 8.1 Hz, 1H), 5.35-5.14 (m, 2H), 4.80 (ddd, *J*= 12.4, 9.6, 5.9 Hz, 1H), 4.76-4.63 (m, 2H), 4.54 (dd, *J* = 17.8, 3.1 Hz, 1H), 4.33 (s, 4H), 4.07 (q, *J*= 7.1 Hz, 1H), 2.81 (ddd, *J*= 16.9, 5.9, 2.2 Hz, 1H), 2.63 (dt, *J*= 16.8, 7.1 Hz, 1H), 2.03 (s, 2H), 1.84 (s, 1H), 1.22 (t, *J*= 7.1 Hz, 1H), 0.89 (t, *J* = 7.3 Hz, 3H).

MS (ESI, LR) Calculated for $C_{31}H_{27}H_4N_2O_8$ ($MH^+$): 631.2, found 631.2.

**Example 55. 3-((*S*)-2-(6-(2,3-dihydrobenzo[b][1,4]dioxin-6-yl)-1-oxoisoindolin-2-yl)butanamido)-5-fluoro-4-oxo-pentanoic acid**

<Scheme 55>

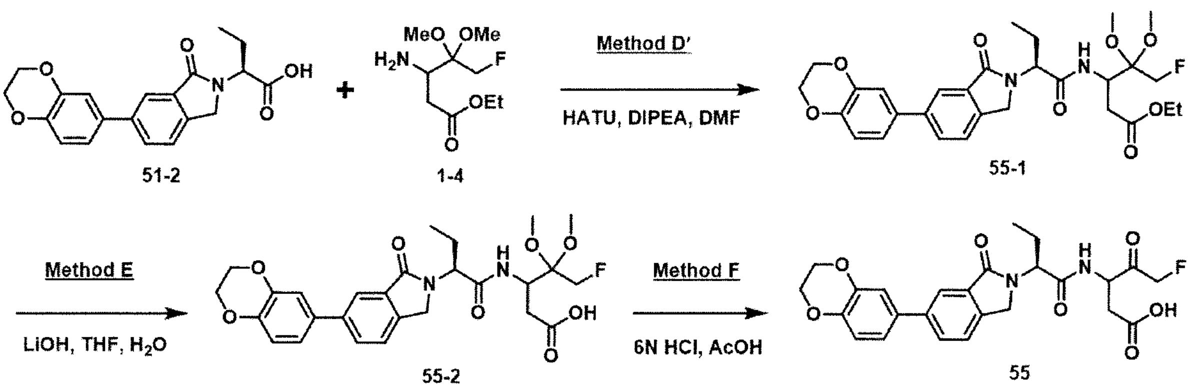


Using compound **51-2** and **1-4,** the target compound **55** was obtained using Method D7E/F sequentially.

$^1$H NMR (400 MHz, DMSO-$d_6$) δ (ppm) = 12.51 (s, 1H), 8.78 (dd, *J* = 10.8, 7.3 Hz, 1H), 7.92-7.76 (m, 2H), 7.69-7.60 (m, 1H), 7.20 (dd, *J* = 8.1, 2.0 Hz, 2H), 7.02-6.90 (m, 1H), 5.32-5.05 (m, 1H), 4.88-4.56 (m, 3H), 4.55-4.49 (m, 1H), 4.49-4.34 (m, 1H), 4.29 (s, 4H), 2.91-2.67 (m, 1H), 2.58 (ddd, *J* = 16.9, 7.3, 5.9 Hz, 1H), 1.96 (d, *J* = 17.8 Hz, 1H), 1.81 (dp, *J* = 15.0, 7.1 Hz, 1H), 0.94-0.75 (m, 3H).

MS (ESI, LR) Calculated for $C_{30}H_{25}F_4N_4O_7$ ($MH^+$): 485.2, found 485.2.

**Example 56. 5-fluoro-4-oxo-3-((*S*)-2-(1-oxo-6-(2-oxo-2,3-dihydro-1H-**benzo**[d]imidazol-5-yl)isoindolin-2-yl)butanamido)pentanoic acid**

<Scheme 56>

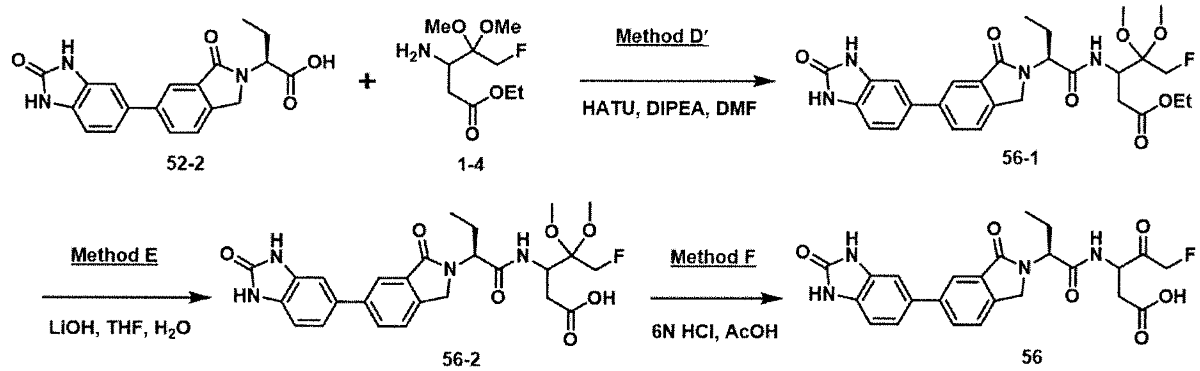


Using compound 52-2 and 1-4, the target compound 56 was obtained using Method D'/E/F sequentially.

$^1H$ NMR (400 MHz, DMSO-$d_6$) δ (ppm) = 12.51 (s, 1H), 10.72 (d, $J$ = 7.3 Hz, 2H), 8.96-8.31 (m, 1H), 7.85 (d, $J$ = 11.4 Hz, 2H), 7.67 (dd, $J$ = 8.3, 3.9 Hz, 1H), 7.30 (d, $J$ = 8.2 Hz, 1H), 7.21 (s, 1H), 7.03 (d, $J$ = 8.1 Hz, 1H), 5.36-5.06 (m, 1H), 4.91-4.57 (m, 3H), 4.51 (*d*, $J$ = 17.8 Hz, 2H), 2.78 (d, $J$= 17.1 Hz, 1H), 2.65-2.55 (m, 1H), 1.89 (d, $J$=62.7 Hz, 2H), 0.87 (qt, $J$ = 9.1, 6.4 Hz, 3H).

MS (ESI, LR) Calculated for $C_{30}H_{25}F_4N_4O_7$ ($MH^+$): 483.2, found 483.2.

**Example 57. 3-((*S*)-2-(5-(benzofuran-3-yl)-1-oxoisoindolin-2-yl)butanamido)-5-fluoro-4-oxopentanoic acid**

<Scheme 57>

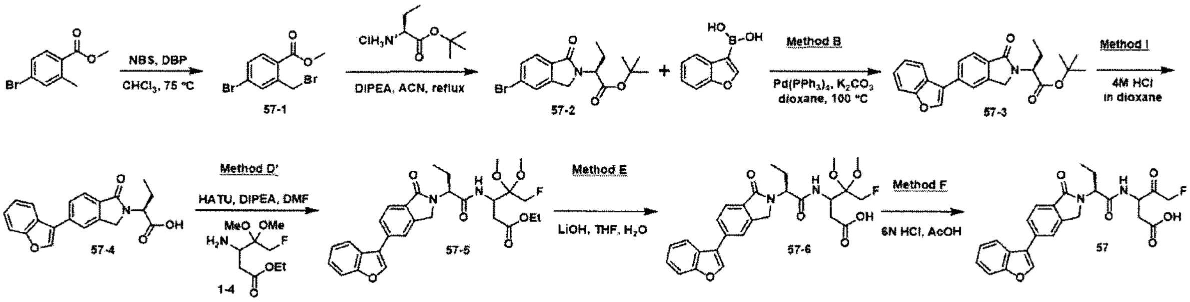


Step 57-1: 4-Bromo-2-methylbenzoate (10 g, 43.65 mmol) was dissolved in chloroform (50 mL), then NBS (7.77 g, 43.65 mmol) and dibenzoyl peroxide (0.528 g, 2.18 mmol) were added and the reaction was stirred at 75 °C for 1 hr. The reaction solution was washed with water (100 mL), the organic layer was dried over anhydrous magnesium sulfate, filtered and concentrated to give the target compound **50-1.** This concentrate was used in the next reaction without further purification.

Step 57-2: Compound **57-1** was dissolved in acetonitrile (150 mL) and [(1S)-1-tert-butoxycarboylpropyl]ammonium chloride (8.541 g, 43.65 mmol) and DIPEA (22.81 mL, 130.95 mmol) were added and the reaction was stirred at reflux for 5 hours. After completion of the reaction, the reaction solution was diluted with ethyl acetate (300 mL), then washed sequentially with water (100 mL), saturated aqueous solution of ammonium chloride (300 mL) and brine (300 mL), the organic layer was dried over magnesium anhydride, filtered, concentrated and separated by column chromatography to give the target compound **50-2** (10.4 g, 67% over 2 steps).

$^1H$ NMR (400 MHz, $CDCl_3$) δ (ppm) = 7.74-7.73(m, 1H), 7.64-7.61 (m, 2H), 4.99 (m, 1H), 4.61-4.36 (m, 2H), 2.16-1.84 (m, 2H), 1.49 (s, 9H), 0.97-0.94 (m, 3H).

Step 57-3: Using compound **57-2** and benzofuran-3-ylboronic acid as starting materials, the target compound **57-3** was obtained using Method B.

$^1H$ NMR (400 MHz, $CDCl_3$) δ (ppm) = 7.99-9.94 (m, 1H), 7.89-7.84 (m, 2H), 7.77-7.75 (m, 2H), 7.60-7.58 (m, 1H), 7.49-7.35 (m, 2H), 5.05-5.00 (m, 1H), 4.74-4.43 (m, 2H), 2.23-1.87 (m, 2H), 1.48 (s, 9H), 1.01-0.97 (m, 3H)

Step 57-4 (Method I): By reacting compound **57-3** with Method I, the target compound **57-4** was obtained.

$^1H$ NMR (400 MHz, DMSO-$d_6$) δ (ppm) = 8.08-8.07 (m, 1H), 8.01-7.99 (m, 1H), 7.92-7.90 (m, 1H), 7.81-7.66 (m, 5H), 4.78-4.68 (m, 1H), 4.62-4.48 (m, 2H), 2.13-1.82 (m, 2H), 0.92-0.85 (m, 3H).

Step 57-5 (Method D'): Using compound **57-4** and compound **1-4,** the target compound **57-5** was obtained using Method D'.

$^1H$ NMR (400 MHz, $CDCl_3$) δ (ppm) = 7.95-7.51 (m, 7H), 6.8 (s, 1H), 6.66-6.60 (m, 1H), 4.87-4.05 (m, 9H), 3.36-3.08 (m, 6H), 2.75-2.65 (m, 1H), 2.52-2.45 (m, 1H), 2.23-2.08 (m, 1H), 1.27-0.93 (m, 6H).

Step 57-6 (Method E): By reacting compound **57-5** with Method E, the target compound **57-6** was obtained.

$^1H$ NMR (400 MHz, DMSO-$d_6$) δ (ppm) = 8.41-7.51 (m, 7H), 7.05 (s, 1H), 4.86-4.25 (m, 6H), 3.34-3.23 (m, 6H), 2.63-2.45 (m, 2H), 2.03-1.71 (m, 2H), 0.84-0.82 (m, 3H).

Step 57-7 (Method F): By reacting compound **57-6** with Method F, the target compound **57** was obtained.

$^1H$ NMR (400 MHz, DMSO-$d_6$) δ (ppm) = 8.87-8.73 (m, 1H), 8.50 (s, 1H), 8.06-7.93 (m, 2H), 7.83-7.75 (m, 1H), 7.80-7.73 (m, 1H), 7.46-7.35 (m, 2H), 5.33-4.25 (m, 6H), 2.92-2.36 (m, 2H), 2.11-1.70 (m, 2H), 0.96-0.74 (m, 3H).

MS (ESI, LR) Calculated for $C_{25}H_{24}FN_2O_6$ ($MH^+$): 467.2, found: 467.1.

**Example 58. 3-((*S*)-2-(5-(2,3-dihydrobenzo[b][1,4]dioxin-6-yl)-1-oxoisoindolin-2-yl)butanamido)-5-fluoro-4-oxo-pentanoic acid**

<Scheme 58>

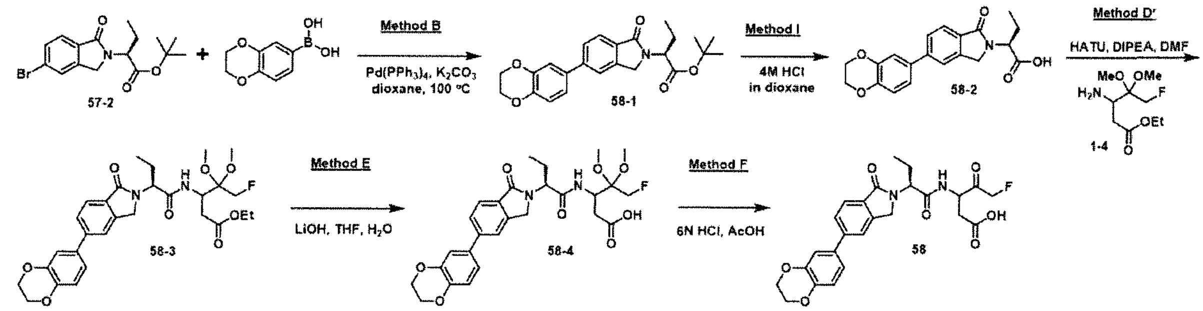


Using compound **57-2** and (2,3-dihydrobenzo[b][1,4]dioxin-6-yl)boronic acid, the target compound **58** was obtained using Method B/I/D'/E/F sequentially.

$^1H$ NMR (400 MHz, DMSO-$d_6$) δ (ppm) = 8.85-8.66 (m, 1H), 7.83-7.82 (m, 1H), 7.71 (s, 2H), 7.23-7.20 (m, 2H), 6.99-6.97 (m, 1H), 5.27-4.32 (m, 6H), 4.28 (s, 4H), 2.77-2.29 (m, 2H), 2.16-1.62 (m, 2H), 0.93-0.62 (m, 3H).

MS (ESI, LR) Calculated for $C_{25}H_{26}FN_2O_7$ ($MH^+$): 485.4 found: 485

**Example 59. 3-((*S*)-2-(5-(benzo[b]thiophen-3-yl)-1-oxoisoindolin-2-yl)butanamido)-5-fluoro-4-oxopentanoic acid**

<Scheme 59>

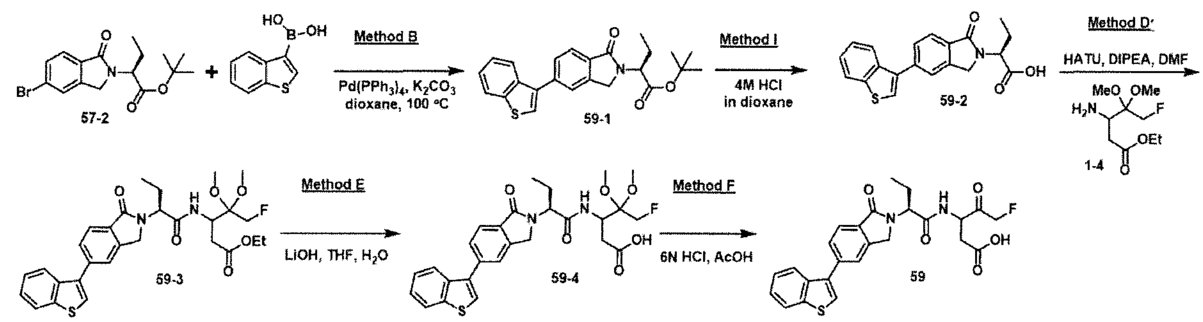

Using compound **57-2** and benzothiophen-3-ylboronic acid, the target compound **59** was obtained using Method B/I/D'/E/F sequentially.

$^1H$ NMR (400 MHz, DMSO-$d_6$) δ (ppm) = 8.79-8.72 (m, 1H), 8.13-8.10 (m, 1H), 7.97-7.96 (m, 2H), 7.86-7.75 (m, 2H), 7.71-7.68 (m, 1H), 7.48-7.46 (m, 2H), 5.37-4.41 (m, 6H), 2.54-2.46 (m, 2H), 2.16-1.61 (m, 2H), 1.00-0.75 (m, 3H).

MS (ESI, LR) Calculated for $C_{25}H_{24}FN_2O_5S$ ($MH^+$): 483.1, found: 483.1

**Example 60. 3-((*S*)-2-(5-(benzofuran-5-yl)-1-oxoisoindolin-2-yl)butanamido)-5-fluoro-4-oxopentanoic acid**

<Scheme 60>

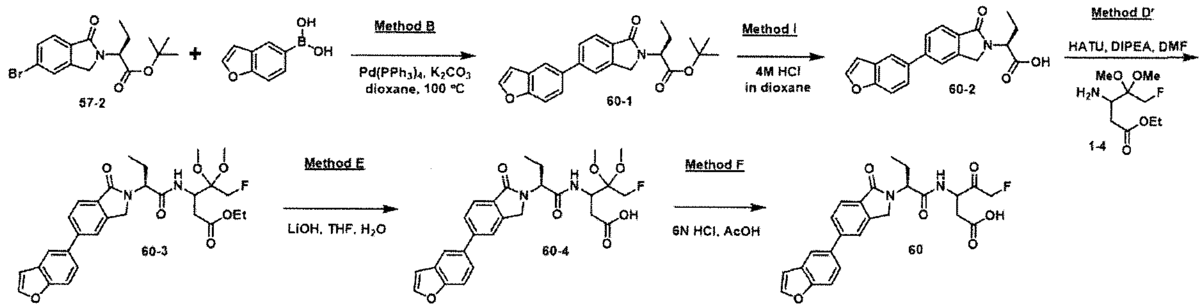


Using compound **57-2** and benzofuran-3-ylboronic acid, the target compound **60** was obtained using Method B/I/D'/E/F sequentially.

$^1H$ NMR (400 MHz, DMSO-$d_6$) δ (ppm) = 8.80-8.72 (m, 1H), 8.11-7.85 (m, 3H), 7.77-7.54 (m, 4H), 7.04 (s, 1H), 5.28-4.23 (m, 6H), 2.66-2.59 (m, 2H), 2.03-1.71 (m, 2H), 0.87-0.82 (m, 3H).

MS (ESI, LR) Calculated for $C_{25}H_{23}FN_2O_6$ ($MH^+$): 467.2, found: 467.1.

**Example 61. 5-fluoro-3-((*S*)-2-(5-(naphthalen-1-yl)-1-oxoisoindolin-2-yl)butanamido)-4-oxopentanoic acid**

<Scheme 61>

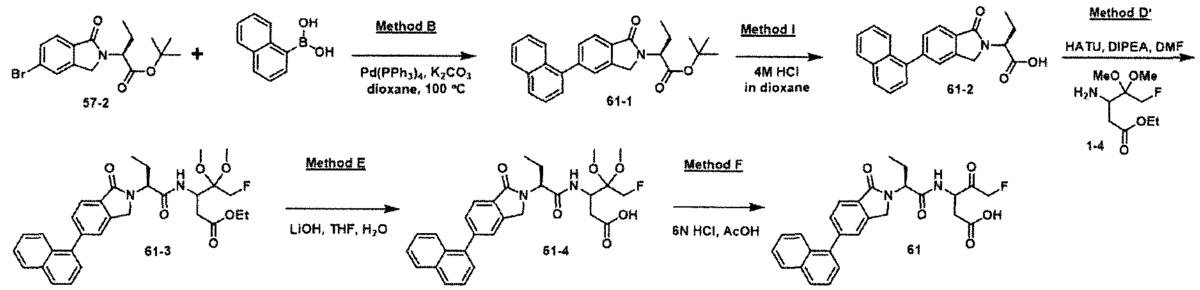


Using compound **57-2** and naphthalen-1-boronic acid, the target compound **61** was obtained using Method B/I/D'/E/F sequentially.

$^1H$ NMR (400 MHz, DMSO-$d_6$) δ (ppm) = 8.75-8.69 (m, 1H), 8.09 (m, 2H), 7.91-7.77 (m, 2H), 7.64-7.43 (m, 6H), 5.28-4.39 (m, 6H), 2.74-2.42 (m, 2H), 2.21-1.70 (m, 2H), 0.85-0.70 (m, 3H).

MS (ESI, LR) Calculated for $C_{27}H_{26}FN_2O_5$ ($MH^+$): 477.2, found: 477.2.

**Example 62. 5-fluoro-4-oxo-3-((S)-2-(1-oxo-5-(2-(trifluoromethyl)-5,6-dihydroimidazo[1,2-a]pyrazin-7(8H)-yl) isoindolin-2-yl)butanamido)pentanoic acid**

<Scheme 62>

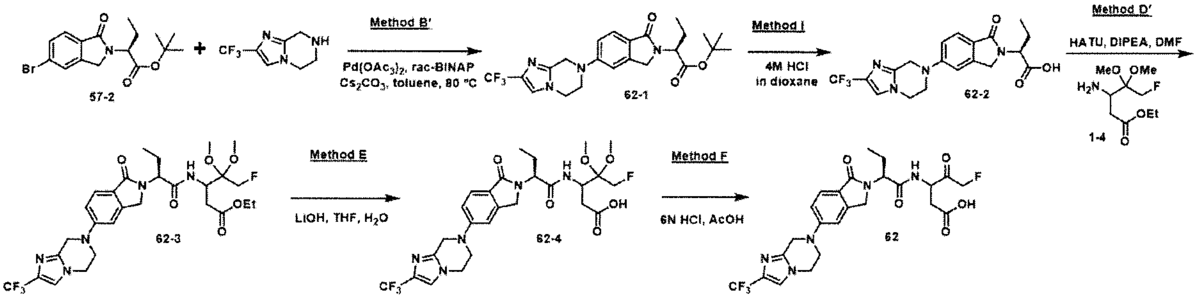


Using compound **57-2** and 2-(trifluoromethyl)-5,6,7,8-tetrahydroimidazo[1,2-a]pyrazine, the target compound **62** was obtained using Method B'/I/D'/E/F sequentially.

$^1$H NMR (400 MHz, DMSO-$d_6$) δ (ppm) = 8.67-8.59 (m, 1H), 7.80 (s, 1H), 7.57-7.45 (m, 1H), 7.23-7.18 (m, 2H), 5.39-3.67 (m, 12H), 2.14-1.62 (m, 2H), 1.05-0.60 (m, 3H).

MS (ESI, LR) Calculated for $C_{24}H_{26}F_4N_5O_5$ ($MH^+$): 540.2, found: 540.1.

**Example 63. 3-((*S*)-2-(5-(4-(benzo[d]isothiazol-3-yl)piperazin-1-yl)-1-oxoisoindolin-2-yl)butanamido)-5-fluoro-4-oxopentanoic acid**

<Scheme 63>

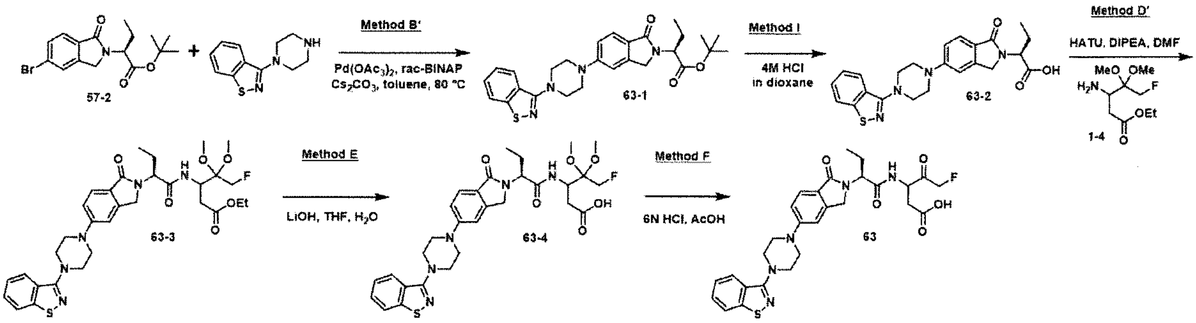


Using compound **57-2** and 3-(piperazin-1-yl)benzo[d]isothiazole, the target compound **63** was obtained using Method B'/I/D'/E/F sequentially.

$^1$H NMR (400 MHz, DMSO-$d_6$) δ (ppm) = 8.72-8.64 (m, 1H), 8.20-8.03 (m, 2H), 7.66-7.42 (m, 3H), 7.20-7.15 (m, 2H), 5.31-4.10 (m, 6H), 3.62 (s, 4H), 3.44 (s, 4H), 2.57-2.44 (m, 2H), 1.94-1.67 (m, 2H), 0.83-0.73(m, 3H).

MS (ESI, LR) Calculated for $C_{28}H_{31}FN_5O_5S$ ($MH^+$): 568.2, found: 568.2.

**Example 64. 5-fluoro-4-oxo-3-((*S*)-2-(1-oxo-5-(pyrrolidin-1-yl)isoindolin-2-yl)butanamido)pentanoic acid**

<Scheme 64>

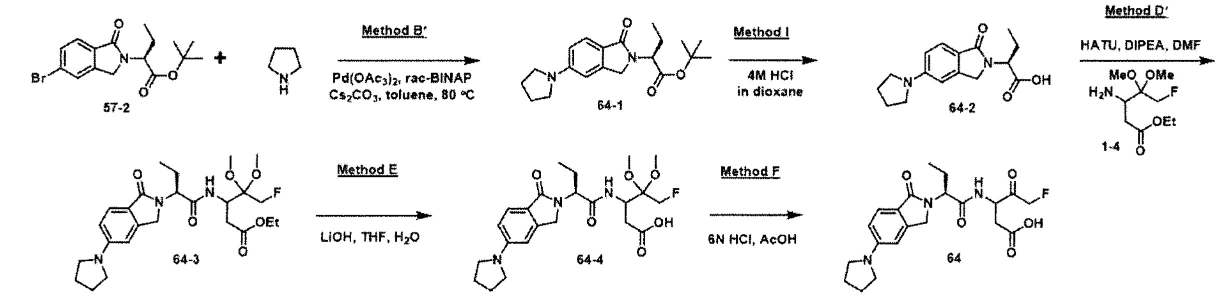


Using compound **57-2** and pyrrolidine, the target compound **64** was obtained using Method B'/I/D'/E/F sequentially.

$^1$H NMR (400 MHz, DMSO-$d_6$) δ (ppm) = 7.47-7.41 (m, 1H), 6.64-6.59 (m, 2H), 5.13-4.18 (m, 6H), 3.29 (s, 4H), 2.56-2.50 (m, 2H), 2.05-1.65 (m, 6H), 0.821-0.74 (m, 3H).

MS (ESI, LR) Calculated for $C_{21}H_{27}FN_3O_5$ ($MH^+$): 420.2, found: 420.2.

## Experimental example 1. Evaluation of Caspase-1 inhibitory activity

To determine the Caspase-1 inhibitory activity of the compounds of the present invention prepared in Example, Caspase-1 activity was measured using a substrate with a fluorescent dye attached to it. The fluorescent dye attached to the substrate is released from the substrate by caspase and exhibits fluorescence.

The compound was dissolved in DMSO and stored at -20°C. Enzymatic reactions were carried out in a buffer comprising 50 mM HEPES (pH 8.0), 50 mM KCl, 200 mM NaCl, 10 mM DTT, 0.1% CHAPS (w/v). Enzyme inactivation was measured in the presence of different concentrations of the compound using the fluorescent substrate N-Acetyl-Tyr-Val-Ala-Asp-7-amido-4-trifluoromethylcoumarin (Ac-YVAD-AFC) (Sigma) at a concentration of 50 μM and 1 unit of recombinant human Caspase-1 (Enzo life science). A CLARIOstar Plus spectrometer (BMG Labtech) was used to measure the reaction rate as a function of time in the 400/505 nm (Ex/Em) wavelength band. The inhibition rate constant of the compound, $k_{observance}$ ($k_{obs}$), was derived from Equation 1 below and expressed as $k_{obs}$/[I] by linear regression analysis of concentration versus $k_{obs}$. If $k_{obs}$/[I] is 20 or more, it is denoted as A, if it is 10 or more but less than 20, it is denoted as B, if it is 1 or more but less than 10, it is denoted as C, and if it is less than 1, it is denoted as D.

<Equation 1>

$$k_{obs} = -\ln(1-A_t/A_{max})/t$$

In the above equation, At means the enzyme reaction rate at time t (min), and $A_{max}$ means the maximum reaction rate.

As a result, as shown in Table 1, it was confirmed that each compound inhibited Caspase-1 activity, thus confirming that the compounds of the present invention are direct Caspase-1 inhibitors.

## Experimental example 2. Measurement of pro-inflammatory interleukin-1β inhibitory activity in human monocytic cells THP-1

Caspase-1 is known as IL-1β converting enzyme (ICE). Activated Caspase-1 is involved in the cleavage of proform IL-1β to produce the mature form, which is then secreted to induce inflammation. Therefore, the compounds identified in Experimental example 1 were tested to determine if their Caspase-1 inhibitory activity affected the production of IL-1β.

THP-1 (ATCC) cells were cultured in RPMI 1640 (Hyclone) growth medium comprising 10% Fetal bovine serum (FBS, Gibco) and antibiotics (100 U/ml Penicillin, 0.1 mg/ml Streptomycin, Cytiva) under 37 °C, 5% $CO_2$ conditions. Cultured THP-1 cells were seeded in growth medium to a concentration of 3 x $10^5$ cells/well in 24-well plates (Celvest) and then treated with 1 μM Phorbol 12-myristate 13-acetate (PMA) (Sigma) for 24 hours to induce differentiation. Differentiated THP-1 were pre-treated with 10 μg/ml Lipopolysaccharide (LPS, from *E. coli* 0111:B4 (InvivoGen)) in serum free RPMI 1640 medium for 24 hours to induce an inflammatory response. After removal of LPS, the compounds were pretreated for 1 h at a concentration of 100 nM using the same medium, followed by the addition of 5 mM ATP (InvivoGen) and incubation for 24 h in an incubator. The treated medium was collected and centrifuged, and IL-1β was quantified using an Enzyme

linked immunoassay kit (ELISA kit, R&D system), and absorbance was measured at 450 nm using a CLARIOstar Plus spectrometer. Inhibitory activity was denoted as a if it was 50% or more, b if it was 30% or more but less than 50%, c if it was 10% or more but less than 30%, and d if it was less than 10%.

As shown in Table 1, treatment with the compounds of the present invention resulted in a decrease in IL-1β. Therefore, we confirmed the potential of the compounds of the present invention to inhibit inflammation by reducing IL-1β. In particular, since intracellular Caspase-1 is involved in the maturation of IL-1β, we confirmed the association with the Caspase-1 inhibitory activity identified in Experimental Example 1 above.

**Experimental example 3. Measurement of apoptosis inhibition activity in human T-lymphocyte cells Jurkat**

Apoptosis, a type of programmed cell death, is a genetically regulated pathway that plays an important role in maintaining cellular homeostasis. It is typically induced through either an exogenous pathway via apoptosis receptors or an endogenous pathway via mitochondria. Caspase-8 and 9, known as initiators, are activated by exogenous and endogenous factors, respectively, and mediate cell death by activating the downstream executioner Caspase-3. Morphologically, apoptosis is characterized by cell shrinkage, DNA fragmentation, and a decrease in intracellular ATP.

To evaluate the effect of the compound of the present invention on apoptosis, Jurkat (ATCC) cells were treated with anti-FAS antibody CH-11 (Merck) to induce apoptosis via the Fas (CD95) receptor, which was assessed by the amount of intracellular ATP.

Jurkat cells were cultured in RPMI 1640 growth medium comprising 10% FBS and antibiotics (100 U/ml Penicillin, 0.1 mg/ml Streptomycin) under 37 °C, 5% $CO_2$ conditions. Cultured cells were seeded in growth medium to a concentration of 8 x $10^4$ cells/well in 96-well plates (SPL), pretreated with compound at a concentration of 50 nM for 1 hour, and then treated with 1 μg/ml anti-FAS antibody for 24 hours in an incubator. After 24 h, an ATP lite Luminescence assay kit (PerkinElmer) was used to measure ATP and luminescence was measured at 580 nm using a CLARIOstar Plus spectrometer. If the inhibitory activity is 30% or more, it is denoted as a', if it is 20% or more but less than 30%, it is denoted as b', if it is 10% or more but less than 20%, it is denoted as c', and if it is less than 10%, it is denoted as d'.

As shown in Table 1, treatment with the compounds reduced apoptosis, thus confirming that the compounds of the present invention are effective against apoptosis.

[Table 1]

| | $k_{obs}$/[I] ($min^{-1}M^{-1}$) | % of inhibition | |
|---|---|---|---|
| compound | Caspase-1 | Secreted IL-1β | Apoptosis |
| 1 | C | c | b' |
| 2 | C | b | c' |
| 3 | C | b | b' |
| 4 | C | b | c' |
| 5 | C | b | c' |
| 6 | D | c | c' |
| 7 | C | d | NA |
| 8 | C | a | c' |
| 9 | C | b | a' |
| 10 | C | b | c' |
| 11 | D | b | NA |
| 12 | C | c | NA |
| 13 | C | a | b' |
| 14 | C | d | NA |
| 15 | D | d | NA |
| 16 | A | a | b' |
| 17 | C | c | NA |
| 18 | C | a | c' |

(continued)

| | $k_{obs}/[I]$ ($min^{-1}M^{-1}$) | % of inhibition | |
|---|---|---|---|
| compound | Caspase-1 | Secreted IL-1β | Apoptosis |
| 19 | D | d | NA |
| 20 | C | c | NA |
| 21 | C | b | NA |
| 22 | A | b | c' |
| 23 | C | d | NA |
| 24 | C | c | NA |
| 25 | C | c | NA |
| 26 | C | b | a' |
| 27 | C | c | a' |
| 28 | C | c | NA |
| 29 | C | d | d' |
| 30 | B | d | d' |
| 31 | C | d | NA |
| 32 | C | d | NA |
| 33 | C | d | NA |
| 34 | C | d | NA |
| 35 | C | d | NA |
| 36 | C | d | NA |
| 37 | C | c | d' |
| 38 | D | d | d' |
| 39 | C | d | d' |
| 40 | C | d | d' |
| 41 | C | d | d' |
| 42 | C | b | a' |
| 43 | D | c | NA |
| 44 | B | a | b' |
| 45 | C | b | c' |
| 46 | D | NA | NA |
| 47 | C | NA | NA |
| 48 | D | NA | NA |
| 49 | B | NA | NA |
| 50 | B | a | a' |
| 51 | B | b | b' |
| 52 | A | c | c' |
| 53 | C | d | NA |
| 54 | C | b | d' |
| 55 | C | b | a' |
| 56 | B | a | d' |

(continued)

| | $k_{obs}/[I]$ ($min^{-1}M^{-1}$) | % of inhibition | |
|---|---|---|---|
| compound | Caspase-1 | Secreted IL-1β | Apoptosis |
| 57 | C | c | b' |
| 58 | C | c | c' |
| 59 | D | d | NA |
| 60 | D | d | NA |
| 61 | D | d | NA |
| 62 | D | NA | NA |
| 63 | D | NA | NA |
| 64 | D | NA | NA |

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. An isoindolinone derivative compound represented by the following Formula I , a hydrate thereof, a solvate thereof or a pharmaceutically acceptable salt thereof, wherein:

<Formula I >

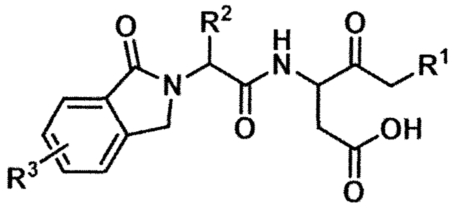

$R^1$ is halogen, or aryloxy substituted with 1 to 5 halogens,
$R^2$ is hydrogen, or $C_{1-6}$ straight or branched alkyl chain,
$R^3$ is halogen or Q, Q is substituted or unsubstituted with 1 to 3 independent $R^a$,
Q is aryl, aminoaryl, heteroaryl or non-aromatic heterocyclic, and Q is optionally fused with saturated or unsaturated 5- to 7-membered ring having 0 to 3 heteroatoms,
$R^a$ is $C_{1-6}$ straight or branched alkyl chain, $C_{1-6}$ alkoxy, halogen, $C_{1-6}$ haloalkyl, $CO_2R^b$, $COR^b$, $CONHR^b$, $CON(R^b)_2$, $NHR^b$, $N(R^b)_2$, $NHCOR^b$, $S(O)_2R^b$, or heteroaryl fused to a saturated or unsaturated 5- to 7-membered ring having 0 to 3 heteroatoms,
$R^b$ is hydrogen or $C_{1-6}$ straight or branched alkyl chain.

2. The isoindolinone derivative compound, a hydrate thereof, a solvate thereof or a pharmaceutically acceptable salt thereof of Claim 1, **characterized in that** $R^1$ is F, or phenoxy substituted with 1 to 5 F.

3. The isoindolinone derivative compound, a hydrate thereof, a solvate thereof or a pharmaceutically acceptable salt thereof of Claim 1, **characterized in that** $R^2$ is hydrogen, methyl, ethyl, or straight or branched chain propyl.

4. The isoindolinone derivative compound, a hydrate thereof, a solvate thereof or a pharmaceutically acceptable salt thereof of Claim 1, **characterized in that** $R^3$ is Br or Q, Q is phenyl, pyridinyl, tetrahydropyridinyl, pyrazolyl, pyrimidinyl, dihydropyranyl, thiophenyl, pyrrolidinyl, piperazinyl, aminonaphthalenyl, naphthalenyl, quinolinyl, isoquinolinyl, benzofuranyl, benzothiazolyl, dihydrobenzodioxynyl, oxo-dihydrobenzoimidazolyl, benzothiophenyl, pyrazolopyridinyl or dihydroimidazopyrazinyl.

5. The isoindolinone derivative compound, a hydrate thereof, a solvate thereof or a pharmaceutically acceptable salt thereof of Claim 1, **characterized in that** $R^a$ is methyl, methoxy, F, trifluoromethyl, carboxy, acetyl, amino, methylsulfonyl or benzoisothiazolyl.

6. The isoindolinone derivative compound, a hydrate thereof, a solvate thereof or a pharmaceutically acceptable salt thereof of Claim 1, **characterized in that** the isoindolinone derivative compound represented by Formula I is selected from the group consisting of:

[1] 5-fluoro-4-oxo-3-(2-(1-oxo-6-phenylisoindolin-2-yl)butanamido)pentanoic acid,
[2] 5-fluoro-3-(2-(6-(naphthalen-1-yl)-1-oxoisoindolin-2-yl)butanamido)-4-oxopentanoic acid,
[3] 5-fluoro-4-oxo-3-(2-(1-oxo-6-(pyrrolidin-1-yl)isoindolin-2-yl)butanamido)pentanoic acid,
[4] 5-fluoro-4-oxo-3-(2-(1-oxo-6-(pyridin-3-yl)isoindolin-2-yl)butanamido)pentanoic acid,
[5] 5-fluoro-4-oxo-3-(2-(1-oxo-6-(1H-pyrazol-4-yl)isoindolin-2-yl)butanamido)pentanoic acid,
[6] 5-fluoro-3-(2-(6-(4-(methylsulfonyl)piperazin-1-yl)-1-oxoisoindolin-2-yl)butanamido)-4-oxopentanoic acid,
[7] 5-fluoro-3-(2-(6-(1-(methylsulfonyl)-1,2,3,6-tetrahydropyridin-4-yl)-1-oxoisoindolin-2-yl)butanamido-4-oxopentanoic acid,
[8] 5-fluoro-4-oxo-3-(2-(1-oxo-6-(2-oxo-2,3-dihydro-1H-benzo[d]imidazol-5-yl)isoindolin-2-yl)butanamido)pentanoic acid,
[9] 3-(2-(6-bromo-1-oxoisoindolin-2-yl)butanamido)-5-fluoro-4-oxopentanoic acid,
[10] 5-fluoro-4-oxo-3-(2-(1-oxo-6-(trifluoromethyl)-5,6-dihydroimidazo[1,2-a]pyrazin-7(8H)-yl)isoindolin-2-yl) butanamido)pentanoic acid,
[11] 5-fluoro-3-(2-(6-(naphthalen-1-ylamino)-1-oxoisoindolin-2-yl)butanamido)-4-oxopentanoic acid,
[12] 5-fluoro-3-(2-(6-(2-fluoro-4-(methylsulfonyl)phenyl)-1-oxoisoindolin-2-yl)butanamido)-4-oxopentanoic acid,
[13] 5-fluoro-3-(2-(6-(6-methoxypyridin-3-yl)-1-oxoisoindolin-2-yl)butanamido)-4-oxopentanoic acid,
[14] 5-fluoro-4-oxo-3-(2-(1-oxo-6-(4-(trifluoromethyl)phenyl)isoindolin-2-yl)butanamido)pentanoic acid,
[15] 3-(2-(6-(3,6-dihydro-2H-pyran-4-yl)-1-oxoisoindolin-2-yl)butanamido)-5-fluoro-4-pentanoic acid,
[16] 3-(2-(6-benzo[b]thiophen-3-yl)-1-oxoisoindolin-2-yl)butanamido)-5-fluoro-4-oxopentanoic acid,
[17] 4-(2-(1-((1-carboxy-4-fluoro-3-oxobutan-2-yl)amino)-1-oxobutan-2-yl)-3-oxoisoindolin-5-yl)pentanoic acid,
[18] 3-(2-(6-(4-acetylphenyl)-1-oxoisoindolin-2-yl)butanamido-5-fluoro-4-oxopentanoic acid,
[19] 3 -(2-(6-(4-(benzo [d] isothiazol-3 -yl)piperazin-1 -yl)-1 -oxoisoindolin-2-yl)butanamido)-5-fluoro-4-oxopentanoic acid,
[20] 5-fluoro-4-oxo-3-(2-(1-oxo-6-(6-(trifluoromethyl)pyridin-3-yl)isoindolin-2-yl)butanamido)pentanoic acid,
[21] (3-(2-(6-(2-aminopyrimidin-5-yl)-1-oxoisoindolin-2-yl)butanamido)-5-fluoro-4-oxopentanoic acid,
[22] 3-(2-(6-(benzofuran-3-yl)-1-oxoisoindolin-2-yl)butanamido)-5-fluoro-4-oxopentanoic acid,
[23] 3-(2-(6-(2-aminopyridin-4-yl)-1-oxoisoindolin-2-yl)butanamido)-5-fluoro-4-oxopentanoic acid,
[24] 5-fluoro-3-(2-(6-(1-methyl-1H-pyrazol-4-yl)-1-oxoisoindolin-2-yl)butanamido)-4-oxopentanoic acid,
[25] 5-fluoro-3-(2-(6-(1-methyl-1H-pyrazol-3-yl)-1-oxoisoindolin-2-yl)butanamido)-4-oxopentanoic acid,
[26] 3-(2-(6-benzofuran-5-yl)-1-oxoisoindolin-2-yl)butanamido)-5-fluoro-4-oxopentanoic acid,
[27] 3-(2-(6-(2,3-dihydrobenzo[b][1,4] dioxin-6-yl)-1-oxoisoindolin-2-yl)butanamido)-5-fluoro-4-oxopentanoic acid,
[28] 5-fluoro-4-oxo-3-(2-(1-oxo-6-(thiophen-3-yl)isoindolin-2-yl)butanamido)pentanoic acid,
[29] 5-fluoro-3-(2-(6-(isoquinolin-4-yl)-1-oxoisoindolin-2-yl)butanamido)-4-oxopentanoic acid,
[30] 5-fluoro-4-oxo-3-(2-(1-oxo-6-(quinolin-6-yl)isoindolin-2-yl)butanamido)pentanoic acid,
[31] 5-fluoro-4-oxo-3-(2-(1-oxo-6-(quinolin-4-yl)isoindolin-2-yl)butanamido)pentanoic acid,
[32] 3-(2-(6-(benzo[d]thiazol-5-yl)-1-oxoisoindolin-2-yl)butanamido)-5-fluoro-4-oxopentanoic acid,
[33] 5-fluoro-4-oxo-3-(2-(1-oxo-6-(pyrazolo[1,5-a]pyridin-3-yl)isoindolin-2-yl)butanamido)pentanoic acid,
[34] 5-fluoro-4-oxo-3-(2-(1-oxo-6-(quinolin-5-yl)isoindolin-2-yl)butanamido)pentanoic acid,
[35] 5-fluoro-3-(2-(6-(isoquinolin-8-yl)-1-oxoisoindolin-2-yl)butanamido)-4-oxopentanoic acid,

[36] 5-fluoro-3-(2-(6-(isoquinolin-5-yl)-1-oxoisoindolin-2-yl)butanamido)-4-oxopentanoic acid,
[37] 5-fluoro-4-oxo-3-(2-(1-oxo-6-(quinolin-3-yl)isoindolin-2-yl)butanamido)pentanoic acid,
[38] 5-fluoro-4-oxo-3-(2-(1-oxo-6-(2-oxo-2,3-dihydro-1H-benzo[d]imidazol-5-yl)isoindolin-2-yl)acetamido)pentanoic acid,
[39] 3-(2-(6-(benzofuran-3-yl)-1-oxoisoindolin-2-yl)acetamido)-5-fluoro-4-oxopentanoic acid,
[40] 3-(2-(6-(benzo[b]thiophen-3-yl)-1-oxoisoindolin-2-yl)acetamido)-5-fluoro-4-oxopentanoic acid,
[41] 3-(2-(6-(benzofuran-5-yl)-1-oxoisoindolin-2-yl)acetamido)-5-fluoro-4-oxopentanoic acid,
[42] 3-((S)-2-(6-(2,3-dihydrobenzo[b][1,4]dioxin-6-yl)-1-oxoisoindolin-2-yl)-3-methylbutanamido)-5-fluoro-4-oxopentanoic acid,
[43] 5-fluoro-3-((S)-3-methyl-2-(1 -oxo-6-(pyrrolidin-1 -yl)isoindolin-2-yl)butanamido)4-oxopentanoic acid,
[44] 3 -((S)-2-(6-(benzofuran-3 -yl)-1 -oxoisoindolin-2-yl)-3 -methylbutanamido)-5 - fluoro-4-oxopentanoic acid,
[45] 5-fluoro-3-((S)-3-methyl-2-(1-oxo-6-(2-oxo-2,3-dihydro-1H-benzo[d]imidazol-5-yl)isoindolin-2-yl)butanamido)-4-oxopentanoic acid,
[46] 5-fluoro-4-oxo-3-(2-(1-oxo-6-(pyrrolidin-1-yl)isoindolin-2-yl)propanamido)pentanoic acid,
[47] 5-fluoro-4-oxo-3-(2-(1-oxo-6-(2-oxo-2,3-dihydro-1H-benzo[d]imidazol-5-yl)isoindolin-2-yl)propanamido) pentanoic acid,
[48] 3-(2-(6-(2,3-dihydrobenzo [b][1,4] dioxin-6-yl)-1 -oxoisoindolin-2-yl)propanamido-5-fluoro-4-oxopentanoic acid,
[49] 3 -(2-(6-(benzofuran-3 -yl)-1 -oxoisoindolin-2-yl)propanamido)-5 -fluoro-4-oxopentanoic acid,
[50] (*S*)-3-((*S*)-2-(6-(benzofuran-3-yl)-1-oxoisoindolin-2-yl)butanamido-4-oxo-5-(2,3,5,6-tetrafluorophenoxy) pentanoic acid,
[51] (*S*)-3-((*S*)-2-(6-(2,3-dihydrobenzo[b][1,4]dioxin-6-yl)-1-oxoisoindolin-2-yl)butanamido)-4-oxo-5-(2,3,5,6-tetrafluorophenoxy)pentanoic acid,
[52] (*S*)-4-oxo-3-((*S*)-2-(1-oxo-6-(2-oxo-2,3-dihydro-1H-benzo[d]imidazol-5-yl)isoindolin-2-yl)butanamido)-5-(2,3,5,6-tetrafluorophenoxy)pentanoic acid,
[53] (*S*)-4-oxo-3-((*S*)-2-(1-oxo-6-(pyrrolidin-1-yl)isoindolin-2-yl)butanamido)-5-(2,3,5,6-tetrafluorophenoxy) pentanoic acid,
[54] (*R*)-3-((*S*)-2-(6-(2,3-dihydrobenzo[b][1,4]dioxin-6-yl)-1-oxoisoindolin-2-yl)butanamido)-4-oxo-5-(2,3,5,6-tetrafluorophenoxy)pentanoic acid,
[55] 3-((*S*)-2-(6-(2,3-dihydrobenzo[b][1,4]dioxin-6-yl)-1 -oxoisoindolin-2-yl)butanamido)-5-fluoro-4-oxopentanoic acid,
[56] 5-fluoro-4-oxo-3-((*S*)-2-(1-oxo-6-(2-oxo-2,3-dihydro-1H-benzo[d]imidazol-5-yl)isoindolin-2-yl)butanamido) pentanoic acid,
[57] 3-((*S*)-2-(5-(benzofuran-3-yl)-1-oxoisoindolin-2-yl)butanamido)-5-fluoro-4-oxopentanoic acid,
[58] 3-((*S*)-2-(5-(2,3-dihydrobenzo[b][1,4]dioxin-6-yl)-1-oxoisoindolin-2-yl)butanamido)-5-fluoro-4-oxopentanoic acid,
[59] 3-((*S*)-2-(5-(benzo[b]thiophen-3-yl)-1-oxoisoindolin-2-yl)butanamido)-5-fluoro-4-oxopentanoic acid,
[60] 3-((*S*)-2-(5-(benzofuran-5-yl)-1-oxoisoindolin-2-yl)butanamido)-5-fluoro-4-oxopentanoic acid,
[61] 5-fluoro-3-((*S*)-2-(5-(naphthalen-1-yl)-1-oxoisoindolin-2-yl)butanamido)-4-oxopentanoic acid,
[62] 5-fluoro-4-oxo-3-((*S*)-2-(1-oxo-5-(2-(trifluoromethyl)-5,6-dihydroimidazo[1,2-a]pyrazin-7(8H)-yl)isoindolin-2-yl)butanamido)pentanoic acid,
[63] 3 -((*5*)-2-(5-(4-(benzo [d] isothiazol-3-yl)piperazin-1-yl)-1 -oxoisoindolin-2-yl)butanamido)-5-fluoro-4-oxopentanoic acid, 및
[64] 5-fluoro-4-oxo-3-((*S*)-2-(1-oxo-5-(pyrrolidin-1-yl)isoindolin-2-yl)butanamido)pentanoic acid.

**7.** A pharmaceutical composition for preventing or treating caspase-related disease comprising the derivative compound, a hydrate thereof, a solvate thereof or a pharmaceutically acceptable salt thereof of any one of Claims 1 to 6 as an active ingredient.

**8.** The pharmaceutical composition of Claim 7, **characterized in that** the caspase-related disease is osteoarthritis or pain.

**9.** The pharmaceutical composition of Claim 7, **characterized in that** preventing or treating caspase-related disease is via a decrease in IL-1β or a decrease in apoptosis.

**10.** A pharmaceutical composition comprising the isoindolinone derivative compound, a hydrate thereof, a solvate thereof or a pharmaceutically acceptable salt thereof of any one of Claims 1 to 6, and a pharmaceutically acceptable additive.

**11.** Method for preparing an isoindolinone derivative compound represented by the following Formula I , comprising:

preparing a compound of Formula I-2 by reacting a compound of Formula I -1 below with one of the compounds of $R^3$-B(OH)$_2$, $R^3$-H, and Formula I-c below;
hydrolyzing the compound of Formula I-2 below to prepare the compound of Formula I-3 below;
preparing a compound of Formula I-5 below by reacting the compound of Formula I-3 below with a compound of Formula I-4 below;
hydrolyzing the compound of Formula I-5 below to prepare the compound of Formula I-6 below; and
acidifying the compound of Formula I -6 below.

<Formula I -1>

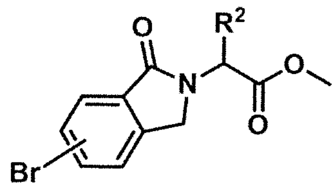

<Formula I -c>

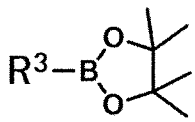

<Formula I -2>

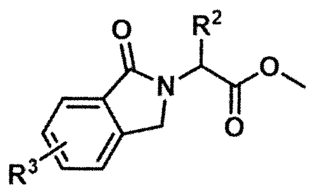

<Formula I -3>

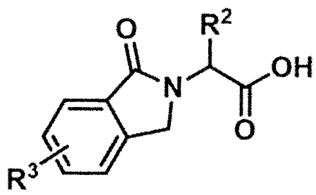

<Formula I -4>

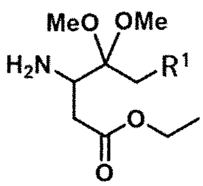

<Formula I -5>

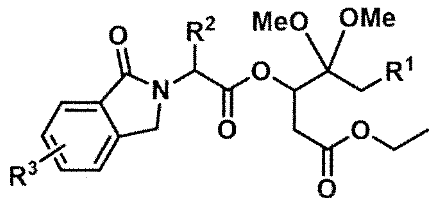

<Formula I -6>

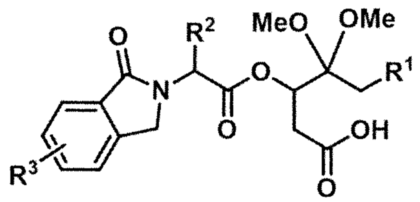

In the above formula, $R^1$, $R^2$, and $R^3$ are the same as defined in Formula I of Claim 1.

**12.** The method of Claim, **characterized in that** the compound of Formula I -1 is obtained by reacting the compound of Formula I -a below with the compound of Formula I -b below:

<Formula I -a>

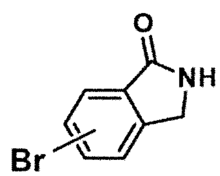

<Formula I -b>

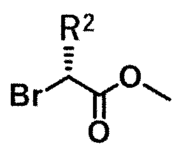

In the above formula, the definition of $R^2$ is the same as the definition of Formula I in Claim 1.

## INTERNATIONAL SEARCH REPORT

International application No.
**PCT/KR2023/000101**

**A. CLASSIFICATION OF SUBJECT MATTER**

**C07D 209/44**(2006.01)i; **A61K 31/4035**(2006.01)i; **A61K 31/4439**(2006.01)i; **A61K 31/4155**(2006.01)i; **A61K 31/506**(2006.01)i; **A61P 19/02**(2006.01)i; **C07D 403/04**(2006.01)i; **C07D 401/04**(2006.01)i; **C07D 487/04**(2006.01)i; **C07D 405/04**(2006.01)i

According to International Patent Classification (IPC) or to both national classification and IPC

**B. FIELDS SEARCHED**

Minimum documentation searched (classification system followed by classification symbols)

C07D 209/44(2006.01); C07D 213/64(2006.01); C07D 239/36(2006.01); C07D 401/06(2006.01); C07D 401/14(2006.01); C07D 403/06(2006.01); C07D 409/14(2006.01); C07D 413/12(2006.01); C07D 413/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 이소인돌리논 유도체 화합물(isoindolinone derivative compound), 캐스파제 저해제(caspase inhibitor), 세포사멸(apoptotic cell death)

**C. DOCUMENTS CONSIDERED TO BE RELEVANT**

| Category* | Citation of document, with indication, where appropriate, of the relevant passages | Relevant to claim No. |
|---|---|---|
| A | KR 10-2008-0022594 A (VERTEX PHARMACEUTICALS INCORPORATED) 11 March 2008 (2008-03-11) See claims 15 and 20; paragraphs [0097]-[0099], [0142], [0143], [0145], [0146], [0149], [0150], [0221]-[0224], [0324] and [0340]; and example 14. | 1-12 |
| DA | KR 10-2006-0094868 A (LG LIFE SCIENCES LTD.) 30 August 2006 (2006-08-30) See entire document. | 1-12 |
| A | US 2004-0048797 A1 (MILLER, K. et al.) 11 March 2004 (2004-03-11) See entire document. | 1-12 |
| A | US 2017-0260175 A1 (VERTEX PHARMACEUTICALS INCORPORATED) 14 September 2017 (2017-09-14) See entire document. | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 April 2023** | **14 April 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/000101**

**C. DOCUMENTS CONSIDERED TO BE RELEVANT**

| Category* | Citation of document, with indication, where appropriate, of the relevant passages | Relevant to claim No. |
|---|---|---|
| A | JP 2018-535927 A (ASTEX THERAPEUTICS LIMITED et al.) 06 December 2018 (2018-12-06) See entire document. | 1-12 |

Form PCT/ISA/210 (second sheet) (July 2022)

**INTERNATIONAL SEARCH REPORT**
**Information on patent family members**

International application No.
**PCT/KR2023/000101**

| Patent document cited in search report | Publication date (day/month/year) | Patent family member(s) | Publication date (day/month/year) |
|---|---|---|---|
| KR 10-2008-0022594 A | 11 March 2008 | AR 026748 A1 | 26 February 2003 |
| | | AU 2001-24283 A1 | 18 June 2001 |
| | | AU 2006-225317 A1 | 02 November 2006 |
| | | AU 2010-202075 A1 | 17 June 2010 |
| | | AU 2010-202075 B2 | 29 September 2011 |
| | | AU 2428301 A | 18 June 2001 |
| | | BR 0016282 A | 27 August 2002 |
| | | CA 2393710 A1 | 14 June 2001 |
| | | CA 2393710 C | 24 May 2011 |
| | | CA 2733434 A1 | 14 June 2001 |
| | | CN 100415718 C | 03 September 2008 |
| | | CN 101348455 A | 21 January 2009 |
| | | CN 1420872 A | 28 May 2003 |
| | | CO 5261609 A1 | 31 March 2003 |
| | | CZ 20021970 A3 | 16 October 2002 |
| | | EA 200200645 A1 | 26 December 2002 |
| | | EP 1244626 A2 | 02 October 2002 |
| | | EP 2308844 A2 | 13 April 2011 |
| | | EP 2308844 A3 | 10 August 2011 |
| | | EP 2308845 A2 | 13 April 2011 |
| | | EP 2308845 A3 | 10 August 2011 |
| | | EP 2308846 A2 | 13 April 2011 |
| | | EP 2308846 A3 | 10 August 2011 |
| | | HU 0300782 A2 | 29 September 2003 |
| | | HU 0300782 A3 | 28 November 2003 |
| | | IS 6411 A | 06 June 2002 |
| | | JP 2003-516393 A | 13 May 2003 |
| | | JP 2008-101019 A | 01 May 2008 |
| | | JP 2012-021032 A | 02 February 2012 |
| | | JP 5021131 B2 | 05 September 2012 |
| | | KR 10-0904697 B1 | 29 June 2009 |
| | | KR 10-0919654 B1 | 30 September 2009 |
| | | KR 10-2002-0060259 A | 16 July 2002 |
| | | KR 10-2009-0035042 A | 08 April 2009 |
| | | MX PA02005779 A | 08 September 2005 |
| | | NO 20022656 L | 06 August 2002 |
| | | NO 20074773 A | 06 August 2002 |
| | | NO 324776 B1 | 10 December 2007 |
| | | NZ 519424 A | 26 March 2004 |
| | | NZ 530485 A | 24 February 2006 |
| | | PL 356066 A1 | 14 June 2004 |
| | | SK 8072002 A3 | 04 February 2003 |
| | | TW I275586 B | 11 March 2007 |
| | | US 2003-0232846 A1 | 18 December 2003 |
| | | US 2009-0131456 A1 | 21 May 2009 |
| | | US 2011-0130436 A1 | 02 June 2011 |
| | | US 2012-0077830 A1 | 29 March 2012 |
| | | US 7517987 B2 | 14 April 2009 |
| | | US 7906650 B2 | 15 March 2011 |
| | | US 8093392 B2 | 10 January 2012 |

Form PCT/ISA/210 (patent family annex) (July 2022)

**INTERNATIONAL SEARCH REPORT**
**Information on patent family members**

International application No.
**PCT/KR2023/000101**

| Patent document cited in search report | Publication date (day/month/year) | Patent family member(s) | Publication date (day/month/year) |
|---|---|---|---|
| | | US 8288537 B2 | 16 October 2012 |
| | | WO 01-42216 A2 | 14 June 2001 |
| | | WO 01-42216 A3 | 28 February 2002 |
| | | ZA 200204390 B | 27 August 2003 |
| KR 10-2006-0094868 A | 30 August 2006 | AP 2374 A | 07 March 2012 |
| | | AR 055314 A1 | 15 August 2007 |
| | | AU 2006-217293 A1 | 31 August 2006 |
| | | AU 2006-217293 B2 | 02 February 2012 |
| | | BR PI0607330 A2 | 29 September 2009 |
| | | BR PI0607330 B1 | 03 August 2021 |
| | | CA 2598347 A1 | 31 August 2006 |
| | | CA 2598347 C | 02 August 2011 |
| | | CN 101128459 A | 20 February 2008 |
| | | CN 101128459 B | 05 October 2011 |
| | | DK 1851214 T3 | 15 October 2012 |
| | | EA 013005 B1 | 26 February 2010 |
| | | EA 200701810 A1 | 28 February 2008 |
| | | EP 1851214 A1 | 07 November 2007 |
| | | EP 1851214 B1 | 05 September 2012 |
| | | ES 2394480 T3 | 01 February 2013 |
| | | HK 1111146 A1 | 01 August 2008 |
| | | JP 2008-531551 A | 14 August 2008 |
| | | JP 4961357 B2 | 27 June 2012 |
| | | KR 10-0747002 B1 | 07 August 2007 |
| | | MA 29311 B1 | 03 March 2008 |
| | | MX 2007010338 A | 11 October 2007 |
| | | MY 149181 A | 31 July 2013 |
| | | NO 20074895 L | 26 November 2007 |
| | | NO 341347 B1 | 16 October 2017 |
| | | NZ 560805 A | 24 December 2010 |
| | | PE 10762006 A1 | 27 December 2006 |
| | | PE 20061076 A1 | 27 December 2006 |
| | | PL 1851214 T3 | 29 March 2013 |
| | | PT 1851214 E | 18 October 2012 |
| | | SG 156689 A1 | 26 November 2009 |
| | | SI 1851214 T1 | 30 November 2012 |
| | | SI EP1851214 T1 | 30 November 2012 |
| | | TW 200640918 A | 01 December 2006 |
| | | TW I377205 B | 21 November 2012 |
| | | UA 94395 C2 | 10 May 2011 |
| | | US 2008-0262032 A1 | 23 October 2008 |
| | | US 8044080 B2 | 25 October 2011 |
| | | WO 2006-090997 A1 | 31 August 2006 |
| | | ZA 200707202 B | 29 October 2008 |
| US 2004-0048797 A1 | 11 March 2004 | AU 2003-225088 A1 | 03 November 2003 |
| | | EP 1499898 A2 | 26 January 2005 |
| | | US 7960398 B2 | 14 June 2011 |
| | | WO 03-088917 A2 | 30 October 2003 |
| US 2017-0260175 A1 | 14 September 2017 | AU 2010-341573 A1 | 19 July 2012 |
| | | AU 2010-341573 B2 | 13 October 2016 |

Form PCT/ISA/210 (patent family annex) (July 2022)

**INTERNATIONAL SEARCH REPORT**
**Information on patent family members**

International application No.
**PCT/KR2023/000101**

| Patent document cited in search report | Publication date (day/month/year) | Patent family member(s) | Publication date (day/month/year) |
|---|---|---|---|
| | | BR 112012018386 A2 | 20 June 2017 |
| | | BR 112012018386 A8 | 19 December 2017 |
| | | CA 2785499 A1 | 21 July 2011 |
| | | CA 2785499 C | 02 May 2017 |
| | | CL 2012001714 A1 | 12 April 2013 |
| | | CN 102762548 A | 31 October 2012 |
| | | CN 102762548 B | 26 November 2014 |
| | | EP 2516417 A1 | 31 October 2012 |
| | | EP 2516417 B1 | 11 October 2017 |
| | | EP 3309152 A1 | 18 April 2018 |
| | | EP 3309152 B1 | 09 September 2020 |
| | | EP 3808739 A1 | 21 April 2021 |
| | | ES 2655391 T3 | 19 February 2018 |
| | | ES 2842399 T3 | 14 July 2021 |
| | | HK 1253412 A1 | 14 June 2019 |
| | | IL 220625 A | 29 May 2017 |
| | | JP 2013-515074 A | 02 May 2013 |
| | | JP 2015-101589 A | 04 June 2015 |
| | | JP 2017-114916 A | 29 June 2017 |
| | | JP 2018-131449 A | 23 August 2018 |
| | | JP 5721187 B2 | 20 May 2015 |
| | | JP 6132410 B2 | 24 May 2017 |
| | | JP 6510580 B2 | 08 May 2019 |
| | | KR 10-1758046 B1 | 14 July 2017 |
| | | KR 10-2012-0097406 A | 03 September 2012 |
| | | MX 2012007403 A | 12 September 2012 |
| | | MX 350761 B | 18 September 2017 |
| | | NZ 600954 A | 29 November 2013 |
| | | RU 2012131416 A | 27 January 2014 |
| | | RU 2573569 C2 | 20 January 2016 |
| | | SG 10201502073 A | 28 May 2015 |
| | | SG 181917 A1 | 30 August 2012 |
| | | UA 113492 C2 | 10 February 2017 |
| | | US 10183933 B2 | 22 January 2019 |
| | | US 2012-0040950 A1 | 16 February 2012 |
| | | US 2012-0202784 A1 | 09 August 2012 |
| | | US 2014-0038936 A1 | 06 February 2014 |
| | | US 8466288 B2 | 18 June 2013 |
| | | US 9676759 B2 | 13 June 2017 |
| | | WO 2011-0087776 A1 | 21 July 2011 |
| JP 2018-535927 A | 06 December 2018 | AU 2016-330029 A1 | 12 April 2018 |
| | | AU 2016-330029 B2 | 18 February 2021 |
| | | AU 2021-202973 A1 | 03 June 2021 |
| | | AU 2021-202973 B2 | 12 January 2023 |
| | | BR 112018005936 A2 | 16 October 2018 |
| | | CA 2999395 A1 | 06 April 2017 |
| | | CN 108473464 A | 31 August 2018 |
| | | CN 108473464 B | 23 August 2022 |
| | | EP 3356344 A1 | 08 August 2018 |
| | | EP 3356344 B1 | 16 November 2022 |

Form PCT/ISA/210 (patent family annex) (July 2022)

**INTERNATIONAL SEARCH REPORT**
**Information on patent family members**

International application No.
**PCT/KR2023/000101**

| Patent document cited in search report | Publication date (day/month/year) | Patent family member(s) | Publication date (day/month/year) |
|---|---|---|---|
| | | HK 1250716 A1 | 11 January 2019 |
| | | JP 7029388 B2 | 03 March 2022 |
| | | KR 10-2018-0081485 A | 16 July 2018 |
| | | MA 43031 A | 08 August 2018 |
| | | MX 2018003993 A | 09 November 2018 |
| | | NZ 740859 A | 24 September 2021 |
| | | PH 12018500626 A1 | 24 September 2018 |
| | | SA 518391217 B1 | 13 October 2021 |
| | | SG 10201912839 A | 27 February 2020 |
| | | TW 201726647 A | 01 August 2017 |
| | | TW I773651 B | 11 August 2022 |
| | | US 10526311 B2 | 07 January 2020 |
| | | US 11261171 B1 | 01 March 2022 |
| | | US 2019-0055215 A1 | 21 February 2019 |
| | | US 2022-0106287 A1 | 07 April 2022 |
| | | WO 2017-055859 A1 | 06 April 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020040066726
- KR 1020060013107
- KR 1020080025123
- WO 2007015931 A
- WO 2006090997 A
- WO 2008068615 A1